# EUROPEAN PATENT APPLICATION

(11) **EP 2 077 331 A1**
(43) Date of publication of application: **08.07.2009**
(21) Application number: 07806132.2
(22) Date of filing: 28.08.2007
(51) Int. Cl.: C12Q 1/02, C12Q 1/48, C12Q 1/68, G01N 33/15, G01N 33/50, C12N 15/09

(54) **METHOD FOR EVALUATING COMPOUND USING PKC-iota**

(30) Priority: 28.08.2006 JP 2006229999
(71) Applicant: BANYU PHARMACEUTICAL CO., LTD., Tokyo 102-8667 (JP)
(72) Inventor: BAMBA, Rie, Tsukuba-shi, Ibaraki 3002611 (JP); EGUCHI, Tomohiro, Ibaraki 3002611 (JP); KOMATANI, Hideya, Ibaraki 3002611 (JP); KOTANI, Hidehito, Ibaraki 3002611 (JP); SHIMOYAMA, Susumu, Ibaraki 3002611 (JP); YAMANAKA, Kazunori, Ibaraki 3002611 (JP)
(74) Representative: Hussain, Deeba
(86) International application number: PCT/JP2007/066653
(87) International publication number: WO 2008/026584

(57) **Abstract**

It is intended to find a target gene for an antitumor agent effective specifically in a cancer cell caused by abnormality of p53 or PI3 kinase, and to realize the evaluation of a compound using the gene. A method for evaluating a compound effective in the treatment of cancer **characterized by** comprising the steps of: preparing a cell expressing PKC-iota by introducing a PKC-iota gene; bringing a test compound into contact with the cell; and detecting a specific binding of the test compound to the PKC-iota. A method for evaluating a compound having the step of measuring the activity or expression level of an intracellular signal transducer induced by the contact instead of the detection step.

## Description

### TECHNICAL FIELD

The present invention relates to a method for evaluation of a compound using PKC-iota, and to a compound obtained according to the method. The invention also relates to a gene/protein marker for estimating the effect of a PKC-iota inhibitor.

### BACKGROUND ART

It is widely known that genetic abnormality is seen in cancer cells; and heretofore, many cancer genes and cancer suppressor genes have been found. In addition, it has been clarified that there exist multi-stage oncogenic mechanisms that require abnormality in multiple genes for oncogenic transformation of normal cells. Concretely, it is said that oncogenic transformation of normal cells requires accumulation of abnormality in multiple genes including DNA repair genes, cancer suppressor genes and cancer genes.

Above all, p53 known as a cancer suppressor gene was first found as a molecule to form a complex with a T-antigen of SV40 that is a tumor virus (Non-Patent Reference 1). After then, it has been clarified that the p53 gene is a cancer suppressor gene existing in the short arm (17p13) of No. 17 chromosome that is deleted in many cancers, and both the gene and its allele are inactivated through deletion and mutation (Non-Patent Reference 2). Further, it has been clarified that the gene product is a transcriptional regulator comprising 393 amino acids, and this functions as forming a tetramer. Moreover, an extremely large number of genes have been identified as target genes for p53, including p21 and 14-3-3 that participate in cell cycle, and bax, PIG3 and GADD45 that participate in apoptosis; and it is considered that p53 abnormality may bring about the abnormality in transcriptional regulation of its target genes.

In fact, it has been clarified that the p53 gene mutation reaches 70 % in lung cancer, 45 % in stomach cancer, 30 % in breast cancer, 65 % in colon cancer, 61 % in bladder cancer, and 70 % in pancreas cancer; and the data are noticeably high as compared with those of other cancer suppressor genes.

In that situation, many studies have been made relating to the relationship between the mutation and dysfunction of p53 gene and diseases. For example, Soussi et al. made database of p53 mutation of 15000 cases or more (Non-Patent Reference 3), indicating frequent mutation occurring in the codons 175, 245, 248 and 273 positioned within the DNA-binding domain region of p53. In addition, it has been clarified that mutation from Arg to Ser is seen in the codon 249 in the DNA-binding domain in some types of hepatocellular carcinoma (Non-Patent Reference 4). Further, it has been reported that in the skin cancer caused by UV rays, CC in the p53 gene is thymine-dimerized (Non-Patent Reference 5). To that effect, p53 has great influences on the onset of many cancers, and therefore it is desired to identify potential drug target genes effective only in p53 abnormal cells.

On the other hand, PI3 kinase comprises subunits of 85 kD (p85) and 110 kD (p110). The p85 subunit does not have a kinase active site, but functions as an adaptor protein to associate with a membrane-binding receptor via the SH2 domain existing in the p85 subunit. On the other hand, the p110 subunit functions as a catalyst subunit having a kinase activity, and the two subunits, as combined together, function as a phosphorylation enzyme that regulates intracellular signal transduction.

PI3 kinase is activated by stimulation via various growth factor receptors, and phosphorylates phosphatidylinositol 4,5-diphosphate (PIP2) to produce phosphatidylinositol 3,4,5-triphosphate (PIP3), and activates a further downstream signal mainly via Akt. Akt is a molecule known as a cancer gene. It is also known that the mutation of a cancer suppressor gene PTEM that regulates the PI3 kinase pathway to be negative with PIP3 serving as a substrate is also a cause of oncogenic transformation, and the abnormality in the PI3 kinase pathway plays an important role in oncogenic transformation. It is reported that PI3 kinase activates PDK1, Akt and protein kinase C existing downstream via PIP3 in normal cells (Non-Patent Reference 6). There is a report saying that a protein kinase C-zeta, a type of atypical PKC may be activated by PDK-1 (Non-Patent Reference 8). Further, it is reported that a protein kinase C-zeta and a protein kinase C-lambda, other types of atypical PKC, are positioned downstream the PI3 kinase activated by insulin stimulation (Non-Patent Reference 9).

In addition, it is known that the mutation of p53 reduces the expression of PTEN, and the p53 pathway and the PI3 kinase pathway, cross-talking with each other, play an indispensable role for growth and living of cells.

It is known that, like that of P53, the mutation of PI3 kinase (phosphatidylinositol 3-kinase) is a cause of various cancers. For example, it is reported that, in many cancers such as endometrial cancer, intestinal cancer, breast cancer, ovarian cancer and others, both subunits of p85 and p110 are mutated (Non-Patent Reference 7)

On the other hand, PKC (protein kinase C) is a serine-threonine phosphorylation enzyme discovered in 1970's. PKC is activated by Ca²⁺ induced by diacyl glycerol produced by cytoplasmic membrane by receptor stimulation or the like and by inositol triphosphate produced simultaneously. The activated PKC phosphorylates the substrate protein to produce a predetermined cellular response, thereby exhibiting a physiological function. PKC in mammals is generally grouped into three types of cPKC (conventional PKC), nPKC (novel PKC) and aPKC (atypical PKC) based on the difference in the structure therebetween. Two C1 domains, on which diacyl glycerol and phorbol ester act, and a C2 domain to which calcium bonds exist in the regulatory region of cPKC; but the C2 domain is not in nPKC, and one C1 domain is not in aPKC.

PKC-iota (PKC-t: SEQ ID NOs 1 and 2) was isolated by using the sequence of the site where the base sequence was highly kept as such in the catalyst site of a known PKC, through degenerate PCR, as a serine-threonine oxidation enzyme to be grouped in aPKC (Non-Patent Reference 10). PKC-iota is a protein comprising 587 amino acids, and has 72 % homology to PKC-ζ belonging to aPKC.

There are still many unclear points relating to the physiological function of PKC-iota; however, Jin Z. et al. reported as follows: A carcinogenic nitrosamine, 4-(methylnirosamine)-1-(3-pyridyl)-1-butanone (NNK) to be produced through nitrosation of nicotine phosphorylates Bad that belongs to a Bc12 family, via PKC-iota, thereby stopping the apoptosis promoting function of Bad, and as a result, lung cancer cells are thereby kept living (Non-Patent Reference 11). Specifically, PKC-iota directly phosphorylates the Bc1 family that induces apoptosis, in the signal transduction pathway that regulates the life support in cancer cells.

Non-Patent Reference 1: J. Virol., Vol. 31, p. 463, 1979
Non-Patent Reference 2: N. Engl. J. Med., Vol. 319, p. 525, 1988
Non-Patent Reference 3: Nucleic Acids Res., Vol. 22, p. 3551, 1994
Non-Patent Reference 4: Nature, Vol. 350, p. 427, 1991
Non-Patent Reference 5: PNAS, Vol. 88, p. 10124, 1991
Non-Patent Reference 6: Science, Vol. 281, p. 2042, 1998
Non-Patent Reference 7: Cancer Cell, Vol. 12, p. 104, 2007
Non-Patent Reference 8: Curr. Biol., Vol. 8, p. 1069, 1998
Non-Patent Reference 9: J. Cell Biol., Vol. 164, p. 279, 2004
Non-Patent Reference 10: J. Biol. Chem., Vol. 268, p. 24296, 1993
Non-Patent Reference 11: J. Biol. Chem., Vol. 280, p. 16045, 2005

### DISCLOSURE OF THE INVENTION

### PROBLEMS THAT THE INVENTION IS TO SOLVE

However, at present, there is known neither information indicating the relevance between PKC-iota and p53, and nor suggestion indicating the possibility that PKC-iota could be an effective anticancer agent target gene specific to p53 mutation cells. There are reports relating to PKC-iota and PI3 kinase, which indicate that PKC-iota and aPKC function downstream the PI3 kinase in normal cells (Non-Patent References 6, 8 and 9); but at present, there is known neither information relating to the mechanism of activation of PKC-iota in cancer cells nor information relating to the relevance between the expression of PKC-iota and cancer to be caused by PI3 kinase. In addition, at present, there is known little information relating to anticancer agent target genes specifically effective to cancer cells caused by p53 or PI3 kinase abnormality, and much more information is desired for potential drug target genes.

The invention has been made in consideration of the above-mentioned prior art problems, and its object is to find out an anticancer agent target gene specifically effective to cancer cells caused by p53 or PI3 kinase abnormality, and to enable compound evaluation by the use of the gene.

### MEANS FOR SOLVING THE PROBLEMS

The present inventors have assiduously studied for the purpose of attaining the above object and, as a result, have found that, when PKC-iota expression is inhibited, then the growth of cells having a mutant p53 or a mutant PI3 kinase can be thereby inhibited, and in that case, PKC-iota inhibits the cell growth via phosphorylation of BAD, or that is, by inhibiting PKC-iota, BAD is dephosphorylated, thereby inducing apoptosis via the Bcl-XL function inhibition. In other words, the inventors have found that, in cancer cells having mutation in P53 or PI3 kinase, inhibition of the expression or the activity of PKC-iota may result in apoptosis induction, and have completed the present invention. Further, the inventors have found out a series of gene groups, of which the expression may increase or decrease by PKC-iota inhibition and which are therefore usable as a marker for estimating the potency of PKC-iota inhibitors, and have completed the invention.

Specifically, the method for compound evaluation of the invention is a method for evaluating a compound effective for cancer treatment, and is characterized by comprising a step of introducing a PKC-iota gene into a cell to prepare a cell capable of expressing PKC-iota, a step of contacting a test compound with the cell, and a step of detecting the specific binding of the test compound to PKC-iota.

Also, the method for compound evaluation of the invention is a method for evaluating a compound effective for cancer treatment, and is characterized by comprising a step of introducing a PKC-iota gene into a cell to prepare a cell capable of expressing PKC-iota, a step of contacting a test compound with the cell, a step of measuring the activity of the intracellular transmitter formed by the contact, and a step of comparing this activity with the activity of the intracellular transmitter in a case not contacted with the compound.

Also, the compound evaluation method of the invention is a method for evaluation of a compound effective for treatment of cancer, and is characterized by comprising a step of introducing a PKC-iota gene into a cell to prepare a cell capable of expressing PKC-iota, a step of contacting a test compound with the cell, a step of measuring the expression level of PKC-iota or the expression level of the intracellular transmitter via PKC-iota.

In the above-mentioned compound evaluation methods, the intracellular transmitter is preferably at least one selected from a group consisting of BAD, LLGL2, HMGB3, NFIB, PXDN, CLDN 12, RPIA, AKT3 and SNF1LK.

Further, the compound evaluation method of the invention is a method for evaluation of a compound effective for treatment of cancer, and is characterized by comprising a step of contacting a test compound with PKC-iota, and a step of detecting the change in the activity of PKC-iota through the contact.

Also, the compound evaluation method of the invention is a method for evaluation of a compound effective for treatment of cancer, and is characterized by comprising a step of contacting a test compound with PKC-iota in the presence of BAD or LLGL2, and a step of detecting the change in the activity of PKC-iota through the contact.

All the above-mentioned compound evaluation methods are favorably applicable to cancers caused by p53 dysfunction.

Further, all the above-mentioned compound evaluation methods are favorably applicable to cancers caused by PI3 kinase (phosphatidylinositol 3-kinase) dysfunction.

The diagnostic method of the invention is characterized by comprising a step of measuring the expression level of the PKC-iota gene in a test tissue or a test cell, a step of comparing that expression level and the expression level of the PKC-iota gene in a normal tissue or a normal cell, and, as a result of the comparison, a step of determining as to whether or not the expression level of the PKC-iota gene in the test tissue or the test cell is significantly larger than the expression level of the PKC-iota gene in the normal tissue or the normal cell.

Further, the diagnostic method of the invention is characterized by comprising a step of measuring the activity of PKC-iota in a test tissue or a test cell, a step of comparing that activity and the activity of PKC-iota in a normal tissue or a normal cell, and, as a result of the comparison, a step of determining as to whether or not the activity of PKC-iota in the test tissue or the test cell is significantly larger than the activity of PKC-iota in the normal tissue or the normal cell.

The method for estimating or determining the effect of a PKC-iota inhibitor of the invention is characterized by comprising a step of measuring the activity of PKC-iota in the tissue derived from a test subject to which a PKC-iota inhibitor has been administered, and a step of comparing the activity before and after the PKC-iota inhibitor administration.

Further, the gene marker of the invention is a gene marker for estimating or determining the potency of a PKC-iota inhibitor, and is **characterized in that** the gene is at least one selected from a group consisting of BAD, LLGL2, HMGB3, NFIB, PXDN, CLDN12, RPIA, AKT3 and SNF1LK.

The invention also includes a microarray provided with the gene marker.

The protein marker of the invention is a protein marker for estimating or determining the potency of a PKC-iota inhibitor, and is **characterized in that** the protein is at least one selected from a group consisting of LLGL2, HMGB3, NFIB, PXDN, CLDN12, RPIA, AKT3 and SNF1LK.

The invention also includes a microarray provided with the protein marker.

The cancer diagnostic kit of the invention is characterized by containing an antibody capable of detecting the protein marker.

The antibody array of the invention is characterized by comprising an antibody capable of detecting the protein marker.

The method for estimating or determining the effect of a PKC-iota inhibitor of the invention is characterized by comprising a step of detecting any one gene selected from a group consisting of HMGB3, NFIB, PXDN, CLDN12, RPIA, AKT3 and SNF1LK in the tissue derived from a test subject to which a PKC-iota inhibitor has been administered, and a step of comparing the expression level before and after the PKC-iota inhibitor administration.

In the method for estimating or determining the effect of a PKC-iota inhibitor of the invention, the detecting unit for detection is preferably a PCR or DNA microarray.

The method for estimating or determining the effect of a PKC-iota inhibitor of the invention is characterized by comprising a step of measuring the activity of any one protein selected from a group consisting of HMGB3, NFIB, PXDN, CLDN12, RPIA, AKT3 and SNF1LK in the tissue derived from a test subject to which a PKC-iota inhibitor has been administered, and a step of comparing the activity before and after the PKC-iota inhibitor administration.

Further, the method for estimating or determining the effect of a PKC-iota inhibitor of the invention is characterized by comprising a step of measuring the phosphorylation level of BAD or LLGL2 in the tissue derived from a test subject to which a PKC-iota inhibitor has been administered, and a step of comparing the phosphorylation level before and after the PKC-iota inhibitor administration.

### EFFECT OF THE INVENTION

PKC-iota has been found as a target gene specifically acting on cancers caused by abnormality in expression and function of p53 or PI3 kinase, therefore enabling the evaluation of compounds containing the gene as a potential drug target gene (for example, screening for drug candidate compounds). There are extremely many types of cancers caused by the abnormality in expression and function of p53 or PI3 kinase, and it may be possible to provide compounds effective for treatment of many cancers. Diagnosis based the expression level of PKC-iota as an index enables diagnosis of cancers to be caused by the abnormality in expression and function of p53 or PI3 kinase. BAD, LLGL2, HMGB3, NFIB, PXDN, CLDN12, RPIA, AKT3 and SNF1LK may be usable as a marker for estimating the potency of PKC-iota inhibitors.

### BEST MODE FOR CARRYING OUT THE INVENTION

Preferred embodiments of the invention are described in detail hereinunder.

The terms as referred to in the invention are described.

"PKC-iota" in the invention is not specifically defined in point of the origin of the biological species, including, for example, PKC-iota derived from human, monkey, mouse, rat, canine or rabbit. Above all, preferred is a human PKC-iota gene as the objects to which the compound to be evaluated is administered are humans.

The PKC-iota gene in the invention includes any one with one or more base substitution, deletion, addition or insertion, so far as it has a biological function analogous to that of PKC-iota and has a kinase activity. The gene may be any one that codes for the intended protein, not specifically defined in point of its sequence, but its homology is preferably at least 90 % (for example, 91, 92, 93, 94, 95, 96, 97, 98 or 99 % or more).

The PKC-iota gene in the invention includes a nucleic acid that hybridizes with the PKC-iota gene under a stringent condition. "Hybridize under a stringent condition" as referred to herein means that the two nucleic acid fragments hybridize with each other under the hybridization condition described in Molecular Cloning: A Laboratory Manual, 2nd Edition, Cold Spring Harbor (1989), 9.47-9.62 and 11.45-11.61. More concretely, for example, the process under the condition comprises 6.0 × SSC hybridization at about 45°C followed by 2.0 × SSC washing at 50°C. For stringency selection, the salt concentration in the washing step may fall within a range of from about 2.0 × SSC at 50°C of low stringency to about 0.2 × SSC at 50°C of high stringency. Further, the temperature in the washing step may be elevated from room temperature of about 22°C under a low stringency condition up to about 65°C under a high stringency condition.

The PKC-iota protein in the invention includes any one with one or more amino acid substitution, deletion, addition or insertion, so far as it has a biological function analogous to that of PKC-iota and has a kinase activity. The protein sequence is not specifically defined, but its homology is preferably at least 80 %, more preferably at least 90 % (for example, 91, 92, 93, 94, 95, 96, 97, 98 or 99 % or more).

The present inventors have found that, when PKC-iota expression is inhibited, then the growth of cells having a mutant p53 or PI3 kinase can be thereby inhibited. It is known that the p53 or PI3 kinase expression level has close relation to oncogenic transformation of cells. Accordingly, measurement of the expression level and the activity of PKC-iota makes it possible to detect cancer that has abnormality in p53 or PI3 kinase. In addition, since there is a relationship between the expression level of PKC-iota and the mutation of p53 or the expression level of PI3 kinase, the oncogenic transformation of cells owing to dysfunction of p53 or PI3 kinase to be caused by the abnormality in the expression level of p53 or PI3 kinase has close relation to the expression or the activity of PKC-iota. Accordingly, taking PKC-iota as a potential drug target, the search for compounds acting on PKC-iota will make it possible to obtain anticancer agents.

"p53 dysfunction" as referred to in the invention may be caused by base deletion (for example, nonsense mutation), substitution (for example, missense mutation, point mutation), insertion, frame shifting or the like in p53 gene, but the cause of dysfunction to which the invention is directed is not specifically defined, and includes any and every dysfunction caused by any of these.

Examples of p53 dysfunction include a case of p53 protein transcriptional activity reduction or activation, and a case with no transcription, further including a case of gene encoding mutation though no abnormality is recognized in the gene activity. Concretely, for example, it includes Li-Fraumeni syndrome (Science, Vol. 250, p. 1233, 1990), hepatocellular carcinoma (Nature, Vol. 350, p. 377, 1991), osteogenic sarcoma (Proc. Natl. Acad. Sci. USA, Vol. 84, p. 7716, 1987), rhabdomyosarcoma (Proc. Natl. Acad. Sci. USA, Vol. 87, p. 5863, 1990), colon cancer (Science, Vol. 244, p. 217, 1989), lung cancer (Science, Vol. 246, p. 491, 1989), glioblastoma (Am. J. Hum. Genet., Vol. 47 (suppl.), A4, 1990), esophageal cancer (Proc. Natl. Acad. Sci. USA, Vol. 87, p. 9958, 1990), bladder cancer (Science, Vol. 252, p. 706, 1991), squamous cell cancer (Proc. Natl. Acad. Sci. USA, Vol. 88, p. 10124, 1991), cervical cancer (Lancet, Vol. 339, p. 1070, 1992), lung cancer (Proc. Natl. Acad. Sci. USA, Vol. 89, p. 7262, 1992) and leukemia/lymphoma (J. Clin. Invest., Vol. 90, p. 653, 1992), in which p53 participation is suggested.

"PI3 kinase dysfunction" as referred to in the invention may be caused by base deletion (for example, nonsense mutation), substitution (for example, missense mutation, point mutation), insertion, frame shifting or the like in PI3 kinase gene, but the cause of dysfunction to which the invention is directed is not specifically defined, and includes any and every dysfunction caused by any of these.

Examples of PI3 kinase dysfunction include a case of PI3 kinase activity reduction or activation, and a case with no phosphorylation, further including a case of gene encoding mutation though no abnormality is recognized in the gene activity. Concretely, for example, it includes endometrial cancer, intestinal cancer, breast cancer, ovarian cancer.

"Test tissue" as referred to in the invention means a tissue that may be taken out of a living body for cancer examination, and it may be any cancer tissue that must be investigated for p53 participation therein or may also be any other tissue that requires cancer diagnosis, not specifically defined in point of its type. Examples of the tissue include various human tissues, as well as tissues possibly suffering from, for example, neuroblastoma, retinoblastoma, brain tumor, head and neck cancer, pituitary adenoma, glioma, acoustic schwannoma, oral cancer, pharyngeal cancer, laryngeal cancer, thyroid cancer, thymoma, mesothelioma, breast cancer, lung cancer, stomach cancer, esophageal cancer, colon cancer, hepatoma, pancreas cancer, pancreatic endocrine tumor, biliary cancer, penile cancer, vulvar cancer, ureteropelvic cancer, renal cancer, testicular cancer, prostate cancer, bladder cancer, uterine cancer, chorionic disorder, vaginal cancer, ovarian cancer, fallopian tube cancer, ovarian germ cell tumor, skin cancer, myocosis fungoides, malignant melanoma, soft tissue sarcoma, bone neoplasm, malignant lymphoma, leukemia, myelodysplastic syndrome, multiple myeloma, lymphedema.

Similarly, "test cell" as referred to in the invention means a cell that may be taken out of a living body for cancer examination, and it may be any cancer tissue-derived cell that must be investigated for p53 participation therein or may also be any other tissue-derived cell that requires cancer diagnosis, not specifically defined in point of its type. Examples of the cell include cells possibly suffering from, for example, neuroblastoma, retinoblastoma, brain tumor, head and neck cancer, pituitary adenoma, glioma, acoustic schwannoma, oral cancer, pharyngeal cancer, laryngeal cancer, thyroid cancer, thymoma, mesothelioma, breast cancer, lung cancer, stomach cancer, esophageal cancer, colon cancer, hepatoma, pancreas cancer, pancreatic endocrine tumor, biliary cancer, penile cancer, vulvar cancer, ureteropelvic cancer, renal cancer, testicular cancer, prostate cancer, bladder cancer, uterine cancer, chorionic disorder, vaginal cancer, ovarian cancer, fallopian tube cancer, ovarian germ cell tumor, skin cancer, myocosis fungoides, malignant melanoma, soft tissue sarcoma, bone neoplasm, malignant lymphoma, leukemia, myelodysplastic syndrome, multiple myeloma, lymphedema.

### (1) Compound Evaluation:

Using a PKC-iota gene or protein, it is possible to evaluate a compound acting on PKC-iota. For detecting the effect to PKC-iota, employable are a method of detecting specific binding to PKC-iota of a test compound (for example, binding to induce inhibition of enzymatic activity); a method of detecting the expression level of the gene changed through its contact with a test compound; and a method of detecting the activity or expression level of the intracellular transmitter formed through the contact. These are described below in order.

First described is the method of detecting the specific binding to PKC-iota of a test compound to thereby evaluate the test compound.

The first method of compound evaluation of the invention is characterized by comprising a step of introducing a PKC-iota gene into a cell to prepare a cell capable of expressing PKC-iota, a step of contacting a test compound with the cell, and a step of detecting the specific binding of the test compound to PKC-iota.
Not specifically defined, the test compound to be evaluated in the method of the invention includes, for example, single compounds such as natural compounds, organic compounds, inorganic compounds, proteins, peptides, as well as expression products of compound library or gene library, cell extracts, cell culture supernatants, fermented microorganism products, marine organism extracts, plant extracts, procaryotic cell extracts, eucaryotic cell extracts and animal cell extracts. The test sample may be suitably labeled, if desired. The labeling includes, for example, radiolabeling, fluorescence labeling. In addition to the above-mentioned test samples, the test compound further includes mixture of two or more of such test samples.

"Specific binding" means the binding of the test compound to PKC-iota, and the test compound binding to PKC-iota has some influence on the activity and/or the expression of PKC-iota.

The PKC-iota gene-expressing cell may be prepared according to a method known to those skilled in the art, and its concrete method is not specifically defined. For example, the cell may be prepared as follows: A PKC-iota gene or a nucleic acid comprising a part of it is cloned into an expression vector containing a suitable promoter and a suitable transcriptional regulator, and the vector having the cloned nucleic acid is introduced into a host cell, thereby preparing the intended cell. In this, the vector may be any one usable as an expression vector, not specifically defined, and, for example, it includes pCMV-Tag, pcDNA3.1, pBlueBacHis2, pCI-neo, pcDNAI, pMC1neo, pXT1, pSG5, pEF1/V5-HisB, pCR2.1, pET11, λgt11 and pCR3.1.

Next, the expression vector having, as introduced thereinto, the PKC-iota gene or the nucleic acid comprising a part of it is introduced into a host cell. Not specifically defined, the host cell may be any one generally used for gene expression, including, for example, animal cells, insect cells, plant cells, microorganisms. Concretely, for example, they include COS1, COS7, CHO, NIH/3T3, 293, Raji, CV11, C1271, MRC-5, CPAE, HeLa, 293T and Sf9. For introducing the expression vector into a host cell, employable is any known method with no specific limitation. Concretely, for example, employable are an electroporation method, a calcium phosphate method, a DEAE-dextran method, a lipofection method, and a gene gun method.

Next, the PKC-iota-expression cell, thus prepared, is contacted with a test compound. The contact method is not specifically defined. When PKC-iota is expressed inside the cell, then a test sample may be added to the cell culture or a buffer containing the cells.

The PKC-iota-expressing cells, thus prepared in the manner as above, may be pulverized or solubilized, and then the resulting cell extract may be used for contact of PKC-iota with a test compound. For pulverizing the cells, for example, employable are a method of crushing the cells with a homogenizer; and a method of ultrasonically pulverizing the cells. For solubilizing the cells, for example, employable is a method of suspending the cells in a buffer that contains a solubilizer (for example, Triton X-100, sodium cholate, NP-40) and then statically holding it on ice.
In case where the test sample is a protein, for example, a vector that contains a DNA coding for the protein may be introduced into the PKC-iota-expressing cell for the intended contact.

The binding of PKC-iota to the test compound may be detected, for example, through detection of the labeling given to the PKC-iota-binding compound (for example, through detection of the binding amount based on radioactivity or fluorescence intensity); through detection of the index that the test compound inhibits the phosphorylation of the substrate protein (e.g., BAD, LLGL2) or substrate peptide by PKC-iota (for example, through detection of the phosphorylated amount by the use of radioactivity or fluorescence intensity); or through detection of the index of inhibition of signal transmission induced by the binding of PKC-iota to the test compound (for example, phosphorylation of substrate protein such as BAD, induction to apoptosis). The present inventors have found that, when BAD is used as the substrate, the site to be phosphorylated by BAD is the 112th, 136th and 155th amino acids. Accordingly, a partial peptide of BAD containing the site can be used as the substrate.

The binding between PKC-iota and the test compound is detected, and the result is compared with the result of a control reacted in the same manner in the absence of the test compound; and in case where the PKC-iota activity is inhibited by the binding, the test compound functions as a PKC-iota inhibitor.

The second method for compound evaluation of the invention is characterized by comprising a step of introducing a PKC-iota gene into a cell to prepare a cell capable of expressing PKC-iota, a step of contacting a test compound with the cell, a step of measuring the activity of the intracellular transmitter formed by the contact, and a step of comparing this activity with the activity of the intracellular transmitter in a case not contacted with the compound.

In this evaluation method, the cell prepared in the same manner as that of the first compound evaluation method is used, and a test compound is contacted with the cell, and the activity of the intracellular transmitter formed by the contact is measured.

Not specifically defined, the intracellular transmitter may be any molecule positioned downstream the PKC-iota in a signal transmission pathway, of which the expression and the activity are influenced by the expression and the activity of PKC-iota. For example, it includes BAD (Accession No. NM_032989.1: SEQ ID NOs 3 and 4), LLGL2 (Accession No. NM_001031803: SEQ ID NOs 5 and 6), HMGB3 (Accession No. NM_005342: SEQ ID NOs 7 and 8), NFIB (Accession No. NM_005596: SEQ ID NOs 9 and 10), PXDN (Accession No. NM_012293: SEQ ID NOs 11 and 12), CLDN12 (Accession No. NM_012129: SEQ ID NOs 13 and 14), RPIA (Accession No. NM_144563: SEQ ID NOs 15 and 16), AKT3 (Accession No. NM_005465: SEQ ID NOs 17 and 18) and SNF1LK (Accession No. NM_173354: SEQ ID NOs 19 and 20).

The activity of the intracellular transmitter may be measured suitably under a preferred condition in accordance with the type of the transmitter. For example, the activity of the transcriptional regulator HMGB3 may be measured through ELISA assay of a gel shift method or a colorimetric method. The activity condition of BAD may be measured as follows: When PKC-iota is activated, then the 112th, 136th and 155th amino acids of BAD are thereby phosphorylated, and Bcl-xl activity is inhibited by dephosphorylation. Therefore, by measuring the Bcl-xl activity-inhibiting activity, the activity condition of BAD may be thereby determined.

The third compound evaluation method of the invention is characterized by comprising a step of introducing a PKC-iota gene into a cell to prepare a cell capable of expressing PKC-iota, a step of contacting a test compound with the cell, a step of measuring the expression level of PKC-iota or the expression level of the intracellular transmitter via PKC-iota. For selecting (screening) desired compounds as a result of the compound evaluation, the method may further include a step of comparing the expression level as that before contact, thereby selecting a test compound capable of increasing or decreasing the expression level of PKC-iota or the expression level of the intracellular transmitter via PKC-iota.

In this evaluation method, the cell prepared in the same manner as that of the first compound evaluation method is used, and a test compound is contacted with the cell, and the contact-derived expression level of PKC-iota or the expression level of the intracellular transmitter via PKC-iota is measured.

Not specifically defined, the intracellular transmitter via PKC-iota may be any molecule positioned downstream the PKC-iota in a signal transmission pathway, of which the expression and the activity are influenced by the expression and the activity of PKC-iota. For example, it includes BAD, LLGL2, HMGB3, NFIB, PXDN, CLDN12, RPIA, AKT3 and SNF1LK.

In this, when the expression level is taken as the index, then the method for measuring the expressing level is not specifically defined. For example, it may be measured through northern blotting or western blotting or by the use of a DNA chip. The "expression level" as referred to in the invention is meant to indicate the absolute amount or the relative amount of the transcriptional product of the gene that codes for the protein existing on the signal transmission pathway via PKC-iota. In this case, the gene includes any of DNA or mRNA. In case where the object for expression detection is a protein, the "expression level" is meant to indicates the absolute amount or the relative amount of the translation product of the protein existing on the signal transmission pathway via PKC-iota. In case where the activity of the molecule on the signal transmission is taken as the index, then the activity measurement method is not specifically defined. Depending on the type of the molecule to be measured, a suitable method for it may be selected.

On the other hand, an isolated PKC-iota protein may be directly used for compound evaluation. In other words, a test compound is contacted with a PKC-iota protein, and then the change in the activity of the PKC-iota protein produced through the contact is detected.

The contact method is not specifically defined. Concretely, for example, they may be mixed in a solution such as buffer (e.g., phosphate buffer, Tris chloride buffer, HEPES buffer) and may be contacted with each other; or a PKC-iota protein may be fixed on a membrane, and it may be contacted with a test compound on the membrane. The reaction condition for the contact in a buffer is not specifically defined. For example, the system may be incubated at a pH of from 5.0 to 10, preferably from 6.5 to 8.5, more preferably from 7.0 to 8.0, generally under a condition of from 10 to 50°C, preferably from 20 to 40°C, even more preferably from 35 to 40°C, still more preferably at 37°C, for 1 minute to 24 hours, preferably 5 minutes to 10 hours, more preferably 10 minutes to 3 hours, even more preferably 30 minutes to 1 hour.

Next, the change in the activity of PKC-iota induced by the contact is detected.

For measuring the protein activity, a desired method may be selected depending on the property of the protein used. Concretely, for example, a substrate suitable for PKC-iota is prepared, and kinase assay is carried out in the presence of PKC-iota, the substrate and a test compound in a solvent. In this case, the enzymatic reaction may be confirmed by the intake of radiolabeled phosphorus (e.g., ³²P, ³³P)_{.}

For measuring the activity of the PKC-iota protein, also usable is BAD or LLGL2 as the substrate. The present inventors have found that PKC-iota directly phosphorylates BAD or LLGL2 as a substrate thereof. Regarding BAD, at least the 112th, 136th and 155th amino acids of BAD are phosphorylated with PKC-iota, and therefore the partial peptide containing the amino acid may be used as a substrate. Regarding LLGL2, at least 653rd serine of LLGL2 is phosphorylated with PKC-iota, and therefore the partial peptide containing the serine may be used as a substrate. In case where the partial peptide is used, the number of the amino acids constituting it is not specifically defined. For both BAD and LLGL2, the partial peptide preferably comprises from 8 to 50 amino acids, more preferably from 10 to 30 amino acids, even more preferably from 12 to 20 amino acids.

In the method of measuring the activity of PKC-iota, taking BAD as a substrate, usable is any known phosphorylation detecting method. For example, it includes kinase assay of confirming the intake of radiolabeled phosphorus (e.g., ³²P), and western blotting of using a phosphorylated BAD antibody. The phosphorylated BAD antibody may be prepared in any known method. For example, using a peptide fragment containing any of the 112the, 136th and 155th amino acids of BAD, as an antigen, a polyclonal or monoclonal antibody may be prepared according to a known method.

In the method of measuring the activity of PKC-iota, taking LLGL2 as a substrate, usable is any known phosphorylation detecting method. For example, it includes kinase assay of confirming the intake of radiolabeled phosphorus (e.g., ³²P, ³³P), and western blotting of using a phosphorylated LLGL2 antibody. The phosphorylated LLGL2 antibody may be prepared in any known method. For example, using a peptide fragment containing the 653rd serine of LLGL2, as an antigen, a polyclonal or monoclonal antibody may be prepared according to a known method.

As in the above, as a result of evaluation of a compound according to the compound evaluation method of the invention, in case where the PKC-iota activity in the presence of the test compound is lower than the binding activity in the absence of the test compound (control), then it may be determined that the test compound is an antagonist having an activity of inhibiting the binding of PKC-iota to the ligand in the invention. The antagonist inhibits the biological activity of the ligand to PKC-iota and its analogue. Accordingly, the antagonist is useful as a pharmaceutical composition for treatment of cancer caused by the abnormality in the signal transmission system via PKC-iota, based on p53 dysfunction.

According to the compound evaluation method of the invention, it is possible to screen a compound that promotes or inhibits the intracellular signal transmission after binding of the test compound to PKC-iota. In other words, by evaluating plural test compounds according to the evaluation method of the invention, a compound capable of functioning as an agonist or an antagonist may be selected. As a result of the selection, in case where the change is inhibited as compared with the change in the signal transmission in the downstream after acting with the ligand or its analogue in the absence of a test compound, then it is determined that the test compound is a compound that inhibits the signal transmission to the downstream after binding of the test compound to PKC-iota. On the contrary, when the test compound enhances the intracellular signal transmission, then it may be determined that the test compound is a compound that promotes the intracellular signal transmission after the binding of the test compound to PKC-iota. The compound selected according to the screening method is effective for treatment and diagnosis of cancer based on p53 dysfunction.

### (2) PKC-iota Ligand:

According to the compound evaluation method of the invention described in (1), a PKC-iota ligand may be isolated. The ligand may be used as a drug having an anticancer effect by itself.

Test compounds to be a PKC-iota ligand are not specifically defined. For example, they include single compounds such as natural compounds, organic compounds, inorganic compounds, proteins, peptides, as well as PKC-iota antibodies, antisenses, RNAi's and ribozymes. Concretely, for example, PKC-iota ligands include myristoylated atypical PKC pseudosubstrate inhibitor peptide (by BIOSOURCE).

In case where the PKC-iota ligand of the invention is used as medicines for human and other animals, the substance itself may be directly administered to the patients, or it may be formulated into pharmaceutical compositions according to known pharmaceutical preparation methods and may be administered to them. For example, the compositions may be used orally, as optionally sugar-coated tablets, capsules, elixirs or microcapsules; or may be used parenterally as germ-free solution or suspension injections with water or with any other pharmaceutically-acceptable liquid. For example, the ligand may be suitably combined with a pharmaceutically-acceptable carrier or medium, concretely, germ-free water, physiological saline water, vegetable oil, emulsifier, suspending agent, surfactant, stabilizer, flavor, excipient, vehicle, preservative, binder and the like, and may be mixed as a unit dosage form required in generally-admitted pharmaceutical preparation, thereby producing pharmaceutical compositions containing it.

The additive capable of being mixed in the tablets and capsules are, for example, binder such as gelatin, corn starch, tragacanth gum, gum arabic; excipient such as crystalline cellulose; expander such as corn starch, gelatin, alginic acid; lubricant such as magnesium stearate; sweetener such as sucrose, lactose, saccharin; flavor such as peppermint, akamono oil and cherry. The capsules as preparation unit forms can contain liquid carriers such as fats and oils in addition to the above materials. The germ-free compositons for injection may be formulated according to ordinary pharmaceutical preparation using a vehicle such as distilled water for injection.

The aqueous solution for injection includes, for example, isotonic liquid containing physiological saline water, glucose and any other auxiliary agent, for example, D-sorbitol, D-mannose, D-mannitol, sodium chloride; and it may be combined with a suitable dissolution promoter, for example, alcohol, concretely ethanol, polyalcohol such as propylene glycol, polyethylene glycol, as well as nonionic surfactant, such as Polysorbate 80^{(™)}, HCO-50.

The oily liquid includes sesame oil, soybean oil, and it may be combined with benzyl benzoate or benzyl alcohol serving as a dissolution promoter. In addition, the composition may further contain buffer, such as phosphate buffer, sodium acetate buffer; analgesic agent such as procaine chloride; stabilizer such as benzyl alcohol, phenol; and antioxidant. Thus prepared, the injection is generally filled in suitable ampoules.

Administration to patients may be effected in any method known to those skilled in the art, for example, as intra-arterial injection, intravenous injection or subcutaneous injection, or intranasal, transbronchial, intramuscular, percutaneous or oral administration. The dose may vary, depending on the body weight and the age of patients, and the administration route, but may be any suitable one known to those skilled in the art. When the compound is capable of being encoded by DNA, the DNA may be inserted into a vector for gene therapy and may be used for gene therapy. The dose and the administration method may vary depending on the body weight, the age and the condition of patients, but may be suitably selected by anyone skilled in the art.

Though varying depending on the condition, the compound dose may be as follows: In oral administration, the dose may be from about 0.1 to 100 mg a day, preferably from about 1.0 to 50 mg, more preferably from 1.0 to 20 mg to an adult (having a body weight of 60 kg).

In parenteral administration, the dose may differ depending on the object for administration, the targeted organ, the condition and the administration route. For example, as an injection, the dose may be generally from about 0.01 to 30 mg a day, preferably from about 0.1 to 20 mg, more preferably from about 0.1 to 10 mg or so to an adult (having a body weight of 60 kg), and intravenous injection with the dose may be more advantageous.

### (3) Method of Molecular Diagnosis of Cancer:

The expression level or the activity of PKC-iota in a test tissue is measured, and the data are compared with the expression level or the activity of PKC-iota in a normal tissue, whereby the test tissue can be estimated or determined as to whether or not it is under oncogenic transformation.

The first molecular diagnostic method for cancer of the invention is characterized by comprising a step of measuring the expression level of the PKC-iota gene in a test tissue or a test cell, a step of comparing that expression level and the expression level of the PKC-iota gene in a normal tissue or a normal cell, and, as a result of the comparison, a step of determining as to whether or not the expression level of the PKC-iota gene in the test tissue or the test cell is significantly larger than the expression level of the PKC-iota gene in the normal tissue or the normal cell.

In the first molecular diagnostic method for cancer of the invention, first, the expression level of the PKC-iota gene in a test tissue or a test cell is measured. The method for measuring the expression level of the gene is not specifically defined. For example, employable are a method of RT-PCR using the mRNA extracted from a test tissue or a test cell, as a template; a method of using a microarray plotted with the gene; and a northern blotting method. The "expression level" of gene as referred to herein is meant to indicate the absolute amount or the relative amount of the gene transcriptional product. Regarding the relative amount, the expression level of the gene may be determined in relative comparison with the expression level thereof in a normal tissue as mentioned hereinunder.

Next, the gene expression level measured according to the above method is compared with the expression level of the corresponding gene in a normal tissue or a normal cell.

"Normal tissue or normal cell" as referred to herein is not specifically defined in point of its origin, so far as it is a tissue or a cell of a subject to be compared with the test tissue or the test cell; and it may be a healthy person-derived one, or a cancer patient-derived one. It may be a normal tissue or a normal cell existing around a cancer tissue.

In this step, the expression level of the PKC-iota gene expressed in the test tissue or cell is compared with the expression level of the PKC-iota gene (corresponding gene) expressed in the normal tissue or cell, for which, the absolute amount of the expression level may be compared with each other, or the relative value may be computed through comparison.

Next, as a result of the comparison, it is determined whether or not the expression level of the gene in the test tissue or the test cell is significantly higher than that of the gene in the normal tissue or the normal cell.

The method for determination of the significant difference is not specifically defined, for which employable is a statistical test method known to those skilled in the art.

As a result of comparison between the expression level of the gene in the test tissue or the test cell and that in the normal tissue or the normal cell, in case where the expression in the test tissue or the test cell is recognized higher with a significant difference therebetween, then it may be determined that there may be a high possibility of mutation in the expression level of P53 or PI3 kinase in the test tissue or the test cell, and it may be therefore determined that there is a possibility of oncogenic transformation in the tissue or the cell owing to mutation of p53 or PI3 kinase.

The second molecular diagnostic method for cancer of the invention is characterized by comprising a step of measuring the activity of PKC-iota in a test tissue or a test cell, a step of comparing that activity and the activity of PKC-iota in a normal tissue or a normal cell, and, as a result of the comparison, a step of determining as to whether or not the activity of PKC-iota in the test tissue or the test cell is significantly larger than the activity of PKC-iota in the normal tissue or the normal cell.

It is known that PKC-iota activates through self-phosphorylation in cells. Specifically, by measuring the phosphorylation condition of PKC-iota, the activation condition thereof can be known.

In the second molecular diagnostic method for cancer of the invention, first, the PKC-iota activity in a test tissue or a test cell is measured. The method for measuring the activity is not specifically defined. For example, PKC-iota existing in the extract of the test tissue or the test cell is isolated and is used, or the extract itself is used. This is phosphorylated with a suitable substrate in a solvent, and the activity is measured. In this case, the enzymatic reaction can be confirmed by the intake of the radiolabeled or fluorescence-labeled phosphoric acid or the like.

Next, the activity of PKC-iota measured according to the above method is compared with the activity of PKC-iota in a normal tissue or a normal cell.

As a result of the activity comparison, it is determined as to whether or not the PKC-iota activity in the test tissue or the test cell is significantly stronger than the PKC-iota activity in the normal tissue or the normal cell.

The method for determination of the significant difference is not specifically defined, for which employable is a statistical test method known to those skilled in the art.

As a result of comparison between the PKC-iota activity in the test tissue or the test cell and that in the normal tissue or the normal cell, in case where the PKC-iota activity in the test tissue or the test cell is recognized higher with a significant difference therebetween, then it may be determined that there may be a high possibility of mutation in the function or the expression level of P53 or PI3 kinase in the test tissue or the test cell, and it may be therefore determined that there is a possibility of oncogenic transformation in the tissue or the cell owing to mutation of p53 or PI3 kinase.

### (4) Gene Marker:

The gene marker of the invention is a gene marker for estimating or determining the potency of a PKC-iota inhibitor, and is **characterized in that** the gene is at least one selected from a group consisting of BAD, LLGL2, HMGB3, NFIB, PXDN, CLDN12, RPIA, AKT3 and SNF1LK, or a gene having substantially the same function as that of the gene.

The "gene marker" of the invention is one that can be an index to the potency of a PKC-iota inhibitor to be analyzed and the effect thereof to bodies; and concretely, it includes a gene or its related substances (e.g., DNA and RNA and their fragments) of which the expression level and the activity vary depending on the effect of the PKC-iota inhibitor thereto.

The gene marker of the invention also includes polynucleotides comprising a part of BAD, LLGL2, HMGB3, NFIB, PXDN, CLDN12, RPIA, AKT3 or SNF1LK. The polynucleotides may be RNA transcribed from a BAD, LLGL2, HMGB3, NFIB, PXDN, CLDN12, RPIA, AKT3 or SNF1LK gene, or cDNA produced from it, or synthetic nucleic acids having a sequence derived from these genes.

The present inventors have confirmed the expression of the gene marker of the invention increases or decreases by inhibiting the expression of PKC-iota. Specifically, by inhibiting the expression of PKC-iota, the expression of three genes of SNF1 LK, CLDN 12 and RPIA increases and the expression of four genes of HMGB3, NFIB, PXDN and AKT3 decreases. The present inventors have also found that the 112th, 136th and 155th amino acids of BAD, or the 653rd serine of LLGL2 is phosphorylated dependently of the kinase activity of PKC-iota; and therefore, by inhibiting the activity of PKC-iota, BAD and/or LLGL2 are not phosphorylated, or are dephosphorylated. Accordingly, the phosphorylation condition of BAD and/or LLGL2 is also usable as the gene marker for the PKC-iota inhibitor. (Detection of phosphorylation of BAD and LLGL2 is described in the section of (5) protein marker.)

The expression of the gene marker of the invention increases or decreases in a biological tissue, depending on a PKC-iota inhibitor thereto. In case where the potency of a PKC-iota inhibitor is estimated or determined, the expression level of the gene marker in any desired tissue where the gene marker has been expressed may be measured; and from the viewpoint of easy sample availability, the expression level in blood or skin tissue is preferably measured.

The method for measuring the expression level of the gene marker of the invention is not specifically defined. Concretely, for example, the expression level may be determined by measuring the amount of mRNA according to a northern blotting method, a quantitative RT-PCR method, or a DNA microarray method.

The probe to be used in the northern blotting method may be any probe capable of detecting the gene marker of the invention, a BAD, LLGL2, HMGB3, NFIB, PXDN, CLDN12, RPIA, AKT3 or SNF1LK gene. Concretely, a partial sequence or a complete sequence of the base sequence of these genes may be used. The number of the bases to constitute the probe is not also specifically defined. Preferably, the probe comprises a nucleic acid having a length of at least 20 continuous bases, more preferably at least 40 bases, even more preferably at least 60 bases, still more preferably at least 80 bases. If desired, the probe may be optionally labeled for detection. Concretely, for example, it may be labeled with a radioactive isotope such as ³²P, ¹⁴C, ¹²⁵I, ³H, ³⁵S, or may be labeled with biotin, fluorescent dye, enzyme, gold colloid or the like.

The primer to be used in the above quantitative RT-PCR method may be any primer capable of detecting the gene marker of the invention, BAD, LLGL2, HMGB3, NFIB, PXDN, CLDN12, RPIA, AKT3 or SNF1LK gene; and the number of the bases constituting it is not specifically defined but may be suitably determined depending on the base sequence of the primer, the base sequence of the gene to be isolated, etc. In general, it comprises preferably from 10 to 60 continuous bases, more preferably from 15 to 30 bases. The base sequence is determined based on the base sequence of the BAD, LLGL2, HMGB3, NFIB, PXDN, CLDN12, RPIA, AKT3 or SNF1LK gene to be detected.

In case where the expression level of the gene marker of the invention is measured according to the above DNA microarray method, a DNA microarray in which at least one of BAD, LLGL2, HMGB3, NFIB, PXDN, CLDN12, RPIA, AKT3 and SNF1LK genes or a partial nucleic acid of the gene is spotted, is prepared, and used for the measurement.

The gene marker of the invention is used for estimating and determining the potency of a PKC-iota inhibitor. Specifically, a PKC-iota inhibitor is administered to a living body, then the expression level of the gene marker therein is measured; and in case where the expression level has increased or decreased as compared with the expression level before the administration, it may be recognized that the inhibitor acts specifically to PKC-iota, therefore exhibiting a predetermined potency. Specifically, in case where the expression level has increased for three genes of SNF1LK, CLDN12 and RPIA, and in case where the expression level had decreased for four genes of HMGB3, NFIB, PXDN and AKT3, it may be recognized that the PKC-iota inhibitor exhibits a predetermined potency. Concretely, for the three genes of SNF 1 LK, CLDN12 and RPIA, when the expression level of the gene marker of the invention has increased after the administration of the PKC-iota inhibitor, it may be anticipated that the PKC-iota inhibitor can exhibit its potency even though the potency of the inhibitor could not be on a visible level (for example, for relieving cancer symptom) or even in a case where the cancer tissue is extremely small and its diagnosis is difficult according to a conventional diagnostic method such as X-ray photography. On the other hand, for four genes of HMGB3, NFIB, PXDN and AKT3, in case where the expression level of the gene marker of the invention has decreased after the administration of the PKC-iota inhibitor, then the potency of the inhibitor may also be anticipated. When the clinical test in a stage of developing a PKC-iota inhibitor as a pharmaceutical agent is directed to healthy persons, the potency of the pharmaceutical agent can be evaluated based on the expression level of the gene marker of the invention.

The gene marker of the invention can also be used in determining the healing result of cancer. Specifically, in case where a PKC-iota inhibitor is administered to a living body as an anticancer agent, it must be confirmed that the cancer tissue has actually reduced or the number of the cancer cells has actually reduced in order to confirm as to whether or not the anticancer agent could be effective. The confirmation requires examination with exposure to radiations such as X-ray tomography or X-ray imaging photography, or requires examination with load to patients such as endoscopy or biopsy. However, the gene marker of the invention requires only a simple examination of such that only an extremely small amount of a part of a body tissue such as blood or skin is collected, and the expression level of the gene marker in the tissue is measured. Accordingly, in place of the examination with X-ray tomography, X-ray imaging photography, endoscopy or biopsy, or as a preliminary examination for the examination, the potency of a PKC-iota inhibitor can be determined with neither pain nor load given to patients.

### (5) Protein Marker:

The protein marker of the invention is a protein marker for estimating or determining the potency of a PKC-iota inhibitor, and is **characterized in that** the protein is at least one selected from a group consisting of BAD, LLGL2, HMGB3, NFIB, PXDN, CLDN12, RPIA, AKT3 and SNF1LK, or one having substantially the same function as that of the protein.

The "protein marker" of the invention is one that can be an index to the potency of a PKC-iota inhibitor to be analyzed and the effect thereof to bodies; and concretely, it includes a protein or its related substances (e.g., partial peptides) of which the expression level and the activity vary depending on the effect of the PKC-iota inhibitor thereto.

The present inventors have confirmed that the expression level of not only the gene coding for those proteins but also the protein itself increases or decreases by administration of a PKC-iota inhibitor.

The expression level of the protein marker of the invention increases or decreases in the tissue of a living body by administration of a PKC-iota inhibitor. In case where the potency of a PKC-iota inhibitor is estimated or determined, the expression level of the protein marker in a desired tissue in which the protein marker has been expressed may be determined; and from the viewpoint of easy sample availability, the expression level in blood or skin tissue is preferably measured.

The protein marker of the invention includes a protein having substantially the same function as that of at least one protein selected from a group consisting of BAD, LLGL2, HMGB3, NFIB, PXDN, CLDN12, RPIA, AKT3 and SNF1LK. The protein includes those derived from a protein of BAD, LLGL2, HMGB3, NFIB, PXDN, CLDN12, RPIA, AKT3 or SNF1LK through substitution, addition, deletion or insertion at one or more amino acids constituting the amino acid sequence of the protein, having the activity on the same level. The activity on the same level means as follows: The protein having substantially the same function as that of BAD is a protein having a BAD activity; the protein having substantially the same function as that of LLGL2 is a protein having an LLGL2 activity; the protein having substantially the same function as that of HMGB3 is a protein having an HMGB3 activity; the protein having substantially the same function as that of NFIB is a protein having an NFIB activity; the protein having substantially the same function as that of PXDN is a protein having a PXDN activity; the protein having substantially the same function as that of CLDN12 is a protein having a CLDN12 activity; the protein having substantially the same function as that of RPIA is a protein having an RPIA activity; the protein having substantially the same function as that of AKT3 is a protein having an AKT3 activity; the protein having substantially the same function as that of SNF1LK is a protein having an SNF1LK activity.

The method for measuring the expression level of the protein marker of the invention is not specifically defined. Concretely, for example, the expression level may be determined by measuring the amount by mass of the protein produced, according to a western blotting method or an ELISA method or by the use of a protein chip.

The western blotting method may be attained according to a method known to those skilled in the art. Concretely, for example, the total protein of the tissue collected from a living body to which a PKC-iota inhibitor has been administered is developed in SDS-PAGE, and transferred onto a nitrocellulose membrane, a PVDF membrane or the like. Next, an antibody to the protein marker of the invention is added to and reacted with the membrane, and further a secondary antibody is added to and reacted with it, thereby detecting the secondary antibody to detect the protein marker. The total protein of the tissue collected from the living body may be immunized and precipitated with an antibody to the protein marker, then the protein obtained as a precipitate may be developed in SDS-PAGE, and may be detected according to a western blotting method.

The antibody to be used in the western blotting method may be any antibody capable of detecting the protein marker of the invention, a protein of BAD, LLGL2, HMGB3, NFIB, PXDN, CLDN12, RPIA, AKT3 or SNF1LK, and it may be a polyclonal antibody or a monoclonal antibody. The polyclonal antibody may be prepared according to a method known to those skilled in the art, and for example, it may be prepared according to the method mentioned below. Specifically, for example, a mammal of mouse, rat, hamster, guinea pig, rabbit or the like is immunized with an antigen optionally along with a Freund's adjuvant, and the intended polyclonal antibody may be collected from the serum of the thus-immunized animal.

The monoclonal antibody may also be prepared as follows: For example, a mammal of mouse, rat, hamster, guinea pig, rabbit or the like is immunized with an antigen optionally along with a Freund's adjuvant. Next, an antibody-producing cell collected from the immunized animal is hybridized with a myeloma cell not having autoantibody producibility, thereby preparing a hybridoma (fused cell); then the hybridoma is cloned, and a clone that produces a monoclonal antibody having a specific affinity to the antigen used for the mammal immunization is selected, thereby preparing the intended monoclonal antibody. Further concretely, an antigen is injected or transplanted subcutaneously, intramuscularly, intravenously or intraabdominally once or a few times into a mammal optionally along with a Freund's adjuvant for immunization. In general, the immunization is effected once to four times or so at intervals of from 1 to 14 days from the first immunization, and after 1 to 5 days or so from the final immunization, an antibody-producing cell is collected from the immunized mammal.

The hybridoma (fused cell) of secreting a monoclonal antibody may be prepared according to a conventional method. Specifically, the antibody-producing cell in the spleen, the lymph node, the bone marrow, the tonsil or the like, preferably in the spleen collected from the mammal thus immunized according to the above-mentioned method is fused with a myeloma cell derived from mouse, rat, human or the like and not having autoantibody producibility, thereby preparing a hybridoma. The hybridoma clone capable of producing a monoclonal antibody may be screened as follows: The hybridoma is cultured on a micro-titer plate or the like, and the reactivity to immunogen of the culture supernatant in the well in which the hybridoma cell has grown is measured, for example, according to enzyme immunoassay such as RIA or ELISA.

A monoclonal antibody may be produced from the hybridoma, as follows: The hybridoma is cultured in-vitro, or in-vivo in the ascites or the like of mouse, rat, guinea pig, hamster, rabbit or the like, and then the intended monoclonal antibody may be isolated from the resulting culture supernatant or from the ascites of the mammal. The monoclonal antibody may be isolated and purified by processing the above-mentioned culture supernatant or ascites through saturated ammonium sulfate treatment, euglobulin precipitation, caproic acid treatment, caprylic acid treatment, ion-exchange chromatography (DEAE, DE52, etc.), or affinity column chromatography with an antiimmunoglobulin column, a protein A column or the like.

Using the polyclonal antibody or the monoclonal antibody produced according to the above-mentioned method, an antibody array may be prepared. Specifically, the antibody may be fixed on a membrane such as a nitrocellulose membrane or a PVDF membrane, or on a nitrocellulose-coated slide or glass substrate, using a microarray spotter.

BAD and LLGL2 are phosphorylated with PKC-iota. Accordingly, the phosphorylation condition of BAD and LLGL2 can be an index of the activity of PKC-iota, or that is, a marker for it. BAD phosphorylation can be detected according to a western blotting method using an anti-phosphorylation BAD antibody. Another method is as follows: A BAD band is confirmed through western blotting, using the anti-BAD antibody prepared according to the above-mentioned method, and then the band is further confirmed as to whether or not it can be detected with an anti-phosphorylation serine/threonine antibody, whereby the BAD phosphorylation can also be confirmed. LLGL2 can also be confirmed for its phosphorylation; and based on the LLGL2 phosphorylation as a marker, the PKC-iota inhibiting activity can also be detected.

On the other hand, at least one protein of BAD, LLGL2, HMGB3, NFIB, PXDN, CLDN12, RPIA, AKT3 and SNF1LK, the protein marker of the invention, may be fixed on a membrane such as a nitrocellulose membrane or a PVDF membrane, or on a nitrocellulose-coated substrate or glass substrate, using a microarray spotter, thereby preparing a protein array. In this case, the protein may be fixed directly, or may be fixed via a linker held between the protein and the membrane or the substrate. Fixation via a linker makes it possible to prepare a protein array (microarray) having the activity of the protein not detracting from the stereostructure thereof.

The protein marker of the invention may be used for estimating or determining the potency of a PKC-iota inhibitor. Specifically, after a PKC-iota inhibitor has been administered to a living body, the expression level of the protein marker is measured, and in case where the expression level has increased or decreased as compared with the expression level before administration, then it may be recognized that the inhibitor acts specifically to PKC-iota and exhibits the predetermined potency. Specifically, in case where the expression level of three proteins, SNF1LK, CLDN12 and RPIA has increased, and in case where the expression level of four proteins, HMGB3, NFIB, PXDN and AKT3 has decreased, it may be recognized that the PKC-iota inhibitor exhibits the predetermined potency. Concretely, for the three proteins SNF1LK, CLDN12 and RPIA, when the expression level of the protein marker of the invention has increased after the administration of the PKC-iota inhibitor, then it may be anticipated that the PKC-iota inhibitor can exhibit its potency even though the potency of the inhibitor could not be on a visible level (for example, for relieving cancer symptom) or even in a case where the cancer tissue is extremely small and its diagnosis is difficult according to a conventional diagnostic method such as X-ray photography. On the other hand, for the four proteins, HMGB3, NFIB, PXDN and AKT3, in case where the expression level of the protein marker of the invention has decreased after the administration of the PKC-iota inhibitor, then the potency of the inhibitor may also be anticipated. When the clinical test in a stage of developing a PKC-iota inhibitor as a pharmaceutical agent is directed to healthy persons, the potency of the pharmaceutical agent can be evaluated based on the expression level of the protein marker of the invention.

The protein marker of the invention can also be used in determining the healing result of cancer. Specifically, in case where a PKC-iota inhibitor is administered to a living body as an anticancer agent, it must be confirmed that the cancer tissue has actually reduced or the number of the cancer cells has actually reduced in order to confirm as to whether or not the anticancer agent could be effective. The confirmation requires examination with exposure to radiations such as X-ray tomography or X-ray imaging photography, or requires examination with load to patients such as endoscopy or biopsy. However, the protein marker of the invention requires only a simple examination of such that only an extremely small amount of a part of a body tissue such as blood or skin is collected, and the expression level of the protein marker in the tissue is measured. Accordingly, in place of the examination with X-ray tomography, X-ray imaging photography, endoscopy or biopsy, or as a preliminary examination for the examination, the potency of a PKC-iota inhibitor can be determined with neither pain nor load given to patients.

### (6) Cancer Diagnostic Kit:

The cancer diagnostic kit of the invention is characterized by containing an antibody capable of detecting the protein marker of the invention. The antibody is a polyclonal antibody or a monoclonal antibody to at least one protein selected from a group consisting of BAD, LLGL2, HMGB3, NFIB, PXDN, CLDN12, RPIA, AKT3 and SNF1LK mentioned in the above, or to a protein having substantially the same function as that of the former.

The cancer diagnostic kit of the invention may be provided with, for example, a reagent for antibody detection, a secondary antibody for antibody detection and a plate for reaction, in addition to the antibody capable of detecting the protein marker of the invention.

Using the cancer diagnostic kit of the invention makes it possible to rapidly and simply detect a protein marker in a tissue (for example, blood, skin) collected from a living body to which a PKC-iota inhibitor has been administered; and by comparing the protein amount by mass before and after the administration, the potency of the PKC-iota inhibitor can be thereby estimated or determined.

### (7) Method for Estimating or Determining the Effect of PKC-iota Inhibitor:

First described is the first method for estimating or determining the effect of a PKC-iota inhibitor of the invention.

The first method for estimating or determining the effect of a PKC-iota inhibitor of the invention is characterized by comprising a detection step of detecting at least one gene selected from a group consisting of HMGB3, NFIB, PXDN, CLDN12, RPIA, AKT3 and SNF1LK or a gene having substantially the same function as that of the gene in the tissue derived from a test subject to which a PKC-iota inhibitor has been administered, and a step of comparing the expression level before and after the PKC-iota inhibitor administration.

The detection step in the invention is a step of detecting at least one gene selected from a group consisting of HMGB3, NFIB, PXDN, CLDN12, RPIA, AKT3 and SNF1LK or a gene having substantially the same function as that of the gene (that is, the gene marker of the invention) in the tissue derived from a test subject to which a PKC-iota inhibitor has been administered.

"Tissue" in the invention means the tissue derived from a subject to which a PKC-iota inhibitor has been administered, and may be any one in which the gene marker to be detected is expressed; and its type is not specifically defined. However, from the viewpoint of easy sample availability, the tissue is preferably blood or skin tissue.

In this step, the tissue is collected from a living body to which a PKC-iota inhibitor has been administered, and a DNA for gene marker detection is extracted from it according to a conventional method. The DNA to be extracted may be a genome DNA, or may also be a cDNA obtained from the extracted RNA through reverse transcription. Next, obtained DNA is used for detection of a gene HMGB3, NFIB, PXDN, CLDN12, RPIA, AKT3 or SNF1LK. Not specifically defined, the detection method may be any quantitative one, concretely, for example, includes PCR or microarray.

After a gene HMGB3, NFIB, PXDN, CLDN12, RPIA, AKT3 or SNF1LK has been detected in the detection step, the step is then followed by the comparing step of comparing the expression level before and after the PKC-iota inhibitor administration. In the comparing step, the expression level of the gene HMGB3, NFIB, PXDN, CLDN12, RPIA, AKT3 or SNF1LK detected in the detection step is compared with the expression level of that gene before administration of the PKC-iota inhibitor. As a result of the comparison, in case where the expression of three genes SNF1LK, CLDN12 and RPIA has increased as a result of the PKC-iota inhibitor administration, or in case where the expression of four genes HMGB3, NFIB, PXDN and AKT3 has decreased, then it may be determined that the PKC-iota inhibitor administered to the administration subject, human body, acts on the human body according to a suitable mechanism process, and exhibits its administration effect thereto. The method is extremely advantageous as a method for determining the potency of a PKC-iota inhibitor in case where a cancer tissue is extremely small or where the potency of the inhibitor could hardly be determined on the basis of the physical size of a cancer tissue as in the stage of a clinical test in drug development.

Next described is the second method for estimating or determining the effect of a PKC-iota inhibitor of the invention.

The second method for estimating or determining the effect of a PKC-iota inhibitor of the invention is characterized by comprising a detection step of detecting at least one protein selected from a group consisting of HMGB3, NFIB, PXDN, CLDN12, RPIA, AKT3 and SNF1LK or a protein having substantially the same function as that of the protein in the tissue derived from a test subject to which a PKC-iota inhibitor has been administered, and a comparing step of comparing the expression level before and after the PKC-iota inhibitor administration.

The detection step in the invention is a step of detecting at least one protein selected from a group consisting of HMGB3, NFIB, PXDN, CLDN12, RPIA, AKT3 and SNF1LK or a protein having substantially the same function as that of the protein (that is, the protein marker of the invention) in the tissue derived from a test subject to which a PKC-iota inhibitor has been administered.

"Tissue" in the invention means the tissue derived from a subject to which a PKC-iota inhibitor has been administered, and may be any one in which the protein marker to be detected is expressed; and its type is not specifically defined. However, from the viewpoint of easy sample availability, the tissue is preferably blood or skin tissue.

In this step, the tissue is collected from a living body to which a PKC-iota inhibitor has been administered, and a protein for protein marker detection is extracted from it according to a conventional method, or a total protein including the protein is collected. Concretely, for example, for detecting according to a western blotting method, a total protein including the protein is prepared as a solubilized sample, and this is may be used as the extract protein in this step. The total protein may be processed for immunoprecipitation using an antibody to the protein to be detected, whereby the protein alone may be extracted.

Next, the obtained protein is processed for detection of the intended protein HMGB3, NFIB, PXDN, CLDN12, RPIA, AKT3 or SNF1LK. Not specifically defined, the detection method may be any quantitative one. Concretely, for example, it includes a western blotting method and an ELISA method. Detection according to a western blotting method and an ELISA method requires an anti-HMGB3 antibody, an anti-NFIB antibody, an anti-PXDN antibody, an anti-CLDN12 antibody, an anti-RPIA antibody, an anti-AKT3 antibody or an anti-SNF1LK antibody. The antibody may be a polyclonal antibody or a monoclonal antibody. The antibody may be prepared according to a conventional method, and for example, it may be prepared according to the method described hereinabove in the section of (5) protein marker.

BAD and LLGL2 are phosphorylated with PKC-iota. Accordingly, the phosphorylation condition of BAD and LLGL2 can be an index of the activity of PKC-iota, or that is, a marker for it. BAD phosphorylation can be detected according to a western blotting method using an anti-phosphorylatied BAD antibody. Another method is as follows: A BAD band is confirmed through western blotting, using the anti-BAD antibody prepared according to the above-mentioned method, and then the band is further confirmed as to whether or not it can be detected with an anti-phosphorylatied serine/threonine antibody, whereby the BAD phosphorylation can also be confirmed. LLGL2 can also be confirmed for its phosphorylation; and based on the LLGL2 phosphorylation as a marker, the PKC-iota inhibiting activity can also be detected.

After a protein HMGB3, NFIB, PXDN, CLDN12, RPIA, AKT3 or SNF1LK has been detected in the detection step, the step is then followed by the comparing step of comparing the expression level before and after the PKC-iota inhibitor administration. In the comparing step, the expression level of the protein HMGB3, NFIB, PXDN, CLDN12, RPIA, AKT3 or SNF1LK detected in the detection step is compared with the expression level of that protein before administration of the PKC-iota inhibitor. For BAD and LLGL2, their phosphorylation level is compared. As a result of the comparison, in case where the expression of three proteins SNF1LK, CLDN12 and RPIA has increased as a result of the PKC-iota inhibitor administration, or in case where the expression of four proteins HMGB3, NFIB, PXDN and AKT3 has decreased, then it may be determined that the PKC-iota inhibitor administered to the administration subject, human body, acts on the human body according to a suitable mechanism process, and exhibits its administration effect thereto. For BAD and LLGL2, in case where their phosphorylation level has decreased, then it may be determined that the PKC-iota inhibitor acts according to a suitable mechanism process, and exhibits its administration effect. The method is extremely advantageous as a method for determining the potency of a PKC-iota inhibitor in case where a cancer tissue is extremely small or where the potency of the inhibitor could hardly be determined on the basis of the physical size of a cancer tissue as in the stage of a clinical test in drug development.

### EXAMPLES

The invention is described more concretely with reference to the following Examples, to which, however, the invention should not be limited.

### Example 1

### (Identification of kinase of which the activity inhibition is specifically cytotoxic to cells having mutant p53)

First, a mutant p53 expression induction cell was prepared. A plasmid for expression of a mutant p53 (where the 157th codon is mutated) was introduced into a wild-type p53-having U2OS Tet-on cell (hereinafter referred to a U2OS WTp53), thereby establishing a mutant p53 expression inducing cell (hereinafter referred to as U2OS MT157). U2OS MT157 can express a mutant p53 through doxycycline addition thereto.

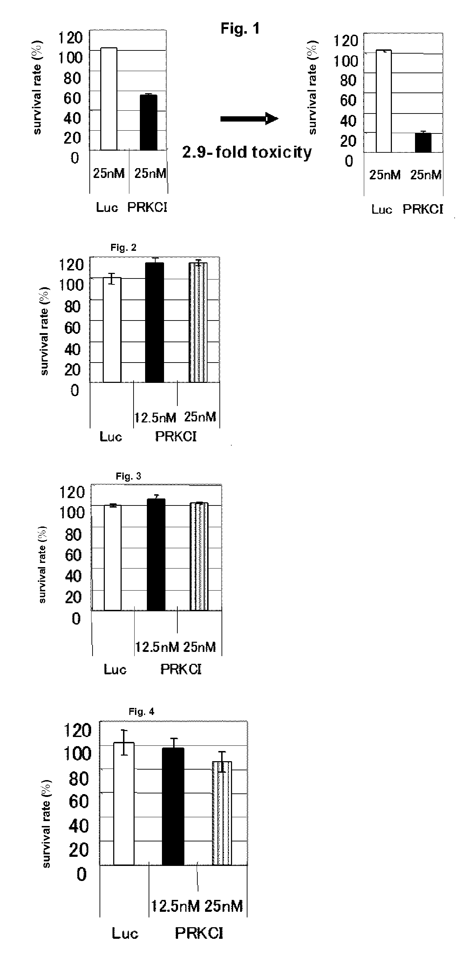

Next, the established cell was evaluated for siRNA transfection and cytotoxicity. Specifically, a kinome wide siRNA was transfected to U2OS WTp53 cells and U2OS mtp53 cells incubated in the presence of doxycycline. After 72 hours, the number of the cells was counted, using WST-8 (by Kishida Chemical); and the cytotoxicity effect by the phosphorylase inhibition was determined. The phosphorylase of which the cytotoxic effect to U2OS mtp53 cells could be at least 1.5 times higher than that to U2OS WTp53 was selected for a mutant p53 context specific target gene. This included PKC-iota. As in Fig. 1, the PKC-iota inhibition shows specific cytotoxicity to mutant p53-having cells.

### Example 2

### (PKC-iota inhibition effect with lung cancer cell line)

An siRNA of PKC-iota was transfected to four types of cells (A427, A549, LU99 and NCI-H460) known to have a wild p53, and to four types of cells (HOP62, HOP92, NCI-H322M and NCI-H226) known to have a mutant p53. After 72 hours, the number of the cells was counted, using WST-8 (by Kishida Chemical); and the cytotoxicity effect by the phosphorylase inhibition was determined. The HOP62 cell has a mutation at the 212th codon; the HOP92 cell at the 175th codon; and the NCI-H226 cell at the 158th codon.

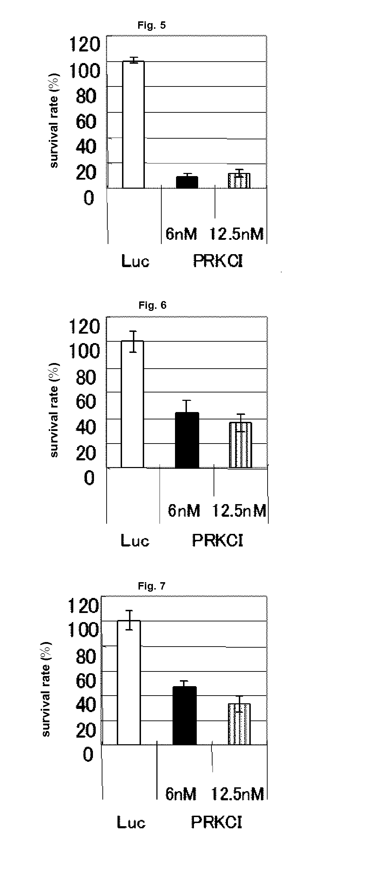

As in Figs. 2 to 9, the growth of all the mutant p53-having cells (Figs. 2 to 5) was inhibited by the PKC-iota expression inhibition; however, the growth of three of the four types of the wild-type p53-having cells (Figs. 6 to 9) was not almost inhibited. From this, it was considered that, in the lung cancer cell lines, the PKC-iota inhibition could be more effective to the mutant p53-having cells.

### Example 3

### (PKC-iota inhibition inhibits phosphorylation of BAD.)

For clarifying the growth inhibition mechanism by PKC-iota inhibition, the phosphorylation of BAD, which is known as a substrate for PKC-iota, with PKC-iota was investigated. Specifically, an siRNA of PKC-iota was transfected to U2OS WTp53 cells and U2OS mtp53 cells incubated in the presence of doxycycline. After 72 hours, the cells were collected, each cell solution was analyzed through western blotting, for the phosphorylation condition of BAD ser155 therein.

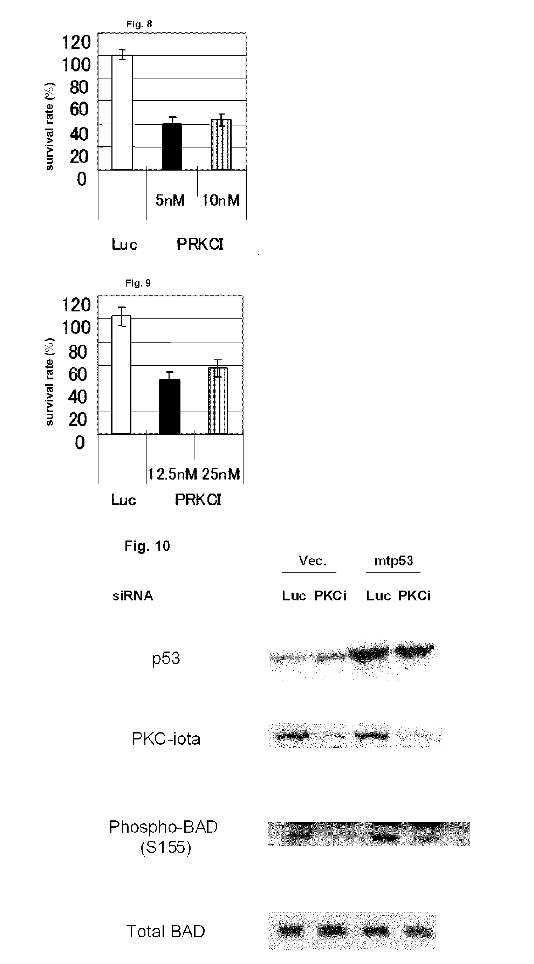

As in Fig. 10, in both of the U2OS WTp53 cells and the U2OS mtp53 cells, BAD Ser155 phosphorylation was inhibited by the PKC-iota inhibition. From this, it was confirmed that the PKC-iota inhibition results in dephosphorylation of BAD Ser155, therefore bringing about apoptosis.

### Example 4

### (Excessively expressed p53 binds to Bcl-XL.)

From a known literature report (J. Biol. Chem., Vol. 276, p. 40583, 2001), it is known that a mutant p53-having cell has a higher p53 expression level than a wild-type p53-having cell and that p53 binds to Bcl-XL and thereby inactivates it. Accordingly, for clarifying the mechanism that a mutant p53-having cell has a higher sensitivity to PKC-iota inhibition than a wild-type p53-having cell, the following test was carried out.

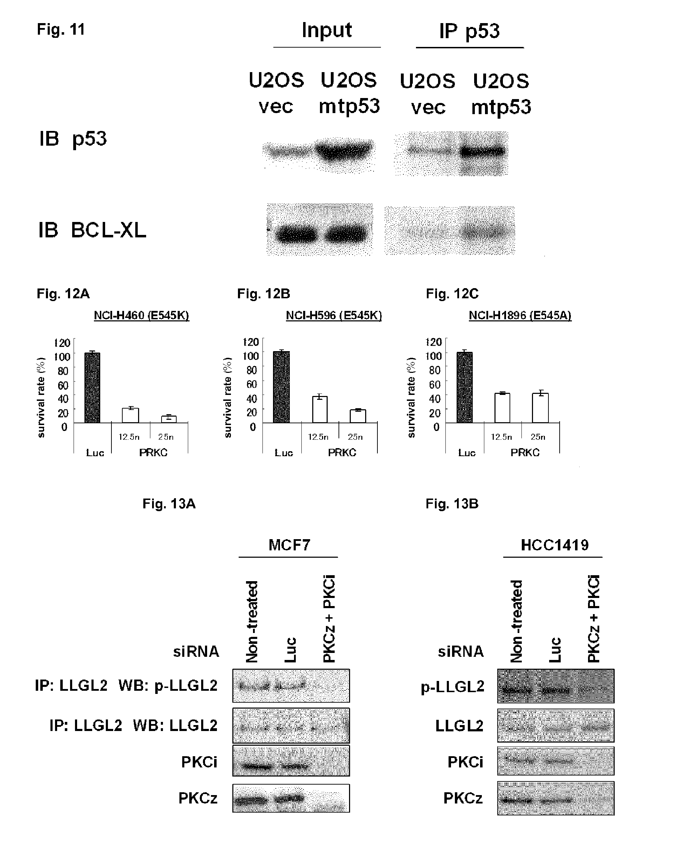

First, U2OS WT p53 cells and U2OS mt p53 cells incubated in the presence of doxycycline were collected. Next, an anti-p53 antibody and a protein A agarose were added to each cell solution, and centrifuged to thereby collect p53 and a p53-bound product. Thus collected, the p53-bound product was analyzed to determine the Bcl-X1 protein amount therein, according to a western blotting method (Fig. 11, IB).

As in Fig. 11, it is known that the Bcl-XL-binding amount increased in excessive p53 expression. From this, it is concluded that in the cells with excessive p53 expression therein, Bcl-XL is inactivated by the binding of p53 thereto, therefore readily bringing about apoptosis via mitochondria.

### Example 5

### (PKC-iota expression inhibition effect in cells with PI3 kinase pathway activated therein)

It is known that PKC-iota is activated with PI3 kinase. From this, it is anticipated that PKC-iota activity inhibition could bring about an effect of inhibiting the growth of cancer cells having an abnormality on the pathway. Accordingly, PI3 kinase-activated cells were tested for the effect of PKC-iota inhibition.

PKC-iota siRNA was introduced into three types of cells (NCI-H460, NCI-H596 and NCI-H1869) each having a mutation (E545K or E545A) in the helical domain of PI3 kinase. After 72 hours, the number of the cells was counted, using WST-8, to thereby determine the cytotoxicity effect by PKC-iota inhibition.

As in Fig. 12A (NCI-H460 cells), Fig. 12B (NCI-H596 cells) and Fig. 12C (NCI-H1869 cells), the growth of all those cells was inhibited by at least 40 %. From this, it was confirmed that the PKC-iota expression inhibition is more effective to PI3 kinase-activated cells; or that is, the PKC-iota inhibition is expected to be effective for inhibition of the growth of PI3 kinase activated cancer cells.

### Example 6

### (LLGL2 is a substrate for PKC-iota.)

An investigation was made as to whether or not the phosphorylation of LLGL2 could be inhibited by PKC-iota/PKC-zeta double-knockdown with siRNA.

A mixture solution of PKC-iota siRNA (mixture solution of GGUUCGAGACAUGUGUUCUTT: SEQ ID NO 21, GGAAGGAGACCCGUGUACATT: SEQ ID NO 22, and GGAGACCCGUGUACAGUAUTT: SEQ ID NO 23) and PKC-zeta siRNA; or luciferase as a negative control was introduced into MCF7 and HCC1419 cell lines, using siLentFect (by Bio-Rad). MCF7, after 72 hours, and HCC 1419, after 120 hours, were processed for siRNA, thereby preparing cell solutions. Using a BCA protein assay kit (by PIERCE), the protein amount by mass in each cell solution was measured and adjusted; and according to a western blotting method, the phosphorylated LLGL2(pS653), LLGL2, PKC-iota and PKC-zeta were detected. However, phospho-LLGL2(pS653) and LLGL2 in the MCF7 cell solution were detected through western blotting after immunoprecipitation with an LLGL2 antibody.

As in Fig. 13A (MCF7 cells) and Fig. 13B (HCC1419 cells), the western blotting confirmed the reduction in the protein amount in PKC-iota and PKC-zeta as a result of siRNA treatment of PKC-iota and PKC-zeta. In this case, the expression level of phosphorylated LLGL2 (pSer653) also significantly reduced as compared with the non-processed sample and the luciferase siRNA-processed sample. Specifically, it was confirmed that the phosphorylation of LLGL2 Ser653 depends on the expression of atypical PKC.

Next, for establishing a cell line capable of inducing PKC-iota expression with doxycycline, cells were prepared according to the following process.

First, PKC-iota, ordinary-activity mutants PKCi(A120E) and PKCi(K274W) were cloned in pTRE2hyg vector (hereinafter these are referred to as pTRE2hyg/PKCi-WT, pTRE2hyg/PKCi-CA and pTRE2hyg/PKCi-KD in that order, respectively). Next, the vetctors pTRE2hyg/PKCi-WT, pTRE2hyg/PKCi-CA, pTRE2hyg/PKCi-KD and pTRE2hyg were individually introduced into a U-2OS Tet-on cell line, using Nucleofector (by Amaxa) or Fugene 6 (by Roche). After 24 hours, the cells were collected and again seeded, and incubated for about 2 hours in the presence of hygromycin (150 µg/ml); and the appearing hygromycin-resistant cell colony was isolated according to a cylinder cloning method. Thus established, these cells are referred to as PKCi-WT Tet-on, PKCi-CA Tet-on, PKCi-KD Tet-on and Vector Tet-on U-2OS, respectively.

Next, LLGL2 that had been HA-tagged at its N terminal was cloned in a pcDNA3.1 vector (hereinafter referred to as pcDNA3.1/HA-LLGL2). The pcDNA3.1/HA-LLGL2 was introduced into PKCi-WT Tet-on, PKCi-CA Tet-on, PKCi-KD Tet-on and Vector Tet-on U-20S, using Fugene HD; and doxycycline was added for inducing the expression of PKC-iota. After 24 hours, solutions of these cells were prepared; and using a BCA protein assay kit, the protein amount in each cell solution was measured and adjusted; and then phosphorylated LLGL2 (pS653), LLGL2 (HA) and PKC-iota were detected according to a western blotting method.

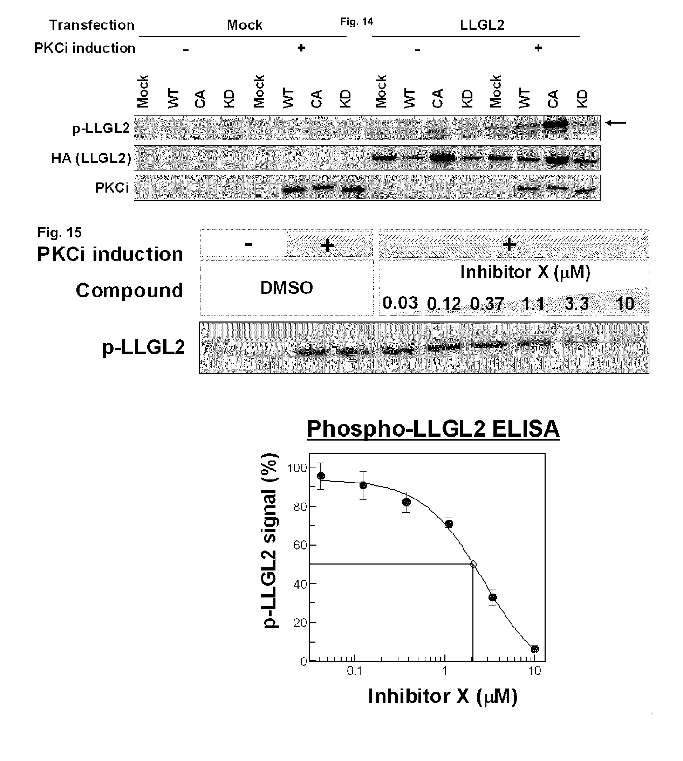

As in Fig. 14, the ordinary-activity mutant PKC-iota (PKCi-CA) expression induction by doxycycline significantly increased the band of phosphorylated LLGL2 (pSer653), which was confirmed according to a western blotting method. On the other hand, kinase dead PKCi (PKCi-KD) expression induction did not incerase the phosphorylation of LLGL2 Ser653. This experiment confirmed that the LLGL2 Ser653 phosphorylation is dependent on the kinase activity of PKC-iota.

The above two experiments confirmed that LLGL2 is a substrate for the downstream of PKC-iota.

### Example 7

### (Establishment of compound evaluation system with LLGL2 phosphorylation as index)

The following experiment is for constructing a system for evaluation of PKC-iota inhibitor, based on the phosphorylation of the 653rd serine (Ser653) of LLGL2 as an index.

First, for preparing cells for compound evaluation, pcDNA3.1/Zeo/HA-LLGL2 was introduced into PKCi-CA Tet-on U-2OS cells, using Fugene HD. After 48 hours, the cells were collected, seeded again, and incubated for about 2 weeks in the presence of zeocin (50 µg/ml); and the appearing zeocin-resistant cell colony was isolated according to a cylinder cloning method. Hereinafter, the thus-established cell is referred to as LLGL2 stable/PKCi-CA Tet-on U-20S.

Next, LLGL2 stable/PKCi-CA Tet-on U-2OS was incubated for 24 hours in the presence of doxycycline, and then further incubated along with a PKC-iota inhibitor X having a different concentration. During the PKC-iota inhibitor treatment, a serum-free culture was used. In 4 hours after the start of the PKC-iota inhibitor treatment, the cell solution was prepared, and analyzed for detection of phosphorylated LLGL2(pS653) therein according to western blotting and according to sandwich ELISA using an LLGL2 antibody and a phosphorylated LLGL2(pS653) antibody.

As in Fig. 15, the phosphorylation of LLGL2 Ser653 induced by the doxycycline addition was inhibited dependently on the PKC-iota inhibitor concentration, as confirmed according to the western blotting and the sandwich ELISA. Specifically, it was confirmed that the present experiment system is usable for PKC-iota inhibitor evaluation. In addition, it was confirmed that using sandwich ELISA in the inhibitor evaluation system can realize high-throughput screening.

### Example 8

### (Search for gene expression marker by microarray analysis)

This is to search for a gene to cause up-regulation or down-regulation of PKC-iota expression inhibited by siRNA.

For preparing cells to be used for the assay, first, one type of siRNA of PKC-iota was introduced into an HOP62 cell in a logarithmic growth phase. Two types of siRNAs of PKC-iota were introduced into an NCI-H596 cell in a logarithmic growth phase. In 16, 24 or 48 hours after the siRNA introduction, the cells were collected, and mRNA was extracted from each sample, using an RNeasy kit (by Qiagen).

Next, the mRNA expression level of each sample was measured, using Affmetrix GeneChip (by Affmetrix). The found data of the mRNA expression level were analyzed using Rosetta Resolver System (by Rosetta), thereby detecting a gene having given a significant expression change between the control (luciferase siRNA) treatment and the PKC-iota siRNA treatment. Specifically, the genes having given a significant expression change in both the HOP62 cell and the NCI-H596 cell were extracted, thereby searching for marker candidate genes capable of changing the mRNA expression level as associated with PKCi control.

As a result, common to the two types of the cells and to the two types of the siRNAs, 28 genes for expression up-regulation and 17 genes for expression down-regulation were found out.

Next, the genes with expression change were analyzed through microarray analysis.

First, RNA was extracted from cells of A549, NCI-H460, HOP62, NCI-H596 and U2OS, using an RNeasy kit. Next, cDNA was prepared from the obtained RNA, using SUPERSCRIPTIII PLATINUM TWO-STEP QRT-PCR (by Invitrogen). Through Taq-man RT-PCR using the thus-obtained cDNA as a template, the mRNA amount of the candidate gene in each cell was quantified.
From the genes having the expression change in the above analysis, marker candidate genes, in which the expression change is statistically significant, were selected based on the PKCi-regulating PI3K pathway and the PKCi-related cell polarity-associated factor.

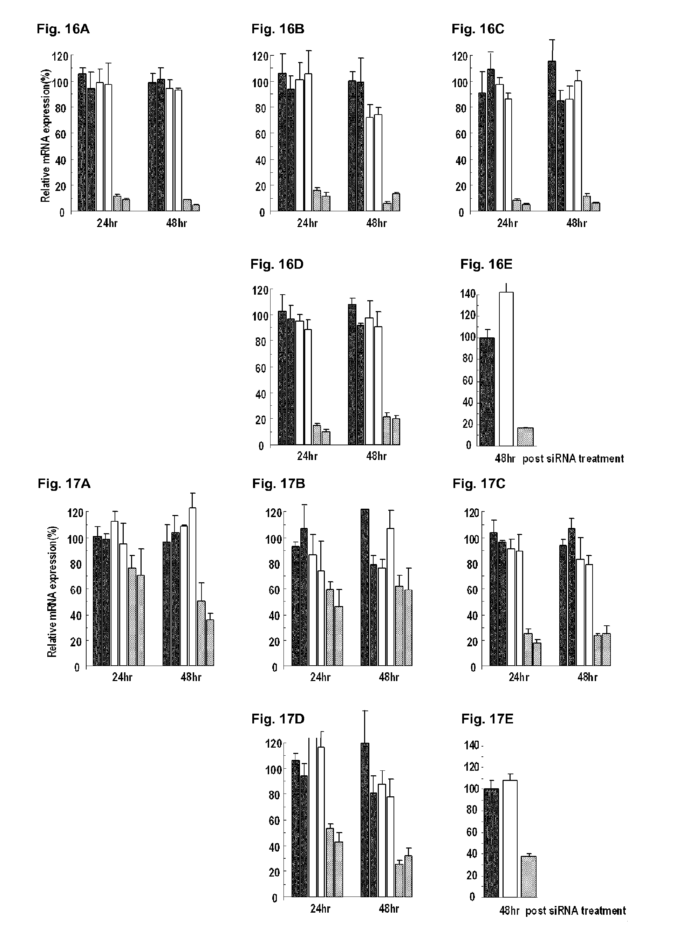

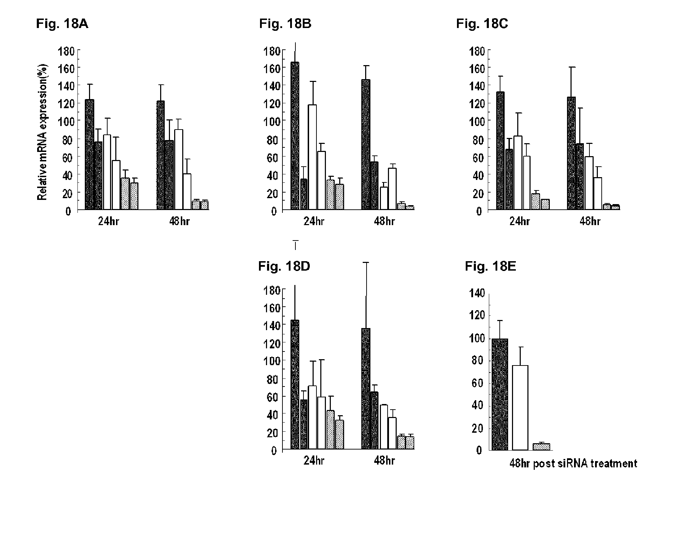

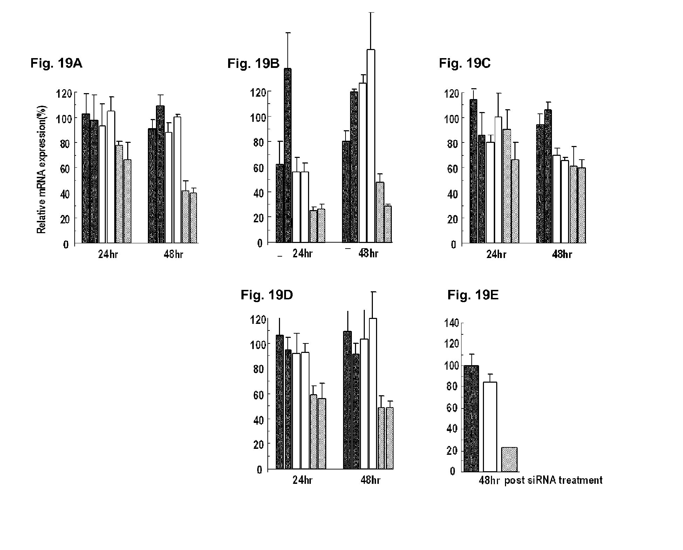

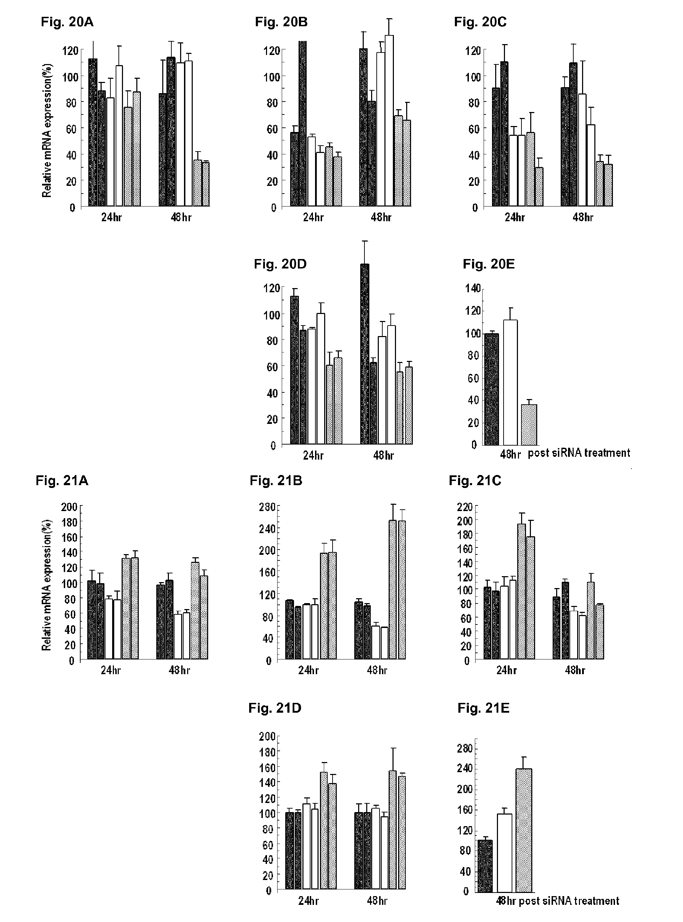

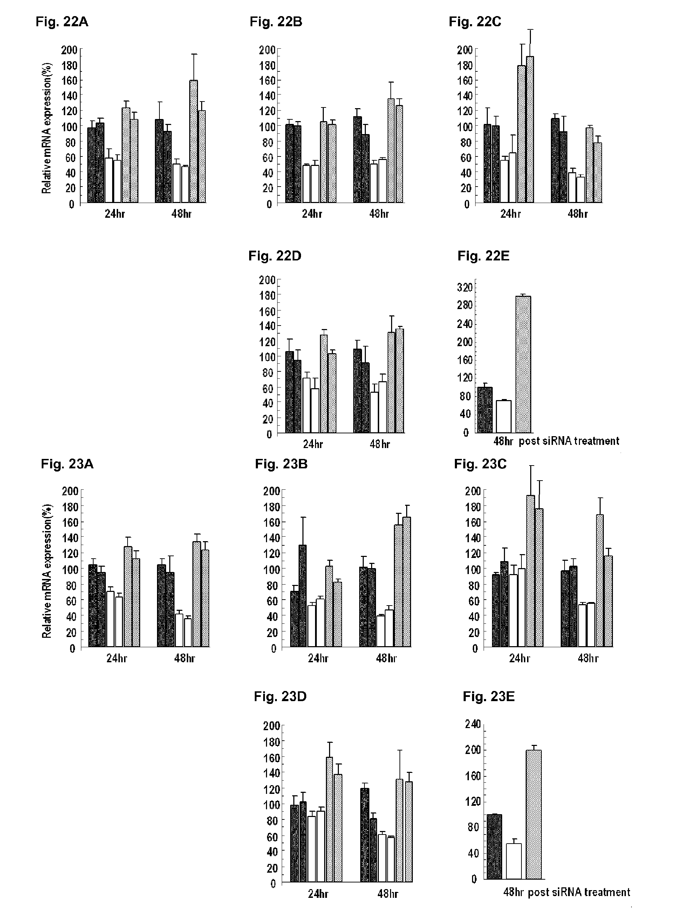

As in Figs. 16 to 23, it was confirmed that, in case where the PKC-iota expression was inhibited, the three genes of SNF1LK, CLDN12 and RPIA were in up-regulation in all the five types of the tested cells, and the four genes of AKT3, HMGB3, NFIB and PXDN were in down-regulation. Specifically, it was confirmed that these genes are useful as gene markers that change in relation to PKC-iota expression regulation. In Figs. 16 to 23, ■ indicates the result of no treatment; □ indicates the result of control; and the shadow indicates the result of PKC-iota siRNA treatment.

### INDUSTRIAL APPLICABILITY

PKC-iota has been found out as a target gene capable of specifically acting on cancer caused by the abnormality of P53 expression/function; and it is possible to evaluate a compound for the gene as a potential drug target gene. There are many varieties of cancers to be caused by the abnormality of p53 expression/function, and it is possible to provide a compound effective for treatment of many cancers. Through diagnosis based on the expression level of PKC-iota as an index, it is possible to determine cancers caused by the abnormality of p53 expression/function.

### BRIEF DESCRIPTION OF THE DRAWINGS

[Fig. 1] This is a graph showing the result of investigation of the influence of cytotoxicity in the presence or absence of mutant p53 introduction into U2-OS cells.
[Fig. 2] This is a graph showing the result of investigation of the cell growth change by PKC-iota siRNA introduction into A427 cells.
[Fig. 3] This is a graph showing the result of investigation of the cell growth change by PKC-iota siRNA introduction into A549 cells.
[Fig. 4] This is a graph showing the result of investigation of the cell growth change by PKC-iota siRNA introduction into LU99 cells.
[Fig. 5] This is a graph showing the result of investigation of the cell growth change by PKC-iota siRNA introduction into NCI-H460 cells.
[Fig. 6] This is a graph showing the result of investigation of the cell growth change by PKC-iota siRNA introduction into HOP62 cells.
[Fig. 7] This is a graph showing the result of investigation of the cell growth change by PKC-iota siRNA introduction into HOP92 cells.
[Fig. 8] This is a graph showing the result of investigation of the cell growth change by PKC-iota siRNA introduction into NCI-H322M cells.
[Fig. 9] This is a graph showing the result of investigation of the cell growth change by PKC-iota siRNA introduction into NCI-H226 cells.
[Fig. 10] This is a view showing the results of confirming the phosphorylation of BAD in the presence or absence of PKC-iota inhibition and in the presence or absence of p53 mutation.
[Fig. 11] This is a view showing the results of confirming the relationship between the presence or absence of p53 mutation (the expression level) and the binding amount of Bcl-XL.
[Fig. 12] Fig. 12A is a graph showing the results of confirming the effect of PKC-iota inhibition by the use of NCI-H460 (E545K) cells.

Fig. 12B is a graph showing the results of confirming the effect of PKC-iota inhibition by the use of NCI-H596 (E545K) cells.

Fig. 12C is a graph showing the results of confirming the effect of PKC-iota inhibition by the use of NCI-H1869 (E545A) cells.
[Fig. 13] Fig. 13A is a view showing the results of confirming the LLGL2 phosphorylation change by PKC-iota and PKC-zeta inhibition with MCF7 cells.

Fig. 13B is a view showing the results of confirming the LLGL2 phosphorylation change by PKC-iota and PKC-zeta inhibition with Hcc1419 cells.
[Fig. 14] This is a view showing the results of confirming the LLGL2 phosphorylation change by ordinary-activity mutant PKC-iota expression induction.
[Fig. 15] This is a view showing the results of confirming the LLGL2 phosphorylation change by addition of PKC-iota inhibitor.
[Fig. 16] Fig. 16A is a graph showing the expression level of PKC-iota in a case of inhibiting the PKC-iota expression in A549 cells.

Fig. 16B is a graph showing the expression level of PKC-iota in a case of inhibiting the PKC-iota expression in HOP62 cells.

Fig. 16C is a graph showing the expression level of PKC-iota in a case of inhibiting the PKC-iota expression in H460 cells.

Fig. 16D is a graph showing the expression level of PKC-iota in a case of inhibiting the PKC-iota expression in H596 cells.

Fig. 16E is a graph showing the expression level of PKC-iota in a case of inhibiting the PKC-iota expression in U2OS cells.
[Fig. 17] Fig. 17A is a graph showing the expression level of AKT3 in a case of inhibiting the PKC-iota expression in A549 cells.

Fig. 17B is a graph showing the expression level of AKT3 in a case of inhibiting the PKC-iota expression in HOP62 cells.

Fig. 17C is a graph showing the expression level of AKT3 in a case of inhibiting the PKC-iota expression in H460 cells.

Fig. 17D is a graph showing the expression level of AKT3 in a case of inhibiting the PKC-iota expression in H596 cells.

Fig. 17E is a graph showing the expression level of AKT3 in a case of inhibiting the PKC-iota expression in U2OS cells.
[Fig. 18] Fig. 18A is a graph showing the expression level of HMGB3 in a case of inhibiting the PKC-iota expression in A549 cells.

Fig. 18B is a graph showing the expression level of HMGB3 in a case of inhibiting the PKC-iota expression in HOP62 cells.

Fig. 18C is a graph showing the expression level of HMGB3 in a case of inhibiting the PKC-iota expression in H460 cells.

Fig. 18D is a graph showing the expression level of HMGB3 in a case of inhibiting the PKC-iota expression in H596 cells.

Fig. 18E is a graph showing the expression level of HMGB3 in a case of inhibiting the PKC-iota expression in U2OS cells.
[Fig. 19] Fig. 19A is a graph showing the expression level of NFIB in a case of inhibiting the PKC-iota expression in A549 cells.

Fig. 19B is a graph showing the expression level of NFIB in a case of inhibiting the PKC-iota expression in HOP62 cells.

Fig. 19C is a graph showing the expression level of NFIB in a case of inhibiting the PKC-iota expression in H460 cells.

Fig. 19D is a graph showing the expression level of NFIB in a case of inhibiting the PKC-iota expression in H596 cells.

Fig. 19E is a graph showing the expression level of NFIB in a case of inhibiting the PKC-iota expression in U2OS cells.
[Fig. 20] Fig. 20A is a graph showing the expression level of PXDN in a case of inhibiting the PKC-iota expression in A549 cells.

Fig. 20B is a graph showing the expression level of PXDN in a case of inhibiting the PKC-iota expression in HOP62 cells.

Fig. 20C is a graph showing the expression level of PXDN in a case of inhibiting the PKC-iota expression in H460 cells.

Fig. 20D is a graph showing the expression level of PXDN in a case of inhibiting the PKC-iota expression in H596 cells.

Fig. 20E is a graph showing the expression level of PXDN in a case of inhibiting the PKC-iota expression in U2OS cells.
[Fig. 21] Fig. 21A is a graph showing the expression level of SNF1LK in a case of inhibiting the PKC-iota expression in A549 cells.

Fig. 21B is a graph showing the expression level of SNF1LK in a case of inhibiting the PKC-iota expression in HOP62 cells.

Fig. 21C is a graph showing the expression level of SNF1LK in a case of inhibiting the PKC-iota expression in H460 cells.

Fig. 21D is a graph showing the expression level of SNF1LK in a case of inhibiting the PKC-iota expression in H596 cells.

Fig. 21E is a graph showing the expression level of SNF1LK in a case of inhibiting the PKC-iota expression in U2OS cells.
[Fig. 22] Fig. 22A is a graph showing the expression level of CLDN12 in a case of inhibiting the PKC-iota expression in A549 cells.

Fig. 22B is a graph showing the expression level of CLDN12 in a case of inhibiting the PKC-iota expression in HOP62 cells.

Fig. 22C is a graph showing the expression level of CLDN12 in a case of inhibiting the PKC-iota expression in H460 cells.

Fig. 22D is a graph showing the expression level of CLDN12 in a case of inhibiting the PKC-iota expression in H596 cells.

Fig. 22E is a graph showing the expression level of CLDN12 in a case of inhibiting the PKC-iota expression in U2OS cells.

## Claims

1. A method for evaluating a compound effective for cancer treatment, **characterized by** comprising:
a step of introducing a PKC-iota gene into a cell to prepare a cell capable of expressing PKC-iota,
a step of contacting a test compound with the cell, and
a step of detecting the specific binding of the test compound to PKC-iota.

2. A method for evaluating a compound effective for cancer treatment, **characterized by** comprising:
a step of introducing a PKC-iota gene into a cell to prepare a cell capable of expressing PKC-iota,
a step of contacting a test compound with the cell,
a step of measuring the activity of the intracellular transmitter formed by the contact, and
a step of comparing this activity with the activity of the intracellular transmitter in a case not contacted with the compound.

3. A method for evaluating a compound effective for treatment of cancer, **characterized by** comprising:
a step of introducing a PKC-iota gene into a cell to prepare a cell capable of expressing PKC-iota,
a step of contacting a test compound with the cell,
a step of measuring the expression level of PKC-iota or the expression level of the intracellular transmitter via PKC-iota.

4. The compound evaluation method as claimed in claim 2 or 3, wherein the intracellular transmitter is at least one selected from a group consisting of BAD, LLGL2, HMGB3, NFIB, PXDN, CLDN12, RPIA, AKT3 and SNF1LK.

5. A method for evaluating a compound effective for treatment of cancer, **characterized by** comprising:
a step of contacting a test compound with PKC-iota, and
a step of detecting the change in the activity of PKC-iota through the contact.

6. A method for evaluating a compound effective for treatment of cancer, **characterized by** comprising:
a step of contacting a test compound with PKC-iota in the presence of BAD or LLGL2, and
a step of detecting the change in the activity of PKC-iota through the contact.

7. The compound evaluation method as claimed in any one of claims 1 to 6, wherein the cancer is caused by p53 dysfunction.

8. The compound evaluation method as claimed in any one of claims 1 to 6, wherein the cancer is caused by PI3 kinase (phosphatidylinositol 3-kinase) dysfunction.

9. A diagnostic method for cancer, **characterized by** comprising:
a step of measuring the expression level of the PKC-iota gene in a test tissue or a test cell,
a step of comparing that expression level and the expression level of the PKC-iota gene in a normal tissue or a normal cell, and,
as a result of the comparison, a step of determining as to whether or not the expression level of the PKC-iota gene in the test tissue or the test cell is significantly larger than the expression level of the PKC-iota gene in the normal tissue or the normal cell.

10. A diagnostic method for cancer, **characterized by** comprising:
a step of measuring the activity of PKC-iota in a test tissue or a test cell,
a step of comparing that activity and the activity of PKC-iota in a normal tissue or a normal cell, and,
as a result of the comparison, a step of determining as to whether or not the activity of PKC-iota in the test tissue or the test cell is significantly larger than the activity of PKC-iota in the normal tissue or the normal cell.

11. A method for estimating or determining the effect of a PKC-iota inhibitor, **characterized by** comprising:
a step of measuring the activity of PKC-iota in the tissue derived from a test subject to which a PKC-iota inhibitor has been administered, and
a step of comparing the activity before and after the PKC-iota inhibitor administration.

12. A gene marker for estimating or determining the potency of a PKC-iota inhibitor,
**characterized in that**
the gene is at least one selected from a group consisting of BAD, LLGL2, HMGB3, NFIB, PXDN, CLDN12, RPIA, AKT3 and SNF1LK.

13. A microarray provided with the gene marker of claim 12.

14. A protein marker for estimating or determining the potency of a PKC-iota inhibitor, **characterized in that**
the protein is at least one selected from a group consisting of BAD, LLGL2, HMGB3, NFIB, PXDN, CLDN12, RPIA, AKT3 and SNF1LK.

15. A microarray provided with the protein marker of claim 14.

16. A diagnostic kit **characterized by** containing an antibody capable of detecting the protein marker of claim 14.

17. An antibody array **characterized by** comprising an antibody capable of detecting the protein marker of claim 14.

18. A method for estimating or determining the effect of a PKC-iota inhibitor, **characterized by** comprising
a step of detecting any one gene selected from a group consisting of HMGB3, NFIB, PXDN, CLDN 12, RPIA, AKT3 and SNF1LK in the tissue derived from a test subject to which a PKC-iota inhibitor has been administered, and
a step of comparing the expression level before and after the PKC-iota inhibitor administration.

19. The method for estimating or determining the effect of a PKC-iota inhibitor as claimed in claim 18, wherein the detecting unit for detection is a PCR or DNA microarray.

20. A method for estimating or determining the effect of a PKC-iota inhibitor, **characterized by** comprising
a step of measuring the activity of any one protein selected from a group consisting of HMGB3, NFIB, PXDN, CLDN12, RPIA, AKT3 and SNF1LK in the tissue derived from a test subject to which a PKC-iota inhibitor has been administered, and
a step of comparing the activity before and after the PKC-iota inhibitor administration.

21. A method for estimating or determining the effect of a PKC-iota inhibitor, **characterized by** comprising
a step of measuring the phosphorylation level of BAD or LLGL2 in the tissue derived from a test subject to which a PKC-iota inhibitor has been administered, and
a step of comparing the phosphorylation level before and after the PKC-iota inhibitor administration.


SEQUENCE LISTING

<110>  Banyu pharmaceutical co., ltd.

<120>  PKC-iota as a drug target for cancer therapy

<130>  BY0186Y

<150>  JP2006229999
<151>  2006-08-28

<160>  23

<170>  PatentIn version 3.1

<210>  1
<211>  4884
<212>  DNA
<213>  Homo sapiens

<220>
<221>  CDS
<222>  (239)..(2029)
<223>

<400>  1
aggtgtcttg ggcccgggcg gctgtagagg cggcggcgcc tacgggcagt gggaggagcc      60

gcgcggttcc ggctgctccg gcgaggcgac ccttgggtcg gcgctgcggg cgaggtgggc     120

aggtaggtgg gcggacggcc gcggttctcc ggcaagcgca ggcggcggag tcccccacgg     180

cgcccgaagc gcccccccgc acccccggcc tccagcgttg aggcggggga gtgaggag       238

atg ccg acc cag agg gac agc agc acc atg tcc cac acg gtc gca ggc       286
Met Pro Thr Gln Arg Asp Ser Ser Thr Met Ser His Thr Val Ala Gly
1               5                   10                  15

ggc ggc agc ggg gac cat tcc cac cag gtc cgg gtg aaa gcc tac tac       334
Gly Gly Ser Gly Asp His Ser His Gln Val Arg Val Lys Ala Tyr Tyr
            20                  25                  30

cgc ggg gat atc atg ata aca cat ttt gaa cct tcc atc tcc ttt gag       382
Arg Gly Asp Ile Met Ile Thr His Phe Glu Pro Ser Ile Ser Phe Glu
        35                  40                  45

ggc ctt tgc aat gag gtt cga gac atg tgt tct ttt gac aac gaa cag       430
Gly Leu Cys Asn Glu Val Arg Asp Met Cys Ser Phe Asp Asn Glu Gln
    50                  55                  60

ctc ttc acc atg aaa tgg ata gat gag gaa gga gac ccg tgt aca gta       478
Leu Phe Thr Met Lys Trp Ile Asp Glu Glu Gly Asp Pro Cys Thr Val
65                  70                  75                  80

tca tct cag ttg gag tta gaa gaa gcc ttt aga ctt tat gag cta aac       526
Ser Ser Gln Leu Glu Leu Glu Glu Ala Phe Arg Leu Tyr Glu Leu Asn
                85                  90                  95

aag gat tct gaa ctc ttg att cat gtg ttc cct tgt gta cca gaa cgt       574
Lys Asp Ser Glu Leu Leu Ile His Val Phe Pro Cys Val Pro Glu Arg
            100                 105                 110

cct ggg atg cct tgt cca gga gaa gat aaa tcc atc tac cgt aga ggt       622
Pro Gly Met Pro Cys Pro Gly Glu Asp Lys Ser Ile Tyr Arg Arg Gly
        115                 120                 125

gca cgc cgc tgg aga aag ctt tat tgt gcc aat ggc cac act ttc caa       670
Ala Arg Arg Trp Arg Lys Leu Tyr Cys Ala Asn Gly His Thr Phe Gln

130                 135                 140

gcc aag cgt ttc aac agg cgt gct cac tgt gcc atc tgc aca gac cga        718
Ala Lys Arg Phe Asn Arg Arg Ala His Cys Ala Ile Cys Thr Asp Arg
145                 150                 155                 160

ata tgg gga ctt gga cgc caa gga tat aag tgc atc aac tgc aaa ctc        766
Ile Trp Gly Leu Gly Arg Gln Gly Tyr Lys Cys Ile Asn Cys Lys Leu
                165                 170                 175

ttg gtt cat aag aag tgc cat aaa ctc gtc aca att gaa tgt ggg cgg        814
Leu Val His Lys Lys Cys His Lys Leu Val Thr Ile Glu Cys Gly Arg
            180                 185                 190

cat tct ttg cca cag gaa cca gtg atg ccc atg gat cag tca tcc atg        862
His Ser Leu Pro Gln Glu Pro Val Met Pro Met Asp Gln Ser Ser Met
        195                 200                 205

cat tct gac cat gca cag aca gta att cca tat aat cct tca agt cat        910
His Ser Asp His Ala Gln Thr Val Ile Pro Tyr Asn Pro Ser Ser His
    210                 215                 220

gag agt ttg gat caa gtt ggt gaa gaa aaa gag gca atg aac acc agg        958
Glu Ser Leu Asp Gln Val Gly Glu Glu Lys Glu Ala Met Asn Thr Arg
225                 230                 235                 240

gaa agt ggc aaa gct tca tcc agt cta ggt ctt cag gat ttt gat ttg       1006
Glu Ser Gly Lys Ala Ser Ser Ser Leu Gly Leu Gln Asp Phe Asp Leu
                245                 250                 255

ctc cgg gta ata gga aga gga agt tat gcc aaa gta ctg ttg gtt cga       1054
Leu Arg Val Ile Gly Arg Gly Ser Tyr Ala Lys Val Leu Leu Val Arg
            260                 265                 270

tta aaa aaa aca gat cgt att tat gca atg aaa gtt gtg aaa aaa gag       1102
Leu Lys Lys Thr Asp Arg Ile Tyr Ala Met Lys Val Val Lys Lys Glu
        275                 280                 285

ctt gtt aat gat gat gag gat att gat tgg gta cag aca gag aag cat       1150
Leu Val Asn Asp Asp Glu Asp Ile Asp Trp Val Gln Thr Glu Lys His
    290                 295                 300

gtg ttt gag cag gca tcc aat cat cct ttc ctt gtt ggg ctg cat tct       1198
Val Phe Glu Gln Ala Ser Asn His Pro Phe Leu Val Gly Leu His Ser
305                 310                 315                 320

tgc ttt cag aca gaa agc aga ttg ttc ttt gtt ata gag tat gta aat       1246
Cys Phe Gln Thr Glu Ser Arg Leu Phe Phe Val Ile Glu Tyr Val Asn
                325                 330                 335

gga gga gac cta atg ttt cat atg cag cga caa aga aaa ctt cct gaa       1294
Gly Gly Asp Leu Met Phe His Met Gln Arg Gln Arg Lys Leu Pro Glu
            340                 345                 350

gaa cat gcc aga ttt tac tct gca gaa atc agt cta gca tta aat tat       1342
Glu His Ala Arg Phe Tyr Ser Ala Glu Ile Ser Leu Ala Leu Asn Tyr
        355                 360                 365

ctt cat gag cga ggg ata att tat aga gat ttg aaa ctg gac aat gta       1390
Leu His Glu Arg Gly Ile Ile Tyr Arg Asp Leu Lys Leu Asp Asn Val
    370                 375                 380

tta ctg gac tct gaa ggc cac att aaa ctc act gac tac ggc atg tgt       1438
Leu Leu Asp Ser Glu Gly His Ile Lys Leu Thr Asp Tyr Gly Met Cys
385                 390                 395                 400

aag gaa gga tta cgg cca gga gat aca acc agc act ttc tgt ggt act       1486
Lys Glu Gly Leu Arg Pro Gly Asp Thr Thr Ser Thr Phe Cys Gly Thr

                405                 410                 415

cct aat tac att gct cct gaa att tta aga gga gaa gat tat ggt ttc 1534
Pro Asn Tyr Ile Ala Pro Glu Ile Leu Arg Gly Glu Asp Tyr Gly Phe
            420                 425                 430

agt gtt gac tgg tgg gct ctt gga gtg ctc atg ttt gag atg atg gca 1582
Ser Val Asp Trp Trp Ala Leu Gly Val Leu Met Phe Glu Met Met Ala
        435                 440                 445

gga agg tct cca ttt gat att gtt ggg agc tcc gat aac cct gac cag 1630
Gly Arg Ser Pro Phe Asp Ile Val Gly Ser Ser Asp Asn Pro Asp Gln
    450                 455                 460

aac aca gag gat tat ctc ttc caa gtt att ttg gaa aaa caa att cgc 1678
Asn Thr Glu Asp Tyr Leu Phe Gln Val Ile Leu Glu Lys Gln Ile Arg
465                 470                 475                 480

ata cca cgt tct ctg tct gta aaa gct gca agt gtt ctg aag agt ttt 1726
Ile Pro Arg Ser Leu Ser Val Lys Ala Ala Ser Val Leu Lys Ser Phe
                485                 490                 495

ctt aat aag gac cct aag gaa cga ttg ggt tgt cat cct caa aca gga 1774
Leu Asn Lys Asp Pro Lys Glu Arg Leu Gly Cys His Pro Gln Thr Gly
            500                 505                 510

ttt gct gat att cag gga cac ccg ttc ttc cga aat gtt gat tgg gat 1822
Phe Ala Asp Ile Gln Gly His Pro Phe Phe Arg Asn Val Asp Trp Asp
        515                 520                 525

atg atg gag caa aaa cag gtg gta cct ccc ttt aaa cca aat att tct 1870
Met Met Glu Gln Lys Gln Val Val Pro Pro Phe Lys Pro Asn Ile Ser
    530                 535                 540

ggg gaa ttt ggt ttg gac aac ttt gat tct cag ttt act aat gaa cct 1918
Gly Glu Phe Gly Leu Asp Asn Phe Asp Ser Gln Phe Thr Asn Glu Pro
545                 550                 555                 560

gtc cag ctc act cca gat gac gat gac att gtg agg aag att gat cag 1966
Val Gln Leu Thr Pro Asp Asp Asp Asp Ile Val Arg Lys Ile Asp Gln
                565                 570                 575

tct gaa ttt gaa ggt ttt gag tat atc aat cct ctt ttg atg tct gca 2014
Ser Glu Phe Glu Gly Phe Glu Tyr Ile Asn Pro Leu Leu Met Ser Ala
            580                 585                 590

gaa gaa tgt gtc tga tcctcatttt tcaaccatgt attctactca tgttgccatt 2069
Glu Glu Cys Val
        595

taatgcatgg ataaacttgc tgcaagcctg gatacaatta accattttat atttgccacc 2129

tacaaaaaaa cacccaatat cttctcttgt agactatatg aatcaattat tacatctgtt 2189

ttactatgaa aaaaaaatta atactactag cttccagaca atcatgtcaa aatttagttg 2249

aactggtttt tcagttttta aaaggcctac agatgagtaa tgaagttatc ttttttgttt 2309

aaaaaaaaaa aaaacactgc attaaaaaag tatctgttgc attaaggcac atagtgggat 2369

tacatcataa acctcccata atttttgtca ttctgtgtta aatcatttca gggtttaatt 2429

ttgaaataaa agattaatat aaaatgcaac aactttttat attacctatt agttttggag 2489

ttctttatgt ttaaaaattc aggtgtaaat tttattgcct tggataaata aattattgat 2549

cttttttaag gcagcagtta ttaaattggt aatagaagat gctgcttgct tagaattggg 2609

aaattcggct gggtgcagtg actcaatgcc tgtgatccca gtactttggg aggttgaggc 2669
gggcagatca cttgaggtca ggagtttgag attggcctgg tcaacatggt gaaatcctgt 2729
ctctactaaa aatacaaaaa ttagctgggc atggtggcac atacctgtaa tcccagctac 2789
tcgggaggct gaaacaggag aatcgcttga acccaggaga tggagttggc agtgagccaa 2849
gattagcctg ggcaacagtg agtgagactc ggtctcaaac aaaaaccaaa ttgggaaatt 2909
cttcttgagt taaaaaagac actttttatt atgaatcaga ttccaaatca ttaactattt 2969
ttgcaaaact ggcacaagtg gtttttcttt ttaagtatat taacagggta gctcctgagc 3029
ctttcccttt attggaaact ttaactgcag tgttaatata tacactgcaa aagaccaagg 3089
atgcagttga atggaaaatt actcagtgta tagaaaggaa aaaaaccaga aatttaaaat 3149
agatacagaa aagtacactg gtaatatttt tatttcctat tggagagtga atccccacag 3209
ttgctttttg tgacttgatt caaatgaggc actcattatt gttaccaaac ttgtaaaagc 3269
attccatgta ttcagtggag gcagaatgta taatattttt tccttataat ttagcataca 3329
ggaaaagtat ttatttttta agtagggtat cttaattatg ctcatttctt cccctaaacc 3389
acattatcta tatgattgga ccctttagtg aaatcagaaa aataaagtag gcatgaataa 3449
atgttcagga cacacacata cactcacaca catcacagta tcagctcttt cagtgagttt 3509
ttattaacta gataacacat gtaatacata gtagaataca tttcagaggc agagttcctc 3569
ggattatttt ttatggatat tagtgttcag cagcttatta tttaaagggg tgccaaatat 3629
agcatccttc tgttgtttat tttaaatata tttttaatga aaagggactt aaaaagtaaa 3689
catgattatc taaattactt aaaatttttt aatcttagta aaaattttag gaaagttatc 3749
tagtttaaat ttcaaatgtt ttaaaatttg taggcattta ataaattatc tttttttgga 3809
ggggtggatg ctgcatttca ttggaaatgt catttttcca aagtttctag ctatatactc 3869
tctttccttc aaataacata agtctgaaag caatgaaaac gtcaaaggaa aatgtttata 3929
tacatccaca tgtttattta atatgtttct cttaacacct gaaacttgtt ttaaaacaaa 3989
aagcagtatt cacttcaaat cataaactta gaattctgta tctcatgtca gaaatatgtt 4049
tatggccttg cttttaagtg gtgctgtttc ccatagggca ttagttctca gattttggaa 4109
tgtgaaataa tcacctagga agttggttta aaatgcacac tagtgagccc accttagact 4169
tcttttgggg ccttcgacct cactttgaga aataccttct tcaggttatt ctttatgttt 4229
gtcctgtggt taaatattag agtattcatg ataaaacatt ttgagaaggg gtaagcagtg 4289
agttattacc cacaatttaa agcaaatttt gaatgaatct ttaagttagg tatctacctt 4349
cccaccagcc ccccaaaaaa cctctcagta gtttctttca gtgtacaaaa tgatgagcat 4409
ttttctatga tgaggtttta accattattc agggtggtct tttgttttta aatctttttt 4469
taactaataa gatttacggt gtgtatttta tacagaaatg cattataaat gtttttaatt 4529
gtgttctgtt ttttgcagtc tttaagtgcc atgccaattg ttcttatatt ctatagaagt 4589
tcgctcaaaa tactcaacag gggaataggc agcggacagt cagaatggtt ggaattttgg 4649

ctttctaaga aaaactttat tttgcataag catgtggtca gatcattttg tgcatatgca 4709

gcctggattg gatgttaagt aaatgcttgt tcagtgccgg tacatttact taaatctgtt 4769

tttatttttg tcatgtagaa tactactgtg gtcatcataa tgtaatctat ttctgtacct 4829

tttttttttt tttttacttt gaagtcttta aataaaatgt ataataccca ttgag 4884

<210> 2
<211> 596
<212> PRT
<213> Homo sapiens

<400> 2

Met Pro Thr Gln Arg Asp Ser Ser Thr Met Ser His Thr Val Ala Gly
1               5                   10                  15

Gly Gly Ser Gly Asp His Ser His Gln Val Arg Val Lys Ala Tyr Tyr
            20                  25                  30

Arg Gly Asp Ile Met Ile Thr His Phe Glu Pro Ser Ile Ser Phe Glu
        35                  40                  45

Gly Leu Cys Asn Glu Val Arg Asp Met Cys Ser Phe Asp Asn Glu Gln
    50                  55                  60

Leu Phe Thr Met Lys Trp Ile Asp Glu Glu Gly Asp Pro Cys Thr Val
65                  70                  75                  80

Ser Ser Gln Leu Glu Leu Glu Glu Ala Phe Arg Leu Tyr Glu Leu Asn
                85                  90                  95

Lys Asp Ser Glu Leu Leu Ile His Val Phe Pro Cys Val Pro Glu Arg
            100                 105                 110

Pro Gly Met Pro Cys Pro Gly Glu Asp Lys Ser Ile Tyr Arg Arg Gly
        115                 120                 125

Ala Arg Arg Trp Arg Lys Leu Tyr Cys Ala Asn Gly His Thr Phe Gln
    130                 135                 140

Ala Lys Arg Phe Asn Arg Arg Ala His Cys Ala Ile Cys Thr Asp Arg
145                 150                 155                 160

Ile Trp Gly Leu Gly Arg Gln Gly Tyr Lys Cys Ile Asn Cys Lys Leu
                165                 170                 175

Leu Val His Lys Lys Cys His Lys Leu Val Thr Ile Glu Cys Gly Arg
            180                 185                 190

His Ser Leu Pro Gln Glu Pro Val Met Pro Met Asp Gln Ser Ser Met
        195                 200                 205

His Ser Asp His Ala Gln Thr Val Ile Pro Tyr Asn Pro Ser Ser His
    210                 215                 220

Glu Ser Leu Asp Gln Val Gly Glu Glu Lys Glu Ala Met Asn Thr Arg
225                 230                 235                 240

Glu Ser Gly Lys Ala Ser Ser Ser Leu Gly Leu Gln Asp Phe Asp Leu
                245                 250                 255

Leu Arg Val Ile Gly Arg Gly Ser Tyr Ala Lys Val Leu Leu Val Arg
            260                 265                 270

Leu Lys Lys Thr Asp Arg Ile Tyr Ala Met Lys Val Val Lys Lys Glu
        275                 280                 285

Leu Val Asn Asp Asp Glu Asp Ile Asp Trp Val Gln Thr Glu Lys His
    290                 295                 300

Val Phe Glu Gln Ala Ser Asn His Pro Phe Leu Val Gly Leu His Ser
305                 310                 315                 320

Cys Phe Gln Thr Glu Ser Arg Leu Phe Phe Val Ile Glu Tyr Val Asn
                325                 330                 335

Gly Gly Asp Leu Met Phe His Met Gln Arg Gln Arg Lys Leu Pro Glu
            340                 345                 350

Glu His Ala Arg Phe Tyr Ser Ala Glu Ile Ser Leu Ala Leu Asn Tyr
        355                 360                 365

Leu His Glu Arg Gly Ile Ile Tyr Arg Asp Leu Lys Leu Asp Asn Val
    370                 375                 380

Leu Leu Asp Ser Glu Gly His Ile Lys Leu Thr Asp Tyr Gly Met Cys
385                 390                 395                 400

Lys Glu Gly Leu Arg Pro Gly Asp Thr Thr Ser Thr Phe Cys Gly Thr
                405                 410                 415

Pro Asn Tyr Ile Ala Pro Glu Ile Leu Arg Gly Glu Asp Tyr Gly Phe
            420                 425                 430

Ser Val Asp Trp Trp Ala Leu Gly Val Leu Met Phe Glu Met Met Ala
        435                 440                 445

Gly Arg Ser Pro Phe Asp Ile Val Gly Ser Ser Asp Asn Pro Asp Gln
    450                 455                 460

Asn Thr Glu Asp Tyr Leu Phe Gln Val Ile Leu Glu Lys Gln Ile Arg
465                 470                 475                 480

Ile Pro Arg Ser Leu Ser Val Lys Ala Ala Ser Val Leu Lys Ser Phe
                485                 490                 495

Leu Asn Lys Asp Pro Lys Glu Arg Leu Gly Cys His Pro Gln Thr Gly
            500                 505                 510

Phe Ala Asp Ile Gln Gly His Pro Phe Phe Arg Asn Val Asp Trp Asp
        515                 520                 525

Met Met Glu Gln Lys Gln Val Val Pro Pro Phe Lys Pro Asn Ile Ser
    530                 535                 540

Gly Glu Phe Gly Leu Asp Asn Phe Asp Ser Gln Phe Thr Asn Glu Pro
545                 550                 555                 560

Val Gln Leu Thr Pro Asp Asp Asp Asp Ile Val Arg Lys Ile Asp Gln
                565                 570                 575

Ser Glu Phe Glu Gly Phe Glu Tyr Ile Asn Pro Leu Leu Met Ser Ala
            580                 585                 590

Glu Glu Cys Val
        595

<210>  3
<211>  956
<212>  DNA
<213>  Homo sapiens

<220>
<221>  CDS
<222>  (71)..(577)
<223>

<400>  3
gagcccgggg tgctggaggg aggcggcagg cccgggtcag gtgcctcgag atcgggcttg      60

ggcccagagc atg ttc cag atc cca gag ttt gag ccg agt gag cag gaa        109
           Met Phe Gln Ile Pro Glu Phe Glu Pro Ser Glu Gln Glu
           1               5                   10

gac tcc agc tct gca gag agg ggc ctg ggc ccc agc ccc gca ggg gac       157
Asp Ser Ser Ser Ala Glu Arg Gly Leu Gly Pro Ser Pro Ala Gly Asp
    15                  20                  25

ggg ccc tca ggc tcc ggc aag cat cat cgc cag gcc cca ggc ctc ctg       205
Gly Pro Ser Gly Ser Gly Lys His His Arg Gln Ala Pro Gly Leu Leu
30                  35                  40                  45

tgg gac gcc agt cac cag cag gag cag cca acc agc agc agc cat cat       253
Trp Asp Ala Ser His Gln Gln Glu Gln Pro Thr Ser Ser Ser His His
                50                  55                  60

gga ggc gct ggg gct gtg gag atc cgg agt cgc cac agc tcc tac ccc       301
Gly Gly Ala Gly Ala Val Glu Ile Arg Ser Arg His Ser Ser Tyr Pro
            65                  70                  75

gcg ggg acg gag gac gac gaa ggg atg ggg gag gag ccc agc ccc ttt       349
Ala Gly Thr Glu Asp Asp Glu Gly Met Gly Glu Glu Pro Ser Pro Phe
        80                  85                  90

cgg ggc cgc tcg cgc tcg gcg ccc ccc aac ctc tgg gca gca cag cgc        397
Arg Gly Arg Ser Arg Ser Ala Pro Pro Asn Leu Trp Ala Ala Gln Arg
    95                  100                 105

tat ggc cgc gag ctc cgg agg atg agt gac gag ttt gtg gac tcc ttt        445
Tyr Gly Arg Glu Leu Arg Arg Met Ser Asp Glu Phe Val Asp Ser Phe
110                 115                 120                 125

aag aag gga ctt cct cgc ccg aag agc gcg ggc aca gca acg cag atg        493
Lys Lys Gly Leu Pro Arg Pro Lys Ser Ala Gly Thr Ala Thr Gln Met
                130                 135                 140

cgg caa agc tcc agc tgg acg cga gtc ttc cag tcc tgg tgg gat cgg        541
Arg Gln Ser Ser Ser Trp Thr Arg Val Phe Gln Ser Trp Trp Asp Arg
            145                 150                 155

aac ttg ggc agg gga agc tcc gcc ccc tcc cag tga ccttcgctcc             587
Asn Leu Gly Arg Gly Ser Ser Ala Pro Ser Gln
        160                 165

acatcccgaa actccacccg ttcccactgc cctgggcagc catcttgaat atgggcggaa      647

gtacttccct caggcctatg caaaaagagg atccgtgctg tctcctttgg agggagggct      707

gacccagatt cccttccggt gcgtgtgaag ccacggaagg cttggtccca tcggaagttt      767

tgggttttcc gcccacagcc gccggaagtg gctccgtggc cccgccctca ggctccgggc      827

tttcccccag gcgcctgcgc taagtcgcga gccaggttta accgttgcgt caccgggacc      887

cgagcccccg cgatgccctg ggggccgtgc tcactaccaa atgttaataa agcccgcgtc       947

tgtgccgcc                                                              956

<210>  4
<211>  168
<212>  PRT
<213>  Homo sapiens

<400>  4

Met Phe Gln Ile Pro Glu Phe Glu Pro Ser Glu Gln Glu Asp Ser Ser
1               5                   10                  15

Ser Ala Glu Arg Gly Leu Gly Pro Ser Pro Ala Gly Asp Gly Pro Ser
            20                  25                  30

Gly Ser Gly Lys His His Arg Gln Ala Pro Gly Leu Leu Trp Asp Ala
        35                  40                  45

Ser His Gln Gln Glu Gln Pro Thr Ser Ser Ser His His Gly Gly Ala
    50                  55                  60

Gly Ala Val Glu Ile Arg Ser Arg His Ser Ser Tyr Pro Ala Gly Thr
65                  70                  75                  80

Glu Asp Asp Glu Gly Met Gly Glu Glu Pro Ser Pro Phe Arg Gly Arg
                85                  90                  95

Ser Arg Ser Ala Pro Pro Asn Leu Trp Ala Ala Gln Arg Tyr Gly Arg

            100                 105                 110

Glu Leu Arg Arg Met Ser Asp Glu Phe Val Asp Ser Phe Lys Lys Gly
        115                 120                 125

Leu Pro Arg Pro Lys Ser Ala Gly Thr Ala Thr Gln Met Arg Gln Ser
    130                 135                 140

Ser Ser Trp Thr Arg Val Phe Gln Ser Trp Trp Asp Arg Asn Leu Gly
145                 150                 155                 160

Arg Gly Ser Ser Ala Pro Ser Gln
                165

<210> 5
<211> 3590
<212> DNA
<213> Homo sapiens

<220>
<221> CDS
<222> (155)..(3217)
<223>

<400> 5
ggaggtgagc aggaaggaga cggccgccca gcagcccgtg ggcaggcgcg gcggagcgag 60

cggggccggc ggcgggcgcc gagggacgcc gaggcctcgg gcgggggctg gcccggggtt 120

ccaggtctcc agtgggggct gcagactaag caaa atg agg cgg ttc ctg agg cca 175
Met Arg Arg Phe Leu Arg Pro
1               5

ggg cat gac cct gtg cgg gag agg ctc aag cgg gac ctg ttc cag ttt 223
Gly His Asp Pro Val Arg Glu Arg Leu Lys Arg Asp Leu Phe Gln Phe
        10                  15                  20

aac aag acg gtg gag cat ggc ttc ccg cac cag ccc agc gcc ctc ggc 271
Asn Lys Thr Val Glu His Gly Phe Pro His Gln Pro Ser Ala Leu Gly
    25                  30                  35

tac agc ccg tcc ctg cgc atc ctg gcc atc ggc acc cgt tct gga gcc 319
Tyr Ser Pro Ser Leu Arg Ile Leu Ala Ile Gly Thr Arg Ser Gly Ala
40                  45                  50                  55

atc aag ctc tac gga gcc cca ggc gtg gag ttc atg ggg ctg cac cag 367
Ile Lys Leu Tyr Gly Ala Pro Gly Val Glu Phe Met Gly Leu His Gln
                60                  65                  70

gag aac aac gct gtg acg cag atc cac ctc ctg ccc ggc cag tgc cag 415
Glu Asn Asn Ala Val Thr Gln Ile His Leu Leu Pro Gly Gln Cys Gln
            75                  80                  85

ctg gtc acc ctg ctg gat gac aac agc ctg cac ctt tgg agc ctg aag 463
Leu Val Thr Leu Leu Asp Asp Asn Ser Leu His Leu Trp Ser Leu Lys
        90                  95                  100

gtc aag ggc ggg gca tcg gag ctg cag gag gat gag agc ttc aca ctg 511
Val Lys Gly Gly Ala Ser Glu Leu Gln Glu Asp Glu Ser Phe Thr Leu
    105                 110                 115

cgt gga ccc cca ggg gct gcc ccc agt gcc aca cag atc acc gtg gtc 559
Arg Gly Pro Pro Gly Ala Ala Pro Ser Ala Thr Gln Ile Thr Val Val

120                 125                 130                 135

ctg cca cat tcc tcc tgc gag ctg ctc tac ctg ggc acc gag agt ggc       607
Leu Pro His Ser Ser Cys Glu Leu Leu Tyr Leu Gly Thr Glu Ser Gly
                140                 145                 150

aac gtg ttt gtg gtg cag ctg cca gct ttt cgt gcg ctg gag gac cgg       655
Asn Val Phe Val Val Gln Leu Pro Ala Phe Arg Ala Leu Glu Asp Arg
            155                 160                 165

acc atc agc tcg gac gcg gtg ctg cag cgg ttg cca gag gag gcc cgc       703
Thr Ile Ser Ser Asp Ala Val Leu Gln Arg Leu Pro Glu Glu Ala Arg
        170                 175                 180

cac cgg cgt gtg ttc gag atg gtg gag gca ctg cag gag cac cct cga       751
His Arg Arg Val Phe Glu Met Val Glu Ala Leu Gln Glu His Pro Arg
    185                 190                 195

gac ccc aac cag atc ctg atc ggc tac agc cga ggc ctc gtt gtc atc       799
Asp Pro Asn Gln Ile Leu Ile Gly Tyr Ser Arg Gly Leu Val Val Ile
200                 205                 210                 215

tgg gac cta cag ggc agc cgc gtg ctc tac cac ttc ctc agc agc cag       847
Trp Asp Leu Gln Gly Ser Arg Val Leu Tyr His Phe Leu Ser Ser Gln
                220                 225                 230

caa ctg gag aac atc tgg tgg cag cgg gac ggc cgc ctg ctc gtc agc       895
Gln Leu Glu Asn Ile Trp Trp Gln Arg Asp Gly Arg Leu Leu Val Ser
            235                 240                 245

tgt cac tct gac ggc agc tac tgc cag tgg ccc gtg tcc agc gaa gcc       943
Cys His Ser Asp Gly Ser Tyr Cys Gln Trp Pro Val Ser Ser Glu Ala
        250                 255                 260

cag caa cca gag ccc ctc cgc agc ctc gtg cct tac ggt ccc ttt cct       991
Gln Gln Pro Glu Pro Leu Arg Ser Leu Val Pro Tyr Gly Pro Phe Pro
    265                 270                 275

tgc aaa gcg att acc aga atc ctc tgg ctg acc act agg cag ggg ttg      1039
Cys Lys Ala Ile Thr Arg Ile Leu Trp Leu Thr Thr Arg Gln Gly Leu
280                 285                 290                 295

ccc ttc acc atc ttc cag ggt ggc atg cca cgg gcc agc tac ggg gac      1087
Pro Phe Thr Ile Phe Gln Gly Gly Met Pro Arg Ala Ser Tyr Gly Asp
                300                 305                 310

cgc cac tgc atc tca gtg atc cac gat ggc cag cag acg gcc ttc gac      1135
Arg His Cys Ile Ser Val Ile His Asp Gly Gln Gln Thr Ala Phe Asp
            315                 320                 325

ttc acc tcc cgt gtc atc ggc ttc act gtc ctc aca gag gca gac cct      1183
Phe Thr Ser Arg Val Ile Gly Phe Thr Val Leu Thr Glu Ala Asp Pro
        330                 335                 340

gca gcc acc ttt gac gac ccc tat gcc ctg gtg gtg ctg gct gag gag      1231
Ala Ala Thr Phe Asp Asp Pro Tyr Ala Leu Val Val Leu Ala Glu Glu
    345                 350                 355

gag ctg gtg gtg att gac ctg cag aca gca ggc tgg cca ccg gtc cag      1279
Glu Leu Val Val Ile Asp Leu Gln Thr Ala Gly Trp Pro Pro Val Gln
360                 365                 370                 375

ctg ccc tac ctg gct tct ctg cac tgt tcc gcc atc acc tgc tct cac      1327
Leu Pro Tyr Leu Ala Ser Leu His Cys Ser Ala Ile Thr Cys Ser His
                380                 385                 390

cac gtc tcc aac atc ccg ctg aag ctg tgg gag cgg atc att gcc gcc      1375
His Val Ser Asn Ile Pro Leu Lys Leu Trp Glu Arg Ile Ile Ala Ala

            395                 400                 405

ggc agc cgg cag aac gca cac ttc tcc acc atg gag tgg cca att gat 1423
Gly Ser Arg Gln Asn Ala His Phe Ser Thr Met Glu Trp Pro Ile Asp
        410                 415                 420

ggt ggc acc agc ctg acc cca gcc cca ccc cag agg gac ctg ctg ctc 1471
Gly Gly Thr Ser Leu Thr Pro Ala Pro Pro Gln Arg Asp Leu Leu Leu
    425                 430                 435

aca ggg cac gag gac ggc acg gtg cgg ttc tgg gat gcc tcg ggt gtc 1519
Thr Gly His Glu Asp Gly Thr Val Arg Phe Trp Asp Ala Ser Gly Val
440                 445                 450                 455

tgc ctg cgg ctg ctc tac aaa ctc agc act gtg cgc gtg ttc ctc acc 1567
Cys Leu Arg Leu Leu Tyr Lys Leu Ser Thr Val Arg Val Phe Leu Thr
                460                 465                 470

gac acg gac ccc aac gag aac ttc agt gcc cag ggc gag gac gag tgg 1615
Asp Thr Asp Pro Asn Glu Asn Phe Ser Ala Gln Gly Glu Asp Glu Trp
            475                 480                 485

ccc cca ctc cgc aag gtg ggc tcc ttt gac ccc tac agt gat gac ccc 1663
Pro Pro Leu Arg Lys Val Gly Ser Phe Asp Pro Tyr Ser Asp Asp Pro
        490                 495                 500

cgg ctg ggc atc cag aag atc ttc ctc tgc aag tac agc ggc tac ctg 1711
Arg Leu Gly Ile Gln Lys Ile Phe Leu Cys Lys Tyr Ser Gly Tyr Leu
    505                 510                 515

gct gtg gca ggc acg gca ggg cag gtg ctg gta ctg gaa ctg aat gac 1759
Ala Val Ala Gly Thr Ala Gly Gln Val Leu Val Leu Glu Leu Asn Asp
520                 525                 530                 535

gag gca gcg gag cag gct gtg gag cag gtg gag gcc gac ctg ctg cag 1807
Glu Ala Ala Glu Gln Ala Val Glu Gln Val Glu Ala Asp Leu Leu Gln
                540                 545                 550

gac caa gag ggc tac cgc tgg aag ggg cac gag cgc ctg gca gcc cgc 1855
Asp Gln Glu Gly Tyr Arg Trp Lys Gly His Glu Arg Leu Ala Ala Arg
            555                 560                 565

tca ggg ccc gtg cgc ttt gag cct ggc ttt cag ccc ttc gtg ttg gtg 1903
Ser Gly Pro Val Arg Phe Glu Pro Gly Phe Gln Pro Phe Val Leu Val
        570                 575                 580

cag tgt cag ccc ccg gct gtg gtc acc tcc ttg gcc ctg cac tct gag 1951
Gln Cys Gln Pro Pro Ala Val Val Thr Ser Leu Ala Leu His Ser Glu
    585                 590                 595

tgg cgg ctc gtg gcc ttc ggc acc agc cat ggc ttt ggc ctc ttt gac 1999
Trp Arg Leu Val Ala Phe Gly Thr Ser His Gly Phe Gly Leu Phe Asp
600                 605                 610                 615

cac cag cag cgg cgg cag gtc ttt gtt aag tgc aca ctg cac ccc agt 2047
His Gln Gln Arg Arg Gln Val Phe Val Lys Cys Thr Leu His Pro Ser
                620                 625                 630

gac cag ctg gcc ttg gag ggc cca ctc tcc cgc gtc aag tcc ctc aag 2095
Asp Gln Leu Ala Leu Glu Gly Pro Leu Ser Arg Val Lys Ser Leu Lys
            635                 640                 645

aag tcc ttg cgt cag tca ttc cgc cgg atg cgt cgg agc cgg gtg tcc 2143
Lys Ser Leu Arg Gln Ser Phe Arg Arg Met Arg Arg Ser Arg Val Ser
        650                 655                 660

agc cgg aag cgg cac ccg gct ggc ccc cca gga gag gca cag gag ggg 2191
Ser Arg Lys Arg His Pro Ala Gly Pro Pro Gly Glu Ala Gln Glu Gly

665                 670                 675

agt gcc aag gct gag cgg cca ggc ctc cag aac atg gag ctg gcg cct        2239
Ser Ala Lys Ala Glu Arg Pro Gly Leu Gln Asn Met Glu Leu Ala Pro
680                 685                 690                 695

gtg cag cgc aag atc gag gct cgc tcg gca gag gac tcc ttc aca ggc        2287
Val Gln Arg Lys Ile Glu Ala Arg Ser Ala Glu Asp Ser Phe Thr Gly
                700                 705                 710

ttc gtc cgg acc ctg tac ttt gct gac acc tac ctg aag gac agc tcc        2335
Phe Val Arg Thr Leu Tyr Phe Ala Asp Thr Tyr Leu Lys Asp Ser Ser
            715                 720                 725

cgg cac tgc ccc tcg ctg tgg gct ggc acc aat ggg ggc acc atc tat        2383
Arg His Cys Pro Ser Leu Trp Ala Gly Thr Asn Gly Gly Thr Ile Tyr
        730                 735                 740

gcc ttc tcc ctg cgt gtg cct ccc gcc gag cgg aga atg gat gag cct        2431
Ala Phe Ser Leu Arg Val Pro Pro Ala Glu Arg Arg Met Asp Glu Pro
    745                 750                 755

gtg cgg gca gag cag gcc aag gag atc cag ctg atg cac cgg gcg ccg        2479
Val Arg Ala Glu Gln Ala Lys Glu Ile Gln Leu Met His Arg Ala Pro
760                 765                 770                 775

gtg gtg ggc atc ctg gtg ctc gac gga cac agc gta ccc ctt ccc gag        2527
Val Val Gly Ile Leu Val Leu Asp Gly His Ser Val Pro Leu Pro Glu
                780                 785                 790

ccc ctc gaa gtg gcc cat gat ctg tcg aag agc cct gac atg cag gga        2575
Pro Leu Glu Val Ala His Asp Leu Ser Lys Ser Pro Asp Met Gln Gly
            795                 800                 805

agc cac cag ctg ctc gtc gta tca gag gag cag ttc aag gtg ttc acg        2623
Ser His Gln Leu Leu Val Val Ser Glu Glu Gln Phe Lys Val Phe Thr
        810                 815                 820

ctg ccc aag gtg agt gcc aag ctg aag ttg aag ctg acg gcc ctg gag        2671
Leu Pro Lys Val Ser Ala Lys Leu Lys Leu Lys Leu Thr Ala Leu Glu
    825                 830                 835

ggc tca aga gtg cgg cgg gtc agc gtg gcc cac ttc ggc agt cgt cga        2719
Gly Ser Arg Val Arg Arg Val Ser Val Ala His Phe Gly Ser Arg Arg
840                 845                 850                 855

gcc gag gac tac ggg gag cac cac ctg gca gtc ctt acc aac ctg ggc        2767
Ala Glu Asp Tyr Gly Glu His His Leu Ala Val Leu Thr Asn Leu Gly
                860                 865                 870

gac atc cag gtg gtc tcg ctg ccc ctg ctc aag ccc cag gtg cgc tac        2815
Asp Ile Gln Val Val Ser Leu Pro Leu Leu Lys Pro Gln Val Arg Tyr
            875                 880                 885

agc tgc atc cgc cgg gag gac gtc agt ggc atc gcc tcc tgc gtc ttc        2863
Ser Cys Ile Arg Arg Glu Asp Val Ser Gly Ile Ala Ser Cys Val Phe
        890                 895                 900

acc aaa tat ggc caa ggc ttc tac ctg atc tca ccc tcg gag ttt gag        2911
Thr Lys Tyr Gly Gln Gly Phe Tyr Leu Ile Ser Pro Ser Glu Phe Glu
    905                 910                 915

cgc ttc tct ctc tcc acc aag tgg ctg gtg gag ccc cgg tgt ctg gtg        2959
Arg Phe Ser Leu Ser Thr Lys Trp Leu Val Glu Pro Arg Cys Leu Val
920                 925                 930                 935

gat tca gca gaa acc aag aac cac cgc cct ggt aac ggt gcg ggc ccc        3007
Asp Ser Ala Glu Thr Lys Asn His Arg Pro Gly Asn Gly Ala Gly Pro

                940                 945                 950

aag aag gcc ccg agc cga gcc agg aac tca ggg act cag agt gat ggc 3055
Lys Lys Ala Pro Ser Arg Ala Arg Asn Ser Gly Thr Gln Ser Asp Gly
            955                 960                 965

gag gag aag cag ccc ggc ctg gtg atg gag cgc gct ctg ctc agt gat 3103
Glu Glu Lys Gln Pro Gly Leu Val Met Glu Arg Ala Leu Leu Ser Asp
        970                 975                 980

gag aga gtc ctg aag gaa atc cag agc aca ctg gag gga gac cgc ggg 3151
Glu Arg Val Leu Lys Glu Ile Gln Ser Thr Leu Glu Gly Asp Arg Gly
    985                 990                 995

agc ggc aac tgg cgt tca cat cga gcc gcc gtg ggg tgc agc ctc 3196
Ser Gly Asn Trp Arg Ser His Arg Ala Ala Val Gly Cys Ser Leu
1000                1005                1010

agc aat ggc gga gca gag tga gtggctgagc gtccaggctg cgcgatgagc 3247
Ser Asn Gly Gly Ala Glu
1015                1020

acacactact actgatggcc tttcgggggt ccctgcccca accggagagg ccggtgcaca 3307

gggccccgcc aggggctggg ggcatcccgg cttccacaat gcagctgctc tgggcctcgg 3367

gagaggagag accccagtcc cctgggctgc ccttcccggg cctcgtctgt ctgggtcctt 3427

tggtcaatgt tgcacagttt ttattgctcc catccctttt tgtagtgggc tgggttttaa 3487

gttataaatg ttaactgcct ctgggtgaaa aagtttttaa taaacaccta ttacctcttg 3547

aaaaaaaaaa aaaaaaaaaa aaaaaaaaaa aaaaaaaaaa aaa 3590

<210> 6
<211> 1020
<212> PRT
<213> Homo sapiens

<400> 6

Met Arg Arg Phe Leu Arg Pro Gly His Asp Pro Val Arg Glu Arg Leu
1               5                   10                  15

Lys Arg Asp Leu Phe Gln Phe Asn Lys Thr Val Glu His Gly Phe Pro
            20                  25                  30

His Gln Pro Ser Ala Leu Gly Tyr Ser Pro Ser Leu Arg Ile Leu Ala
        35                  40                  45

Ile Gly Thr Arg Ser Gly Ala Ile Lys Leu Tyr Gly Ala Pro Gly Val
    50                  55                  60

Glu Phe Met Gly Leu His Gln Glu Asn Asn Ala Val Thr Gln Ile His
65                  70                  75                  80

Leu Leu Pro Gly Gln Cys Gln Leu Val Thr Leu Leu Asp Asp Asn Ser
                85                  90                  95

Leu His Leu Trp Ser Leu Lys Val Lys Gly Gly Ala Ser Glu Leu Gln
            100                 105                 110

Glu Asp Glu Ser Phe Thr Leu Arg Gly Pro Pro Gly Ala Ala Pro Ser
        115                 120                 125

Ala Thr Gln Ile Thr Val Val Leu Pro His Ser Ser Cys Glu Leu Leu
    130                 135                 140

Tyr Leu Gly Thr Glu Ser Gly Asn Val Phe Val Val Gln Leu Pro Ala
145                 150                 155                 160

Phe Arg Ala Leu Glu Asp Arg Thr Ile Ser Ser Asp Ala Val Leu Gln
                165                 170                 175

Arg Leu Pro Glu Glu Ala Arg His Arg Arg Val Phe Glu Met Val Glu
            180                 185                 190

Ala Leu Gln Glu His Pro Arg Asp Pro Asn Gln Ile Leu Ile Gly Tyr
        195                 200                 205

Ser Arg Gly Leu Val Val Ile Trp Asp Leu Gln Gly Ser Arg Val Leu
    210                 215                 220

Tyr His Phe Leu Ser Ser Gln Gln Leu Glu Asn Ile Trp Trp Gln Arg
225                 230                 235                 240

Asp Gly Arg Leu Leu Val Ser Cys His Ser Asp Gly Ser Tyr Cys Gln
                245                 250                 255

Trp Pro Val Ser Ser Glu Ala Gln Gln Pro Glu Pro Leu Arg Ser Leu
            260                 265                 270

Val Pro Tyr Gly Pro Phe Pro Cys Lys Ala Ile Thr Arg Ile Leu Trp
        275                 280                 285

Leu Thr Thr Arg Gln Gly Leu Pro Phe Thr Ile Phe Gln Gly Gly Met
    290                 295                 300

Pro Arg Ala Ser Tyr Gly Asp Arg His Cys Ile Ser Val Ile His Asp
305                 310                 315                 320

Gly Gln Gln Thr Ala Phe Asp Phe Thr Ser Arg Val Ile Gly Phe Thr
                325                 330                 335

Val Leu Thr Glu Ala Asp Pro Ala Ala Thr Phe Asp Asp Pro Tyr Ala
            340                 345                 350

Leu Val Val Leu Ala Glu Glu Glu Leu Val Val Ile Asp Leu Gln Thr
        355                 360                 365

Ala Gly Trp Pro Pro Val Gln Leu Pro Tyr Leu Ala Ser Leu His Cys
    370                 375                 380

Ser Ala Ile Thr Cys Ser His His Val Ser Asn Ile Pro Leu Lys Leu
385                 390                 395                 400

Trp Glu Arg Ile Ile Ala Ala Gly Ser Arg Gln Asn Ala His Phe Ser
                405                 410                 415

Thr Met Glu Trp Pro Ile Asp Gly Gly Thr Ser Leu Thr Pro Ala Pro
            420                 425                 430

Pro Gln Arg Asp Leu Leu Leu Thr Gly His Glu Asp Gly Thr Val Arg
        435                 440                 445

Phe Trp Asp Ala Ser Gly Val Cys Leu Arg Leu Leu Tyr Lys Leu Ser
    450                 455                 460

Thr Val Arg Val Phe Leu Thr Asp Thr Asp Pro Asn Glu Asn Phe Ser
465                 470                 475                 480

Ala Gln Gly Glu Asp Glu Trp Pro Pro Leu Arg Lys Val Gly Ser Phe
                485                 490                 495

Asp Pro Tyr Ser Asp Asp Pro Arg Leu Gly Ile Gln Lys Ile Phe Leu
            500                 505                 510

Cys Lys Tyr Ser Gly Tyr Leu Ala Val Ala Gly Thr Ala Gly Gln Val
        515                 520                 525

Leu Val Leu Glu Leu Asn Asp Glu Ala Ala Glu Gln Ala Val Glu Gln
    530                 535                 540

Val Glu Ala Asp Leu Leu Gln Asp Gln Glu Gly Tyr Arg Trp Lys Gly
545                 550                 555                 560

His Glu Arg Leu Ala Ala Arg Ser Gly Pro Val Arg Phe Glu Pro Gly
                565                 570                 575

Phe Gln Pro Phe Val Leu Val Gln Cys Gln Pro Pro Ala Val Val Thr
            580                 585                 590

Ser Leu Ala Leu His Ser Glu Trp Arg Leu Val Ala Phe Gly Thr Ser
        595                 600                 605

His Gly Phe Gly Leu Phe Asp His Gln Gln Arg Arg Gln Val Phe Val
    610                 615                 620

Lys Cys Thr Leu His Pro Ser Asp Gln Leu Ala Leu Glu Gly Pro Leu
625                 630                 635                 640

Ser Arg Val Lys Ser Leu Lys Lys Ser Leu Arg Gln Ser Phe Arg Arg
                645                 650                 655

Met Arg Arg Ser Arg Val Ser Ser Arg Lys Arg His Pro Ala Gly Pro
            660                 665                 670

Pro Gly Glu Ala Gln Glu Gly Ser Ala Lys Ala Glu Arg Pro Gly Leu
        675                 680                 685

Gln Asn Met Glu Leu Ala Pro Val Gln Arg Lys Ile Glu Ala Arg Ser
    690                 695                 700

Ala Glu Asp Ser Phe Thr Gly Phe Val Arg Thr Leu Tyr Phe Ala Asp
705                 710                 715                 720

Thr Tyr Leu Lys Asp Ser Ser Arg His Cys Pro Ser Leu Trp Ala Gly
                725                 730                 735

Thr Asn Gly Gly Thr Ile Tyr Ala Phe Ser Leu Arg Val Pro Pro Ala
            740                 745                 750

Glu Arg Arg Met Asp Glu Pro Val Arg Ala Glu Gln Ala Lys Glu Ile
        755                 760                 765

Gln Leu Met His Arg Ala Pro Val Val Gly Ile Leu Val Leu Asp Gly
    770                 775                 780

His Ser Val Pro Leu Pro Glu Pro Leu Glu Val Ala His Asp Leu Ser
785                 790                 795                 800

Lys Ser Pro Asp Met Gln Gly Ser His Gln Leu Leu Val Val Ser Glu
                805                 810                 815

Glu Gln Phe Lys Val Phe Thr Leu Pro Lys Val Ser Ala Lys Leu Lys
            820                 825                 830

Leu Lys Leu Thr Ala Leu Glu Gly Ser Arg Val Arg Arg Val Ser Val
        835                 840                 845

Ala His Phe Gly Ser Arg Arg Ala Glu Asp Tyr Gly Glu His His Leu
    850                 855                 860

Ala Val Leu Thr Asn Leu Gly Asp Ile Gln Val Val Ser Leu Pro Leu
865                 870                 875                 880

Leu Lys Pro Gln Val Arg Tyr Ser Cys Ile Arg Arg Glu Asp Val Ser
                885                 890                 895

Gly Ile Ala Ser Cys Val Phe Thr Lys Tyr Gly Gln Gly Phe Tyr Leu
            900                 905                 910

Ile Ser Pro Ser Glu Phe Glu Arg Phe Ser Leu Ser Thr Lys Trp Leu
        915                 920                 925

Val Glu Pro Arg Cys Leu Val Asp Ser Ala Glu Thr Lys Asn His Arg
    930                 935                 940

Pro Gly Asn Gly Ala Gly Pro Lys Lys Ala Pro Ser Arg Ala Arg Asn
945                 950                 955                 960

Ser Gly Thr Gln Ser Asp Gly Glu Glu Lys Gln Pro Gly Leu Val Met
                965                 970                 975

Glu Arg Ala Leu Leu Ser Asp Glu Arg Val Leu Lys Glu Ile Gln Ser
            980                 985                 990

Thr Leu Glu Gly Asp Arg Gly Ser  Gly Asn Trp Arg Ser  His Arg Ala
        995                 1000                1005

Ala Val  Gly Cys Ser Leu Ser  Asn Gly Gly Ala Glu
    1010                1015                1020

<210>  7
<211>  3556
<212>  DNA
<213>  Homo sapiens

<220>
<221>  CDS
<222>  (93)..(695)
<223>

<400>  7
ggctctcgtg gaggcagcta gcgcgaggct ggggagcgct gagccgcgcg tcgtgccctg      60

cgctgcccag actagcgaac aatacagtca gg atg gct aaa ggt gac ccc aag      113
                                    Met Ala Lys Gly Asp Pro Lys
                                    1               5

aaa cca aag ggc aag atg tcc gct tat gcc ttc ttt gtg cag aca tgc      161
Lys Pro Lys Gly Lys Met Ser Ala Tyr Ala Phe Phe Val Gln Thr Cys
        10                  15                  20

aga gaa gaa cat aag aag aaa aac cca gag gtc cct gtc aat ttt gcg      209
Arg Glu Glu His Lys Lys Lys Asn Pro Glu Val Pro Val Asn Phe Ala
    25                  30                  35

gaa ttt tcc aag aag tgc tct gag agg tgg aag acg atg tcc ggg aaa      257
Glu Phe Ser Lys Lys Cys Ser Glu Arg Trp Lys Thr Met Ser Gly Lys
40                  45                  50                  55

gag aaa tct aaa ttt gat gaa atg gca aag gca gat aaa gtg cgc tat      305
Glu Lys Ser Lys Phe Asp Glu Met Ala Lys Ala Asp Lys Val Arg Tyr
                60                  65                  70

gat cgg gaa atg aag gat tat gga cca gct aag gga ggc aag aag aag      353
Asp Arg Glu Met Lys Asp Tyr Gly Pro Ala Lys Gly Gly Lys Lys Lys
            75                  80                  85

aag gat cct aat gct ccc aaa agg cca ccg tct gga ttc ttc ctg ttc      401
Lys Asp Pro Asn Ala Pro Lys Arg Pro Pro Ser Gly Phe Phe Leu Phe
        90                  95                  100

tgt tca gaa ttc cgc ccc aag atc aaa tcc aca aac ccc ggc atc tct      449

Cys Ser Glu Phe Arg Pro Lys Ile Lys Ser Thr Asn Pro Gly Ile Ser
    105                 110                 115

att gga gac gtg gca aaa aag ctg ggt gag atg tgg aat aat tta aat      497
Ile Gly Asp Val Ala Lys Lys Leu Gly Glu Met Trp Asn Asn Leu Asn
120                 125                 130                 135

gac agt gaa aag cag cct tac atc act aag gcg gca aag ctg aag gag      545
Asp Ser Glu Lys Gln Pro Tyr Ile Thr Lys Ala Ala Lys Leu Lys Glu
                140                 145                 150

aag tat gag aag gat gtt gct gac tat aag tcg aaa gga aag ttt gat      593
Lys Tyr Glu Lys Asp Val Ala Asp Tyr Lys Ser Lys Gly Lys Phe Asp
            155                 160                 165

ggt gca aag ggt cct gct aaa gtt gcc cgg aaa aag gtg gaa gag gaa      641
Gly Ala Lys Gly Pro Ala Lys Val Ala Arg Lys Lys Val Glu Glu Glu
        170                 175                 180

gat gaa gaa gag gag gag gaa gaa gag gag gag gag gag gag gag gat      689
Asp Glu Glu Glu Glu Glu Glu Glu Glu Glu Glu Glu Glu Glu Glu Asp
    185                 190                 195

gaa taa agaaactgtt tatctgtctc cttgtgaata cttagagtag gggagcgccg      745
Glu
200

taattgacac atctcttatt tgagaagtgt ctgttgccct cattaggttt aattacaaaa      805

tttgatcacg atcatattgt agtctctcaa agtgctctag aaattgtcag tggtttacat      865

gaagtggcca tgggtgtctg gagcaccctg aaactgtatc aaagttgtac atatttccaa      925

acatttttaa aatgaaaagg cactctcgtg ttctcctcac tctgtgcact ttgctgttgg      985

tgtgacaagg catttaaaga tgtttctggc attttctttt tatttgtaag gtggtggtaa     1045

ctatggttat tggctagaaa tcctgagttt tcaactgtat atatctatag tttgtaaaaa     1105

gaacaaaaca accgagacaa acccttgatg ctccttgctc ggcgttgagg ctgtggggaa     1165

gatgcctttt gggagaggct gtagctcagg gcgtgcactg tgaggctgga cctgttgact     1225

ctgcaggggg catccattta gcttcaggtt gtcttgtttc tgtatatagt gacatagcat     1285

tctgctgcca tcttagctgt ggacaaaggg gggtcagctg gcatgagaat attttttttt     1345

tttaagtgcg gtagttttta aactgtttgt ttttaaacaa actatagaac tcttcattgt     1405

cagcaaagca aagagtcact gcatcaatga aagttcaaga acctcctgta cttaaacacg     1465

attcgcaacg ttctgttatt ttttttgtat gtttagaatg ctgaaatgtt tttgaagtta     1525

aataaacagt attacatttt taaaactctt ctctattata acagtcaatt tctgactcac     1585

agcagtgaac aaacccccac tccattgtat ttggagactg gcctccctat aaatgtggta     1645

gcttctttta ttactcagtg gccagctcac ttagggctga gatgaaggag agggctactt     1705

gaagctactg tgtgattttg tttgtgtctg agtggcattc agatgaagtc tggaggagtt     1765

aggagaacga cataggcaag gttcagcagc cttccaaggt ataggaaggt gggtgattag     1825

gactgaggct atctaggttt aacttttgtc ccacctccac cccctatttt gtggggccaa     1885

atgcattgct aaacagcaat ttcagagtgt atggtgtgtc aaaaattaag gccttattgt     1945

ttttctcttt cacccctacc ccccgtgctc ctggcacata tcacattatt tgtggtgccc     2005

aacatttggg gtcttgagcc tgctgctggt ctcctggatg ccagtgaggg tatgtgggat 2065

ggggtggtgg ggtaggggac ggtatccttt ttttgctcct acttggaaac accaaacacc 2125

ccaaggaaga tgataggctc catcttgggc cacctgagct atagggcagg ctaatggaat 2185

caaccatttc tgagcactaa atgtatcatg aaaagttgaa tggcctgctc ataagtttag 2245

ctcattcact ggaaatgtag attgatgttc aatgttaaac tggaaggagc ttggtttgtg 2305

tgtcagtggt tatattagtg ggtagtgtaa cattttatcc aggttggggt gaggggagat 2365

ggccacagta gcaagtggtg acactaaata ccattttgaa ggctgatgtg tatatacatc 2425

attactgtcc gtagcaatga aggatacagt actgtgttgt gggtgagtgt tgctattgcc 2485

cagcattaat atttgggtgt gtatgtttga ggctatgaaa cacgcaggag tgtttttgtg 2545

ctattaattt taagagaaag cagctttttc ttaaaattca ctgttgagaa acttgcatgt 2605

ctggaggcgg tgtcctctcc gccctgtcgg gtcctggatg agtacgagtt atggtcacgg 2665

tcacagcctg atctcttatg tgttcatagc cattcgctct cccatcagaa ctgtttgtcc 2725

tgaatgtgtt cctctagttc tagaaaatga ccactaattt aaaaaactcg gttgtgaggt 2785

ttgcccagag gcacttgttc cagaatttcc cctcctgctt cagccatgtc cttgtcactt 2845

ggcattctaa gctaaagctt tagcttccca attcgtgatg tgctaggcca agattcggga 2905

gctgttgcca gcctcgtcaa atatggaaga gaaacaacct gcggtcaaaa gggagtgatt 2965

tgttaagtgg tgcgcgtcta tctcataact agatgtacca accagggaag ggccaaggat 3025

ggaaaggggt aacttttgtg cttccaaagt agctaagcag aagtggggga gcagtttagc 3085

cagatgatct ttgattaggc aaacattgag ttttaaagag gctgtcaagt tgaggccact 3145

tggtccatta gctggggcag caagatcact actcaacgtt ttcacactgt ggcaagattg 3205

ctcttctagt ggaataatgc cctagtttct ctgagatgat gtaagtggca tgatgttacc 3265

taaggcttag gcttagcttg atttctgggc ccactgtctg tgttcttaag atgccaacct 3325

gttgcttttt tttttttttt cccccattta aaaggatagt acctactccc tctaaccacc 3385

tcaccccatt cttgaatgac attttatcct tcggaaagaa caaggctgtg atgtagtgac 3445

tattgtctgt gtctcctgtg tgtgtctgtt cttgtcacaa atgtatttgg ggacgttgga 3505

tgcattcatt ttctgtaata aagtttctta atcactcttc ccaaaaagta a 3556

<210> 8
<211> 200
<212> PRT
<213> Homo sapiens

<400> 8

Met Ala Lys Gly Asp Pro Lys Lys Pro Lys Gly Lys Met Ser Ala Tyr
1               5                   10                  15

Ala Phe Phe Val Gln Thr Cys Arg Glu Glu His Lys Lys Lys Asn Pro
            20                  25                  30

Glu Val Pro Val Asn Phe Ala Glu Phe Ser Lys Lys Cys Ser Glu Arg
        35                  40                  45

Trp Lys Thr Met Ser Gly Lys Glu Lys Ser Lys Phe Asp Glu Met Ala
    50                  55                  60

Lys Ala Asp Lys Val Arg Tyr Asp Arg Glu Met Lys Asp Tyr Gly Pro
65                  70                  75                  80

Ala Lys Gly Gly Lys Lys Lys Lys Asp Pro Asn Ala Pro Lys Arg Pro
                85                  90                  95

Pro Ser Gly Phe Phe Leu Phe Cys Ser Glu Phe Arg Pro Lys Ile Lys
            100                 105                 110

Ser Thr Asn Pro Gly Ile Ser Ile Gly Asp Val Ala Lys Lys Leu Gly
        115                 120                 125

Glu Met Trp Asn Asn Leu Asn Asp Ser Glu Lys Gln Pro Tyr Ile Thr
    130                 135                 140

Lys Ala Ala Lys Leu Lys Glu Lys Tyr Glu Lys Asp Val Ala Asp Tyr
145                 150                 155                 160

Lys Ser Lys Gly Lys Phe Asp Gly Ala Lys Gly Pro Ala Lys Val Ala
                165                 170                 175

Arg Lys Lys Val Glu Glu Glu Asp Glu Glu Glu Glu Glu Glu Glu Glu
            180                 185                 190

Glu Glu Glu Glu Glu Glu Asp Glu
        195                 200

<210>  9
<211>  8159
<212>  DNA
<213>  Homo sapiens

<220>
<221>  CDS
<222>  (436)..(1698)
<223>

<400>  9
cgcctttttt gaaaaagcat tttaccacca accaccaccc caatccaacc cacaccgaac      60

cttcgcgcac cccctacacc ccaacaacaa caacaactgc aaaatagaaa acaaatcccc     120

aaacccaggc gaaaagcagc caacaccggc ggcggcggcg gcctcggcaa gcacggccag     180

cgcgctcgga ctgcaagagg gttaaaagtg tagattggat ttcacccctg gaaatctagc     240

acgccgagtg aacttgaatc tttggctatt taaggaggac tgggtttgtt gtgaagttgc     300

ggtgatccag cgcagagccc cgtcctgatt gatcgcatcg cggggctcag atgactgtaa     360

aatgaataga tgaaattctt gcttctcgaa gattttcttg ggcatctccc ggaaagtgcg 420

ttttaaggcg aagtc atg atg tat tct ccc atc tgt ctc act cag gat gaa 471
Met Met Tyr Ser Pro Ile Cys Leu Thr Gln Asp Glu
1               5                   10

ttt cac cca ttc atc gag gca ctt ctt cca cat gtc cgt gca att gcc 519
Phe His Pro Phe Ile Glu Ala Leu Leu Pro His Val Arg Ala Ile Ala
        15                  20                  25

tat act tgg ttc aac ctg cag gct cga aaa cgc aag tac ttt aaa aag 567
Tyr Thr Trp Phe Asn Leu Gln Ala Arg Lys Arg Lys Tyr Phe Lys Lys
    30                  35                  40

cat gag aag cga atg tca aag gat gaa gaa aga gca gtc aaa gat gag 615
His Glu Lys Arg Met Ser Lys Asp Glu Glu Arg Ala Val Lys Asp Glu
45                  50                  55                  60

ctt ctc agt gaa aag cct gaa atc aaa cag aag tgg gca tcc agg ctc 663
Leu Leu Ser Glu Lys Pro Glu Ile Lys Gln Lys Trp Ala Ser Arg Leu
                65                  70                  75

ctt gcc aaa ctg cgc aaa gat att cgc cag gag tat cga gag gac ttt 711
Leu Ala Lys Leu Arg Lys Asp Ile Arg Gln Glu Tyr Arg Glu Asp Phe
            80                  85                  90

gtg ctc acc gtg act ggc aag aag cac ccg tgc tgt gtc tta tcc aat 759
Val Leu Thr Val Thr Gly Lys Lys His Pro Cys Cys Val Leu Ser Asn
        95                  100                 105

ccc gac cag aag ggt aag att agg aga atc gac tgc ctg cga cag gca 807
Pro Asp Gln Lys Gly Lys Ile Arg Arg Ile Asp Cys Leu Arg Gln Ala
    110                 115                 120

gac aaa gtc tgg cgt ctg gat cta gtc atg gtg atc ctg ttc aaa ggc 855
Asp Lys Val Trp Arg Leu Asp Leu Val Met Val Ile Leu Phe Lys Gly
125                 130                 135                 140

atc ccc ttg gaa agt acc gat gga gag cgg ctc atg aaa tcc cca cat 903
Ile Pro Leu Glu Ser Thr Asp Gly Glu Arg Leu Met Lys Ser Pro His
                145                 150                 155

tgc aca aac cca gca ctt tgt gtc cag cca cat cat atc aca gta tca 951
Cys Thr Asn Pro Ala Leu Cys Val Gln Pro His His Ile Thr Val Ser
            160                 165                 170

gtt aag gag ctt gat ttg ttt ttg gca tac tac gtg cag gag caa gat 999
Val Lys Glu Leu Asp Leu Phe Leu Ala Tyr Tyr Val Gln Glu Gln Asp
        175                 180                 185

tct gga caa tca gga agt cca agc cac aat gat cct gcc aag aat cct 1047
Ser Gly Gln Ser Gly Ser Pro Ser His Asn Asp Pro Ala Lys Asn Pro
    190                 195                 200

cca ggt tac ctt gag gat agt ttt gta aaa tct gga gtc ttc aat gta 1095
Pro Gly Tyr Leu Glu Asp Ser Phe Val Lys Ser Gly Val Phe Asn Val
205                 210                 215                 220

tca gaa ctt gta aga gta tcc aga acg ccc ata acc cag gga act gga 1143
Ser Glu Leu Val Arg Val Ser Arg Thr Pro Ile Thr Gln Gly Thr Gly
                225                 230                 235

gtc aac ttc cca att gga gaa atc cca agc caa cca tac tat cat gac 1191
Val Asn Phe Pro Ile Gly Glu Ile Pro Ser Gln Pro Tyr Tyr His Asp
            240                 245                 250

atg aac tcg ggg gtc aat ctt cag agg tct ctg tct tct cca cca agc 1239
Met Asn Ser Gly Val Asn Leu Gln Arg Ser Leu Ser Ser Pro Pro Ser

255                 260                 265

agc aaa aga ccc aaa act ata tcc ata gat gaa aat atg gaa cca agt      1287
Ser Lys Arg Pro Lys Thr Ile Ser Ile Asp Glu Asn Met Glu Pro Ser
    270                 275                 280

cct aca gga gac ttt tac ccc tct cca agt tca cca gct gct gga agt      1335
Pro Thr Gly Asp Phe Tyr Pro Ser Pro Ser Ser Pro Ala Ala Gly Ser
285                 290                 295                 300

cga aca tgg cac gaa aga gat caa gat atg tct tct ccg act act atg      1383
Arg Thr Trp His Glu Arg Asp Gln Asp Met Ser Ser Pro Thr Thr Met
                305                 310                 315

aag aag cct gaa aag cca ttg ttc agc tct gca tct cca cag gat tct      1431
Lys Lys Pro Glu Lys Pro Leu Phe Ser Ser Ala Ser Pro Gln Asp Ser
            320                 325                 330

tcc cca aga ctg agc act ttc ccc cag cac cac cat ccc gga ata cct      1479
Ser Pro Arg Leu Ser Thr Phe Pro Gln His His His Pro Gly Ile Pro
        335                 340                 345

gga gtt gca cac agt gtc atc tca act cga act cca cct cca cct tca      1527
Gly Val Ala His Ser Val Ile Ser Thr Arg Thr Pro Pro Pro Pro Ser
    350                 355                 360

ccg ttg cca ttt cca aca caa gct atc ctt cct cca gcc cca tcg agc      1575
Pro Leu Pro Phe Pro Thr Gln Ala Ile Leu Pro Pro Ala Pro Ser Ser
365                 370                 375                 380

tac ttt tct cat cca aca atc aga tat cct ccc cac ctg aat cct cag      1623
Tyr Phe Ser His Pro Thr Ile Arg Tyr Pro Pro His Leu Asn Pro Gln
                385                 390                 395

gat act ctg aag aac tat gta cct tct tat gac cca tcc agt cca caa      1671
Asp Thr Leu Lys Asn Tyr Val Pro Ser Tyr Asp Pro Ser Ser Pro Gln
            400                 405                 410

acc agc cag tcc tgg tac ctg ggc tag cttggttcct ttccaagtgt            1718
Thr Ser Gln Ser Trp Tyr Leu Gly
        415                 420

caaataggac acccatctta ccggccaatg tccaaaatta cggtttgaac ataattggag    1778

aacctttcct tcaagcagaa acaagcaact gagggaaaaa gaaacacaac aatagtttaa    1838

gaaatttttt ttttaaataa aaaaaaagga aaagaggaag actggacaaa acaacacaaa    1898

ggcagaaagg aaagaaactg aagaaagaag ataatagacc agcaattgca gcacttacaa    1958

tcactaattc ccttaaggtt gaaactgtaa tgacataaaa agggtcgatg atatttcact    2018

gatggtagat cgcagcccct gcaacgtagc ctttgttaca tgaagtccgc tgggaaatag    2078

atgttctgtc tctatgacaa tatattttaa ctgactttct agatgcctta atatttgcat    2138

gataagctag ttttattggt ttagtattct tgttgtttac gcatggaatc actattcctg    2198

gttatctcac caacgaaggc taggaggcgg cgtcagaggt gctgggtgac agagccatga    2258

gccagccatt ttataagcac tctgatttct aaaagttaaa aaaaatatat gaaatctctg    2318

tagcctttag ttatcagtac agatttatta aatttcggcc cttaacccag ccttttccag    2378

tgtgtaaccc agtttgaaat cttaaaaaaa gaaaaaatga aaaaaaaagg aaaaaaagaa    2438

aaaaggaaaa aaacagtttg aacacaaagg ctctatggaa gaaatgcctc tatgtaggtg    2498

aagtgttctc tctgcatgca acagtaaaaa ttaatataat attttcccca caaaagaaac 2558
acttaacaga ggcaagtgca atttataaat ttatatctaa aggggaatca tgattataag 2618
tccttcagcc cttggactct aaattgaggg gattaaaaag aatttaaaat aattttgaac 2678
gaatttattt tcccctcagt ttttgagggc attaaaaagg cattaaatca agacaaatca 2738
tgtgcttgag aaaaataaaa ttaatgaaaa cacagcactt atgttggttt agctgcagcc 2798
tccttggagg tagaatttat ttatttaaaa ttactggttg catcaagaac ccatagggtg 2858
tacaaaaggt tctataaaat ctgcattata gagacaaaga ggcaggcaaa tccatgtcac 2918
aagggtaaag cttacagttt acaaactggg aacgccaggg tgtaggatat aaaaacgcac 2978
tcttgagaaa acaaatgtaa tcagggtgct gaaaacttgc atggtgcttt cagacattag 3038
ccttgttcaa caaatttctt gtattgacag atccatagtg tgcatgggca gacacatttt 3098
gcctctatgt ctcttaaaat tttaattaaa aatactcttt ccagtaatcc taatttgcac 3158
gaagatataa tgtccacatt acgtgccttg ccttgaaatc taaaaaacaa aaaacaaaaa 3218
aaaaaaaaca aaaaaataca acaaagtgac atcactacac ttgttttgct gcatttatta 3278
tcattttaaa tctttaccat ttttatgaca aaatattttg tactccagac gaagaaaaat 3338
gtgtgacatc atggattttt tagacagtta tacctttatc tcacatttat aaagcatatc 3398
atggctgtgt atagttgccg cttaaaaatt gtaatcgacc agcaatattt tcagtatttt 3458
ggtgtttttt tctattaacc tttcatgttt ttcatcttcc aattaatatt tgggggggag 3518
gggtttcaaa tttatacgaa ttatgcaata ccaagttttg cctatgtagg tagtgctttt 3578
agctgtattg gttattatag gtaagtacac agatttaaaa aaaaaataat gtatgctttt 3638
ttgtttgttt gtttgtttta attgaccaaa gtgggtactg ctatttttgc agtgtgatga 3698
ggtccttttg tgtactgaga gatggacagg ggattttttt taatatacat atatatatat 3758
tctggggtgg gtgggaggat ttttaacact ttgcagtgta gctgtgaagc agtgcaccct 3818
gagatgggcc tgggctgcaa agcgactgtt ctgcctactg tgacaaactt caacttacac 3878
aggttcccct ctctaacttc ccacctgggt tgcaagctga actcattact ggttttcata 3938
acaacacaat agtaagaaca agcaaacaca acaaattctc ctggaggcag acttggctta 3998
aaaaggcaga cttggcttgg tgatagtttt tcttgaaagt tccagatcca cagtggagag 4058
tgagcctgtc tcatatttgg caaaaatatt tgttgaaatg tccacatagg ggatgttgga 4118
tgtttaacac ttttgagagt ttaacacatg aatattcttt ctcctagaaa acacattaga 4178
cctgttggag ggagtctccc gtattccttt tctgccactt ttcgtcccca tttcatttca 4238
ttaatgatag gatatgattt acctgtgact tactacttca aatggatggc agtgcacttg 4298
gatttttttt taatatccag aagattgaac agagggttgc tattgttgaa tgtatttgga 4358
ctgatagatt aaaatcaaag ttcaattttt aaggaacaaa aaagtaaatc ctgttttcat 4418
tttatctccc cttttaaaac tgagaaccag agcagaaggg aaatatagaa ttttaagcaa 4478
ttaatcttcc tgtggatgaa ttaaacccat tagatgctga tgggattttt ttaaggaatg 4538

gtaccttaac tatatatttg atttcgtttc ccctgagggc tagaggctga atggaggctg 4598
gttttatttt gcctttccct caccgcccag tcccattgag tgtattcatt actagaagga 4658
aaatctttca gaattggtga cacatggtag gctgtcttaa ggagtcccct ggcccccttc 4718
ccctaggcca tggcctaata aaataaactg tcaattgttc tcacagcata tcatttaata 4778
atgaatactt tagaacaatg cttatgggct ggagaattgt atttgattag cccattcagt 4838
ttgatagccc aaatgctgaa cagcacagcg ggatcctagc agtgcaagtt caaaagtaag 4898
tccaatcatt tctgtgatac tcgccctggt agcaaacaga tcatctcagc caagctcttc 4958
atgtatcttt gacctattag gtgaacaaat gaacctcaca ggacacacag tattttttaa 5018
aggcagactc gctctctttt ttgccagtga gcagttctag ctaaccaagt tacacactgt 5078
gggtattcct gcctgcctct tgaatacaaa ggcctagttc aagtgttgct ttttttattt 5138
caaatcaatt ttttcttctt tcctttttga gataaaacta ttaaaagtac tactatatat 5198
ataaaatctc aaatcaactt ttcggcctcc tcctcgtgta ccaggaagta tattctgacg 5258
aagggcccca cttttgcagg tcttgcacgc ccctccctta cccagaactg cagagcttca 5318
ggatggcgaa ggtcacccaa gggcatgagt agggagtggt gtctccaacc atcagttccg 5378
tggcactgtt cagcctttgt gtgctgccct gccacccacc actcacagtg cctctgaagc 5438
gtgttacccc tggagtgacg tgagcatttg aggcttgtct aaggaaaaaa ataaaaggca 5498
gtgaaggaga ctgtacataa agacatggca aaaatcttaa ttatagcaat atagttatcg 5558
ggtaatgttc gggtgggcag ctccattaaa aaatatgtga atgaatctgt gaagctgcaa 5618
gtagcgagaa gagcgaaagg tcttcttaat gaaccgccta ccttgtagac agtaatttgt 5678
acactgtata gttttgttaa gaattttttt taaattaaaa ttcccatgtt tgtaaagcta 5738
actttttaac aattataatg gaactatatg ttgtttccat ttttaaagta aacaagaata 5798
ttccttgttt agagactgga cttgagttaa aactctccag tctcttaagt tatgtattaa 5858
aaagaaaatc tgtccatgtt aggagttatt tcacagattc ctgtgcttga aaagcatagg 5918
atactaatcc tttaaaaaag tgtaaatgga gaaaagttat attttatgaa ggttattttg 5978
ttgtatttag tattggaaaa gttggtttcc agagcatttc agaatgtcga agcaccactg 6038
tctttttatt agtatatacg gcctttagca aaagtttttg tgattgttac gtgatggtat 6098
ttaaggttaa gtttcacaga gcattcagga taggcagaaa actaaaacag tgctatgtct 6158
cacataacgt gtcctcaggg agcagaatct tggatttgtg acttgtagct tcataaggac 6218
tcaacgaaag agattgcaca gggacatctt cagcggtgtg acagcaggac atgttcttta 6278
cctagattca aattctatgt actgtgtgaa atgatgaagg ctgcagaaag ttatcccata 6338
ttcagtgtac agtattcatt tttaatgaaa caactctaca atattgctgg cagataggcc 6398
ccaagcatga cattcaatat agtttacatg ttcctgtcaa ggtcttttgt taacattaac 6458
cagctgcatg ctttctggac tttaagaaat tgggtttcta tagaaaactt tttttttttt 6518
ttttttttta atgtgcaggc tattcaagtt caatagtaaa agctcaaaaa tgaatgttct 6578

actccatgct gaaggagctg aaagctgcct tcttcatatt ttgcactttc tggtagttcc 6638

cctgtttttt ctaattccct aaaattgtgt gggtggagtg gagccctgca gttggggggt 6698

aacatggacc actgattttg ccctttgacc ctgcacaatg acctttgcat cagccaaact 6758

cattgccatg acaactcttt gtactgtgtc cgtgccacag atctgttggt cacattgtta 6818

atagtaaagg ggacaagttg gagacggtca atttttacat tttttgttgc aattttttct 6878

tcaatggttg taagtagttt tttttttttt ttaataataa aagggttcac tagttaatac 6938

tctagaaata tctgtgtgtt gcaattcaaa tgtatgttga gattgtgaaa agcgcttcag 6998

tgccactagc ttaccggtac actagactaa gcccttgatg acttattgca tgatacagta 7058

ccaggaacaa caggtggcct aaatacatga aaagcagtgt aagctagtga cactaaagcc 7118

agtcttgtat tactgtattt ttgacagaat ggttttgaaa actgtgctac agggactgat 7178

gtggcaaata tatctcttta tgcagaagga agtctttttt tttctttttt ttttttttaa 7238

gaagtatggc tttttatgca tccttcatcg agggcattga agttgcatgg actgataaaa 7298

gttgatgcaa aacaagaaag aaacaaacaa aaaaaaaaaa ccagcaaaat gtttaccaaa 7358

aaactcaaac aaatgagcag tgcctgttca atttcacagt ctctgttgag ttcagttgta 7418

aatatgtttc aaatgacatt ttcttgggaa aaaaaatctc tacaacattg tagaatgtga 7478

ggggtaacta catcccaggc ataggtttct caaagctgca gtagattatg tcttcatcaa 7538

gctgttaatt tgtgcttata tcatatagaa cttttagcat cctgggaaga gctgccccca 7598

cctcaatgat atttctctga gaacaacttt tgtaggactg tgtgtttctt tagatacatt 7658

tagtacaact gtaggtgacg agtagtcagt tattgcttgc tagctacaca ccagggttga 7718

tccattttaa aacttttggc attttgtcct catgggccat aaatacagaa ccttgtattt 7778

taattaaatt tttttacaaa aggaggcaca tgcacaatct ccatgtaaca aacctttagc 7838

agtaggatgt attatacgac agttacttaa tttctagagt tcaggcctct gggatcaacc 7898

ccagactggg ccagaatgtt agtgaaggtt ttattgtgcc cggttggagg ataacgttct 7958

ttgggtactt tttgtgggtt gcaaatgaac tcaattgcca caagttttaa actggtgtaa 8018

atcaagcttg acttaatgtg attgttactg ttatatccag cctatactgc tagcagctgc 8078

tcatactgca gtcaattact ggaagcggat atatttccta tgcaaaaact gtttaaacaa 8138

taaaatgagc tatgctacag a 8159

<210> 10
<211> 420
<212> PRT
<213> Homo sapiens

<400> 10

Met Met Tyr Ser Pro Ile Cys Leu Thr Gln Asp Glu Phe His Pro Phe
1               5                   10                  15

Ile Glu Ala Leu Leu Pro His Val Arg Ala Ile Ala Tyr Thr Trp Phe
            20                  25                  30

Asn Leu Gln Ala Arg Lys Arg Lys Tyr Phe Lys Lys His Glu Lys Arg
        35                  40                  45

Met Ser Lys Asp Glu Glu Arg Ala Val Lys Asp Glu Leu Leu Ser Glu
    50                  55                  60

Lys Pro Glu Ile Lys Gln Lys Trp Ala Ser Arg Leu Leu Ala Lys Leu
65                  70                  75                  80

Arg Lys Asp Ile Arg Gln Glu Tyr Arg Glu Asp Phe Val Leu Thr Val
                85                  90                  95

Thr Gly Lys Lys His Pro Cys Cys Val Leu Ser Asn Pro Asp Gln Lys
            100                 105                 110

Gly Lys Ile Arg Arg Ile Asp Cys Leu Arg Gln Ala Asp Lys Val Trp
        115                 120                 125

Arg Leu Asp Leu Val Met Val Ile Leu Phe Lys Gly Ile Pro Leu Glu
    130                 135                 140

Ser Thr Asp Gly Glu Arg Leu Met Lys Ser Pro His Cys Thr Asn Pro
145                 150                 155                 160

Ala Leu Cys Val Gln Pro His His Ile Thr Val Ser Val Lys Glu Leu
                165                 170                 175

Asp Leu Phe Leu Ala Tyr Tyr Val Gln Glu Gln Asp Ser Gly Gln Ser
            180                 185                 190

Gly Ser Pro Ser His Asn Asp Pro Ala Lys Asn Pro Pro Gly Tyr Leu
        195                 200                 205

Glu Asp Ser Phe Val Lys Ser Gly Val Phe Asn Val Ser Glu Leu Val
    210                 215                 220

Arg Val Ser Arg Thr Pro Ile Thr Gln Gly Thr Gly Val Asn Phe Pro
225                 230                 235                 240

Ile Gly Glu Ile Pro Ser Gln Pro Tyr Tyr His Asp Met Asn Ser Gly
                245                 250                 255

Val Asn Leu Gln Arg Ser Leu Ser Ser Pro Pro Ser Ser Lys Arg Pro
            260                 265                 270

Lys Thr Ile Ser Ile Asp Glu Asn Met Glu Pro Ser Pro Thr Gly Asp
        275                 280                 285

Phe Tyr Pro Ser Pro Ser Ser Pro Ala Ala Gly Ser Arg Thr Trp His
    290                 295                 300

Glu Arg Asp Gln Asp Met Ser Ser Pro Thr Thr Met Lys Lys Pro Glu
305                 310                 315                 320

Lys Pro Leu Phe Ser Ser Ala Ser Pro Gln Asp Ser Ser Pro Arg Leu
                325                 330                 335

Ser Thr Phe Pro Gln His His His Pro Gly Ile Pro Gly Val Ala His
            340                 345                 350

Ser Val Ile Ser Thr Arg Thr Pro Pro Pro Pro Ser Pro Leu Pro Phe
        355                 360                 365

Pro Thr Gln Ala Ile Leu Pro Pro Ala Pro Ser Ser Tyr Phe Ser His
    370                 375                 380

Pro Thr Ile Arg Tyr Pro Pro His Leu Asn Pro Gln Asp Thr Leu Lys
385                 390                 395                 400

Asn Tyr Val Pro Ser Tyr Asp Pro Ser Ser Pro Gln Thr Ser Gln Ser
                405                 410                 415

Trp Tyr Leu Gly
            420

<210>  11
<211>  6821
<212>  DNA
<213>  Homo sapiens

<220>
<221>  CDS
<222>  (65)..(4504)
<223>

<400>  11
gggcgcagtc gggagccggc cgtggtggct ccgtgcgtcc gagcgtccgt ccgcgccgtc      60
ggcc atg gcc aag cgc tcc agg ggc ccc ggg cgc cgc tgc ctg ttg gcg      109
ctc gtg ctg ttc tgc gcc tgg ggg acg ctg gcc gtg gtg gcc cag aag      157
ccg ggc gca ggg tgt ccg agc cgc tgc ctg tgc ttc cgc acc acc gtg      205
cgc tgc atg cat ctg ctg ctg gag gcc gtg ccc gcc gtg gcg ccg cag      253
acc tcc atc cta gat ctt cgc ttt aac aga atc aga gag atc caa cct      301
ggg gca ttc agg cgg ctg agg aac ttg aac aca ttg ctt ctc aat aat      349
aat cag atc aag agg ata cct agt gga gca ttt gaa gac ttg gaa aat      397
tta aaa tat ctc tat ctg tac aag aat gag atc cag tca att gac agg      445
caa gca ttt aag gga ctt gcc tct cta gag caa cta tac ctg cac ttt      493
aat cag ata gaa act ttg gac cca gat tcg ttc cag cat ctc ccg aag      541
ctc gag agg cta ttt ttg cat aac aac cgg att aca cat tta gtt cca      589
ggg aca ttt aat cac ttg gaa tct atg aag aga ttg cga ctg gac tca      637
aac aca ctt cac tgc gac tgt gaa atc ctg tgg ttg gcg gat ttg ctg      685
aaa acc tac gcg gag tcg ggg aac gcg cag gca gcg gcc atc tgt gaa      733
tat ccc aga cgc atc cag gga cgc tca gtg gca acc atc acc ccg gaa      781
gag ctg aac tgt gaa agg ccc cgg atc acc tcc gag ccc cag gac gca      829
gat gtg acc tcg ggg aac acc gtg tac ttc acc tgc aga gcc gaa ggc      877
aac ccc aag cct gag atc atc tgg ctg cga aac aat aat gag ctg agc      925
atg aag aca gat tcc cgc cta aac ttg ctg gac gat ggg acc ctg atg      973
atc cag aac aca cag gag aca gac cag ggt atc tac cag tgc atg gca     1021
aag aac gtg gcc gga gag gtg aag acg caa gag gtg acc ctc agg tac     1069
ttc ggg tct cca gct cga ccc act ttt gta atc cag cca cag aat aca     1117
gag gtg ctg gtt ggg gag agc gtc acg ctg gag tgc agc gcc aca ggc     1165
cac ccc ccg ccg cgg atc tcc tgg acg aga ggt gac cgc aca ccc ttg      1213
cca gtt gac ccg cgg gtg aac atc acg cct tct ggc ggg ctt tac ata      1261
cag aac gtc gta cag ggg gac agc gga gag tat gcg tgc tct gcg acc      1309
aac aac att gac agc gtc cat gcc acc gct ttc atc atc gtc cag gct      1357
ctt cct cag ttc act gtg acg cct cag gac aga gtc gtt att gag ggc      1405
cag acc gtg gat ttc cag tgt gaa gcc aag ggc aac ccg ccg ccc gtc      1453
atc gcc tgg acc aag gga ggg agc cag ctc tcc gtg gac cgg cgg cac      1501
ctg gtc ctg tca tcg gga aca ctt aga atc tct ggt gtt gcc ctc cac      1549
gac cag ggc cag tac gaa tgc cag gct gtc aac atc atc ggc tcc cag      1597
aag gtc gtg gcc cac ctg act gtg cag ccc aga gtc acc cca gtg ttt      1645
gcc agc att ccc agc gac aca aca gtg gag gtg ggc gcc aat gtg cag      1693
ctc ccg tgc agc tcc cag ggc gag ccc gag cca gcc atc acc tgg aac      1741
aag gat ggg gtt cag gtg aca gaa agt gga aaa ttt cac atc agc cct      1789
gaa gga ttc ttg acc atc aat gac gtt ggc cct gca gac gca ggt cgc      1837
tat gag tgt gtg gcc cgg aac acc att ggg tcg gcc tcg gtg agc atg      1885
gtg ctc agt gtg aat gtt cct gac gtc agt cga aat gga gat ccg ttt      1933
gta gct acc tcc atc gtg gaa gcg att gcg act gtt gac aga gct ata      1981
aac tca acc cga aca cat ttg ttt gac agc cgt cct cgt tct cca aat      2029
gat ttg ctg gcc ttg ttc cgg tat ccg agg gat cct tac aca gtt gaa      2077
cag gca cgg gcg gga gaa atc ttt gaa cgg aca ttg cag ctc att cag      2125
gag cat gta cag cat ggc ttg atg gtc gac ctc aac gga aca agt tac      2173
cac tac aac gac ctg gtg tct cca cag tac ctg aac ctc atc gca aac      2221
ctg tcg ggc tgt acc gcc cac cgg cgc gtg aac aac tgc tcg gac atg      2269
tgc ttc cac cag aag tac cgg acg cac gac ggc acc tgt aac aac ctg      2317
cag cac ccc atg tgg ggc gcc tcg ctg acc gcc ttc gag cgc ctg ctg      2365
aaa tcc gtg tac gag aat ggc ttc aac acc cct cgg ggc atc aac ccc      2413
cac cga ctg tac aac ggg cac gcc ctt ccc atg ccg cgc ctg gtg tcc      2461
acc acc ctg atc ggg acg gag acc gtc aca ccc gac gag cag ttc acc      2509
cac atg ctg atg cag tgg ggc cag ttc ctg gac cac gac ctc gac tcc      2557
acg gtg gtg gcc ctg agc cag gca cgc ttc tcc gac gga cag cac tgc      2605
agc aac gtg tgc agc aac gac ccc ccc tgc ttc tct gtc atg atc ccc      2653
ccc aat gac tcc cgg gcc agg agc ggg gcc cgc tgc atg ttc ttc gtg      2701
cgc tcc agc cct gtg tgc ggc agc ggc atg act tcg ctg ctc atg aac      2749
tcc gtg tac ccg cgg gag cag atc aac cag ctc acc tcc tac ata gac      2797
gca tcc aac gtg tac ggg agc acg gag cat gag gcc cgc agc atc cgc     2845
gac ctg gcc agc cac cgc ggc ctg ctg cgg cag ggc atc gtg cag cgg     2893
tcc ggg aag ccg ctg ctc ccc ttc gcc acc ggg ccg ccc acg gag tgc     2941
atg cgg gac gag aac gag agc ccc atc ccc tgc ttc ctg gcc ggg gac     2989
cac cgc gcc aac gag cag ctg ggc ctg acc agc atg cac acg ctg tgg     3037
ttc cgc gag cac aac cgc att gcc acg  gag ctg ctc aag ctg  aac ccg     3085
cac tgg gac  ggc gac acc atc tac  tat gag acc agg aag  atc gtg     3130
ggt gcg gag  atc cag cac atc acc  tac cag cac tgg ctc  ccg aag     3175
atc ctg ggg  gag gtg ggc atg agg  acg ctg gga gag tac  cac ggc     3220
tac gac ccc  ggc atc aat gct ggc  atc ttc aac gcc ttc  gcc acc     3265
gcg gcc ttc  agg ttt ggc cac acg  ctt gtc aac cca ctg  ctt tac     3310
cgg ctg gac  gag aac ttc cag ccc  att gca caa gat cac  ctc ccc     3355
ctt cac aaa  gct ttc ttc tct ccc  ttc cgg att gtg aat  gag ggc     3400
ggc atc gat  ccg ctt ctc agg ggg  ctg ttc ggg gtg gcg  ggg aaa     3445
atg cgt gtg  ccc tcg cag ctg ctg  aac acg gag ctc acg  gag cgg     3490
ctg ttc tcc  atg gca cac acg gtg  gct ctg gac ctg gcg  gcc atc     3535
aac atc cag  cgg ggc cgg gac cac  ggg atc cca ccc tac  cac gac     3580
tac agg gtc tac tgc aat cta tcg gcg gca cac acg ttc gag gac        3625
ctg aaa aat gag att aaa aac cct gag atc cgg gag aaa ctg aaa        3670
agg ttg tat ggc tcg aca ctc aac atc gac ctg ttt ccg gcg ctc        3715
gtg gtg gag gac ctg gtg cct ggc agc cgg ctg ggc ccc acc ctg        3760
atg tgt ctt ctc agc aca cag ttc aag cgc ctg cga gat ggg gac        3805
agg ttg tgg tat gag aac cct ggg gtg ttc tcc ccg gcc cag ctg        3850
act cag atc aag cag acg tcg ctg gcc agg atc cta tgc gac aac        3895
gcg gac aac atc acc cgg gtg cag agc gac gtg ttc agg gtg gcg        3940
gag ttc cct cac ggc tac ggc agc tgt gac gag atc ccc agg gta        3985
gac ctc cgg gtg tgg cag gac tgc tgt gaa gac tgt agg acc agg        4030
ggg cag ttc aat gcc ttt tcc tat cat ttc cga ggc aga cgg tct        4075
ctt gag ttc agc tac cag gag gac aag ccg acc aag aaa aca aga        4120
cca cgg aaa ata ccc agt gtt ggg aga cag ggg gaa cat ctc agc        4165
aac agc acc tca gcc ttc agc aca cgc tca gat gca tct ggg aca        4210
aat gac ttc aga gag ttt gtt ctg gaa atg cag aag acc atc aca        4255
gac ctc aga aca cag ata aag aaa ctt gaa tca cgg ctc agt acc        4300
aca gag tgc gtg gat gcc ggg ggc gaa tct cac gcc aac aac acc        4345
aag tgg aaa aaa gat gca tgc acc att tgt gaa tgc aaa gac ggg      4390
cag gtc acc tgc ttc gtg gaa gct tgc ccc cct gcc acc tgt gct      4435
gtc ccc gtg aac atc cca ggg gcc tgc tgt cca gtc tgc tta cag      4480
aag agg gcg gag gaa aag ccc tag gctcctggga ggctcctcag agtttgtctg      4534
ctgtgccatc gtgagatcgg gtggccgatg gcagggagct gcggactgca gaccaggaaa      4594
cacccagaac tcgtgacatt tcatgacaac gtccagctgg tgctgttaca gaaggcagtg      4654
caggaggctt ccaaccagag catctgcgga gaaggaggca cagcaggtgc ctgaagggaa      4714
gcaggcagga gtcctagctt cacgttagac ttctcaggtt tttatttaat tcttttaaaa      4774
tgaaaaattg gtgctactat taaattgcac agttgaatca tttaggcgcc taaattgatt      4834
ttgcctccca acaccatttc tttttaaata aagcaggata cctctatatg tcagccttgc      4894
cttgttcaga tgccaggagc cggcagacct gtcacccgca ggtggggtga gtcttggagc      4954
tgccagaggg gctcaccgaa atcggggttc catcacaagc tatgtttaaa aagaaaattg      5014
gtgtttggca aacggaacag aacctttgat gagagcgttc acagggacac tgtctggggg      5074
tgcagtgcaa gcccccggcc tcttccctgg gaacctctga actcctcctt cctctgggct      5134
ctctgtaaca tttcaccaca cgtcagcatc taatcccaag acaaacattc ccgctgctcg      5194
aagcagctgt atagcctgtg actctccgtg tgtcagctcc ttccacacct gattagaaca      5254
ttcataagcc acatttagaa acaggtttgc tttcagctgt cacttgcaca catactgcct      5314
agttgtgaac caaatgtgaa aaaacctcct tcatcccatt gtgtatctga tacctgccga      5374
gggccaaggg tgtgtgttga caacgccgct cccagccggc cctggttgcg tccacgtcct      5434
gaacaagagc cgcttccgga tggctcttcc caagggagga ggagctcaag tgtcgggaac      5494
tgtctaactt caggttgtgt gagtgcgtta aaaaaaaaaa aaaaaaagaa tccctatacc      5554
tcatttgtat ttttaaaatg cgtgatgttt tatgaaattg tgtccatttt ttaggtatta      5614
gatatggcag aaaaaccatt tccactatgc aaagttcttt tagacgtcag tgaaaatcaa      5674
ctctcatacc tcatggtctc tctttaattg accaaaacct tccatttttc tctaaataca      5734
aagcgatctg tgttctgagc aacctttccc cgaacacaca gcttcagtgc agcacgctga      5794
cctgagtatc caccatgtgc caggcacagt gctgggcaca cgaggcacca aggtccgggc      5854
cacctgcccg cagcaaggcc cagctgaggt ggtggaggga gcccctgagg tcaggggccg      5914
tttcggttca gggtggcagg tgtccagcac tggggtatgg cgtcgaggct tccatggggt      5974
gggggaggcc agcttccttc tgacaggatg ggcgcataca gtgcctggtg tgatttgtgc      6034
acaacccgtg ttccaggtgc acatcctccc aaggagacac ccagaccctt ccagcacggg 6094
ccggccaagt tgctgcggcg gaggcagcat ttcagctgtg aggaaggtca ttggattcat 6154
gtgttttatc tgtaaaaatg gttgtcttaa cttcttaacc tcatattggt aagtgattga 6214
taaaaattgg ttggtgtttc atgacatgtg gacttctttt gaaatagcaa gtcaaatgta 6274
gtgaccaaat tgtggaagag atttctgtca aataggaaat gtgtaagttc gtctaaaagc 6334
tgatggttat gtaagttgct caggcactca gatgacagca gattctgggt tctgggagtg 6394
ttctgtgcct cttacatgcc ctggaggcct catggtctca gtgctgaggc ggcacacctg 6454
tagcacacct gcgtaatgtg cggtctgggc cagtcacaag gaattgtgtt gtctaagcca 6514
aagggggaag ctgactgtga tttaccaaaa aaaattctgt aattcaaacc aaaatgtctg 6574
cggaatcacc agtttgatac tctctgtaat cagaacagtg ggcagtgcct gggtgaacgt 6634
gtctagcagc cactgtgcgg gatcgctgta acaggagtgg aatgtacata tttatttact 6694
tttctaactg ctccaacagc caaatgcctt ttttatgacc attgtattca gttcattacc 6754
aaagaaatgt ttgcactttg taatgatgcc tttcagttca aataaatggg tcacattttc 6814
aaatgga 6821

<210> 12
<211> 1479
<212> PRT
<213> Homo sapiens

<400> 12

<210> 13
<211> 3493
<212> DNA
<213> Homo sapiens

<220>
<221> CDS
<222> (192)..(926)
<223>

<400> 13
ctgagaggga gacgctccaa gaggctcctc agtgtgggcg agtaaaatgc cctgcgtgtg      60

agaagcaggc tcagattatt gctactctgt attcagatct tcatgtgtct cttctaagct     120

gaataaagct gttttggaac tgtcaggtac ctcaaagctc tgactgacag tactccacaa     180

gcttgcctgc c atg ggc tgt cgg gat gtc cac gca gcc aca gtc ctt tcc      230
             Met Gly Cys Arg Asp Val His Ala Ala Thr Val Leu Ser
             1               5                   10

ttc ctg tgt gga atc gcc tca gta gca ggc ctc ttt gca ggg act ctg      278
Phe Leu Cys Gly Ile Ala Ser Val Ala Gly Leu Phe Ala Gly Thr Leu
    15                  20                  25

ctt ccc aac tgg aga aaa tta cga ttg atc aca ttc aac aga aac gag      326
Leu Pro Asn Trp Arg Lys Leu Arg Leu Ile Thr Phe Asn Arg Asn Glu
30                  35                  40                  45

aag aac ctg act gtt tac aca ggc ctg tgg gtg aaa tgt gcc cgg tat      374
Lys Asn Leu Thr Val Tyr Thr Gly Leu Trp Val Lys Cys Ala Arg Tyr
                50                  55                  60

gac ggg agc agt gac tgc ctg atg tac gac act act tgg tac tca tca      422
Asp Gly Ser Ser Asp Cys Leu Met Tyr Asp Thr Thr Trp Tyr Ser Ser
            65                  70                  75

gtt gac cag ctg gac ctg cgt gtc ctc cag ttt gcc cta ccc ctc agc      470
Val Asp Gln Leu Asp Leu Arg Val Leu Gln Phe Ala Leu Pro Leu Ser
        80                  85                  90

atg ctg atc gcc atg ggt gcc ctg ctg ctc tgc ctg att gga atg tgc      518
Met Leu Ile Ala Met Gly Ala Leu Leu Leu Cys Leu Ile Gly Met Cys
    95                  100                 105

aac act gcc ttc agg tcc tcg gtg ccc aac atc aaa ctg gcc aag tgt      566
Asn Thr Ala Phe Arg Ser Ser Val Pro Asn Ile Lys Leu Ala Lys Cys
110                 115                 120                 125

ctg gtc aat agt gca ggt tgc cac ctg gtg gct ggg ctg cta ttt ttc      614
Leu Val Asn Ser Ala Gly Cys His Leu Val Ala Gly Leu Leu Phe Phe
                130                 135                 140

ctg gca ggt act gtg agc ctc tcc cca tct atc tgg gtc atc ttt tat      662
Leu Ala Gly Thr Val Ser Leu Ser Pro Ser Ile Trp Val Ile Phe Tyr
            145                 150                 155

aac atc cat ctg aac aag aag ttt gag cca gtc ttt tca ttt gac tat      710
Asn Ile His Leu Asn Lys Lys Phe Glu Pro Val Phe Ser Phe Asp Tyr

160                 165                 170

gca gtg tat gtc act att gct agt gct ggg ggc ctg ttt atg act tcc      758
Ala Val Tyr Val Thr Ile Ala Ser Ala Gly Gly Leu Phe Met Thr Ser
    175                 180                 185

ctt ata cta ttt att tgg tat tgt aca tgc aaa tct ttg cct tct cct      806
Leu Ile Leu Phe Ile Trp Tyr Cys Thr Cys Lys Ser Leu Pro Ser Pro
190                 195                 200                 205

ttc tgg caa cca ttg tac tcc cat cca ccc agt atg cat act tac tca      854
Phe Trp Gln Pro Leu Tyr Ser His Pro Pro Ser Met His Thr Tyr Ser
                210                 215                 220

cag ccc tat tca gca cgc tct cgc ctc tct gcc att gaa att gac att      902
Gln Pro Tyr Ser Ala Arg Ser Arg Leu Ser Ala Ile Glu Ile Asp Ile
            225                 230                 235

cca gta gtt tca cac acc act taa tggggaaata gttaattgtt aaagaaaact      956
Pro Val Val Ser His Thr Thr
        240

tcttgtagcc tcacattccc cttgtgcaaa gagctctttt ggacctacat acattttcct   1016

ttgtttttga ccaatcaatg aagccaaatt tatatgtcct attagaatga agtgctgcta   1076

gtttttatga gaagtatatt atattaaatg tgaatttttt aaattttgct tcttatactg   1136

gaaggaattt tagccttcat attgatatct aattaattat ttaagtggaa gaggcctgca   1196

tcacaattga ggtaatgtag agcaacatgt taaagaatga tggttagcag aagctgttgt   1256

atacaatctt catgaaaatt tcagtgtgta tttttctttt tctataatac ctttaactgc   1316

aaagaaaagg cagtttcaaa tataagaaat ttatttcagg taagggtaat attttaatag   1376

tagtcagtaa tctagcttaa ggctgtaact cttctatcgg ggctaattgt atgaataggt   1436

gtcagtatgt tgaagattac tttcttttgt gactttcttc tacctcatgc cactgtttaa   1496

aagtaaaatg tattttaatg atgttagaat aagactacca ttctaaatat cacctactta   1556

tgaataacat gtaataattt ttaacattaa tgattccata aattgtatta ttgggattag   1616

aatgtgcttt atgacaggtt agtgtttcct ctgaggcaga aaactctttt ttggagatat   1676

cttccatcaa gcagtactcg tgcccatata caatctctta gtggctagga gaaataaata   1736

aaagggccat aatggtttgt tctctttcag acataattta gtaggggaca agaagtctgt   1796

tcttcagtga gtacactaga gatttactct ggtgactgcc ttttgagtta tgggtgaagt   1856

aaggtatggc tttaccataa ccttgattca ttcacccttg attcatttct cgcccccgtc   1916

actgattatt tccttgagca tatatctctg cctaacactt tagtaggtgc tatagaggat   1976

acatgaaaag tatgagatct ggttccatcc agtaagacat tttaatagag aagatcaaaa   2036

tgttacctgg cagttgggga ataatctgac ttcgttggca gttggcctta acttcttaat   2096

cattgatcca ggaatatttc aaccagagac acaactttct ggcagacaga caaattgtac   2156

aacaccaaca atatcctgga ccttgaaatt ctgtttactt cagtccattg tatcctttaa   2216

ggcacctgtg ctagcctaga ttttgtaata acactgattt atgagaatgg acaaaagtgg   2276

tagggaaatt gttccctctc cacttctgaa agtatgatga tgtattaagg atggaggagt   2336

tattaaaaat gtctcttctg atgaggtaac aattagatga aaccatgtta aagctgagat 2396

gaacacttag aaattcaggg atattgggtc tttagcctta tgaatttgag ctgcttattt 2456

aattggtgta atttactaca tattagtact atattcgtaa ggatttttta ttaaccatta 2516

cagattttac aaacagctag ttatatggta aacagattat tatgcctttt tgcaattctg 2576

aatatgattc tagtatttgt gtagatgtat ttggtacttt ttcccctaat tccaacacta 2636

gtttatatat atagcgaata aatctagttg tataaatttt taaatgccgt cagtagaaag 2696

cacacaaggt tatgattttt ttaattactg gcttctgatt tctttcactt ctgatccttt 2756

tcctttttct cagatgtagc tgagtcttga tcattttaag acaacgatgg gtagaatttt 2816

gagattaatg ttaattttcc ctttttgtta atttcagtcc cctctcacta tgcttttgtc 2876

cagaaggatc aagaattcta ccatcccttg ggtctttgtg tataaacaat gttaaataaa 2936

ggtagactca gtctttaaga tattagacag tttttttagt ccatgggatt gtaaatataa 2996

acattaactt tcctataaga atattttggc tttgtaatct atagcctcaa attggtattt 3056

attatggatt cactagacaa acagctgttt ccttattgtc ttttttcttt agtgtttctg 3116

atttgctatc agtagctgtt tttaaagccg tccaaggaaa ataattattt acagtttttg 3176

aagtcacttt tgagccctca tcaagctctc attgtgatgg gagggatacc tttttgttgt 3236

taaaagccta ttattgttaa aggcctttta tggaaaccaa cttggaaaac aaccttaaat 3296

gtggatgtat cagatttggt ttatccagcc atgggagaga aaacaaacct aagtttactt 3356

tacttgtaca tatacactac aatggatagt atatttgctg taaactacaa tgtaaaacct 3416

caataaaagt gcgctgtact tcttaatgtt tattaaaaga tgtattttta caaaaaaaaa 3476

aaaaaaaaaa aaaaaaa 3493

<210> 14
<211> 244
<212> PRT
<213> Homo sapiens

<400> 14

Met Gly Cys Arg Asp Val His Ala Ala Thr Val Leu Ser Phe Leu Cys
1               5                   10                  15

Gly Ile Ala Ser Val Ala Gly Leu Phe Ala Gly Thr Leu Leu Pro Asn
            20                  25                  30

Trp Arg Lys Leu Arg Leu Ile Thr Phe Asn Arg Asn Glu Lys Asn Leu
        35                  40                  45

Thr Val Tyr Thr Gly Leu Trp Val Lys Cys Ala Arg Tyr Asp Gly Ser
    50                  55                  60

Ser Asp Cys Leu Met Tyr Asp Thr Thr Trp Tyr Ser Ser Val Asp Gln
65                  70                  75                  80

Leu Asp Leu Arg Val Leu Gln Phe Ala Leu Pro Leu Ser Met Leu Ile
                85                  90                  95

Ala Met Gly Ala Leu Leu Leu Cys Leu Ile Gly Met Cys Asn Thr Ala
            100                 105                 110

Phe Arg Ser Ser Val Pro Asn Ile Lys Leu Ala Lys Cys Leu Val Asn
        115                 120                 125

Ser Ala Gly Cys His Leu Val Ala Gly Leu Leu Phe Phe Leu Ala Gly
    130                 135                 140

Thr Val Ser Leu Ser Pro Ser Ile Trp Val Ile Phe Tyr Asn Ile His
145                 150                 155                 160

Leu Asn Lys Lys Phe Glu Pro Val Phe Ser Phe Asp Tyr Ala Val Tyr
                165                 170                 175

Val Thr Ile Ala Ser Ala Gly Gly Leu Phe Met Thr Ser Leu Ile Leu
            180                 185                 190

Phe Ile Trp Tyr Cys Thr Cys Lys Ser Leu Pro Ser Pro Phe Trp Gln
        195                 200                 205

Pro Leu Tyr Ser His Pro Pro Ser Met His Thr Tyr Ser Gln Pro Tyr
    210                 215                 220

Ser Ala Arg Ser Arg Leu Ser Ala Ile Glu Ile Asp Ile Pro Val Val
225                 230                 235                 240

Ser His Thr Thr


<210>  15
<211>  1834
<212>  DNA
<213>  Homo sapiens

<220>
<221>  CDS
<222>  (42)..(977)
<223>

<400>  15
cgggggcggg acttcagcgg aggccggagc gaggcgtcgg g atg cag cgc ccc ggg      56
ccc ttc agc acc ctc tac ggg cgg gtc ttg gcc ccg ctg ccc ggg agg      104
gcc ggg ggc gcg gcc tcc ggc gga gga ggg aac agc tgg gac ctc ccg      152
ggt tcc cac gtg cgg ctg ccg ggg cgt gca cag tct ggg acc cgt ggc      200
ggt gct ggc aac aca agc acc agc tgc ggg gac tcc aac agc atc tgc        248
ccg gcc ccc tcc acg atg tcc aag gcc gag gag gcc aag aag ctg gcg        296
ggc cgc gcg gct gtg gag aac cac gtg agg aat aac caa gtg ctg gga        344
att gga agt ggt tct aca att gtc cat gct gtg cag cga ata gct gaa        392
agg gtg aag caa gag aat ctg aac ctc gtc tgt att ccc act tcc ttc        440
cag gcc cgc cag ctc atc ctg cag tat ggc ttg acc ctc agt gat ctg        488
gat cga cac cca gag atc gac ctt gcc atc gat ggt gct gat gaa gta        536
gat gct gat ctc aat ctc atc aag ggt ggc gga ggc tgc ctg acc cag        584
gag aag att gtg gct ggc tat gct agt cgc ttc atc gtg atc gct gat        632
ttc agg aaa gat tcg aag aat ctc ggg gat cag tgg cac aag gga atc        680
ccc atc gag gtc atc cca atg gcc tat gtc cca gtg agc cga gct gtg        728
agc cag aag ttt ggg ggc gtg gtt gaa ctt cga atg gct gtc aac aag        776
gct ggt cct gtg gtg aca gat aat ggg aat ttt atc ttg gac tgg aag        824
ttt gac cgg gta cac aaa tgg agt gaa gtg aat aca gct atc aaa atg        872
atc cca ggt gtg gtg gac aca ggc cta ttc atc aac atg gct gag aga        920
gtc tac ttt ggg atg cag gat ggc tca gtg aac atg agg gag aag cct        968
ttc tgt tga ccctgcaagg agcagagtgt gttcaccttg agtctccagc               1017
ccacagccaa ggtggacgta cctctccagg agcctttgcc ttaatgtatc tctgcctgga    1077
caacttgtgg tggggggtgg ggggaagagt gggaggggga gttaaatcca gtcttatgaa    1137
gtattgttat taaatgtctt tttaaaaaga gaaatataaa catatatttt tactattaaa    1197
atattcagtt ttttaaatga agtagaactt gagttcatgt tttatatgaa atatttacca    1257
aaaaaaaaaa atgaggtaaa ctgtatttaa aacctttgac ttgagtctgc tggtaaagct    1317
tctgaatatt gagtttgctg agaaataaaa atcaaaactt ctttaagctg gtaaagtgag    1377
gggcccacca gcagtgatct cctgatgcct tactggaaac tttgtttact tgtctgctac    1437
cctctgattt gtttttagtt agtttttatt gtgagcacac atagtaccta gttacatctt    1497
aagatcaggt ttataaaact gtggagtgga gcggtatggt atggaatgac ttggaatgta    1557
agctgtcagg gagaaaatgt tgttacactt ttgctaagat ctgggggttt cttcatattc    1617
ctgctgttgg aagcagttga ccagaaatgc ttgccagtac tgccaaagca ctgctgtgaa    1677
atgtgaagta ctttgttttt ttatttttaa tgattttctt tttgttatta atatttttct    1737
ctgttccttt gttattactt gcatggtttg gcgtcagaag tccttacctc tttatattgt    1797
ttgcaggttt aaataaaaca gtgtggtgcc attttga                              1834

<210>  16
<211>  311
<212>  PRT
<213>  Homo sapiens

<400>  16

<210>  17
<211>  3588
<212>  DNA
<213>  Homo sapiens

<220>
<221>  CDS
<222>  (82)..(1521)
<223>

<400>  17
gcagcagcag agaatccaaa ccctaaagct gatatcacaa agtaccattt ctccaagttg      60

ggggctcaga ggggagtcat c atg agc gat gtt acc att gtg aaa gaa ggt      111
                        Met Ser Asp Val Thr Ile Val Lys Glu Gly
                        1               5                   10

tgg gtt cag aag agg gga gaa tat ata aaa aac tgg agg cca aga tac     159
Trp Val Gln Lys Arg Gly Glu Tyr Ile Lys Asn Trp Arg Pro Arg Tyr
                15                  20                  25

ttc ctt ttg aag aca gat ggc tca ttc ata gga tat aaa gag aaa cct     207
Phe Leu Leu Lys Thr Asp Gly Ser Phe Ile Gly Tyr Lys Glu Lys Pro
            30                  35                  40

caa gat gtg gat tta cct tat ccc ctc aac aac ttt tca gtg gca aaa     255
Gln Asp Val Asp Leu Pro Tyr Pro Leu Asn Asn Phe Ser Val Ala Lys
        45                  50                  55

tgc cag tta atg aaa aca gaa cga cca aag cca aac aca ttt ata atc     303
Cys Gln Leu Met Lys Thr Glu Arg Pro Lys Pro Asn Thr Phe Ile Ile
    60                  65                  70

aga tgt ctc cag tgg act act gtt ata gag aga aca ttt cat gta gat     351
Arg Cys Leu Gln Trp Thr Thr Val Ile Glu Arg Thr Phe His Val Asp
75                  80                  85                  90

act cca gag gaa agg gaa gaa tgg aca gaa gct atc cag gct gta gca     399
Thr Pro Glu Glu Arg Glu Glu Trp Thr Glu Ala Ile Gln Ala Val Ala
                95                  100                 105

gac aga ctg cag agg caa gaa gag gag aga atg aat tgt agt cca act     447
Asp Arg Leu Gln Arg Gln Glu Glu Glu Arg Met Asn Cys Ser Pro Thr
            110                 115                 120

tca caa att gat aat ata gga gag gaa gag atg gat gcc tct aca acc     495
Ser Gln Ile Asp Asn Ile Gly Glu Glu Glu Met Asp Ala Ser Thr Thr
        125                 130                 135

cat cat aaa aga aag aca atg aat gat ttt gac tat ttg aaa cta cta     543
His His Lys Arg Lys Thr Met Asn Asp Phe Asp Tyr Leu Lys Leu Leu
    140                 145                 150

ggt aaa ggc act ttt ggg aaa gtt att ttg gtt cga gag aag gca agt     591
Gly Lys Gly Thr Phe Gly Lys Val Ile Leu Val Arg Glu Lys Ala Ser
155                 160                 165                 170

gga aaa tac tat gct atg aag att ctg aag aaa gaa gtc att att gca     639
Gly Lys Tyr Tyr Ala Met Lys Ile Leu Lys Lys Glu Val Ile Ile Ala
                175                 180                 185

aag gat gaa gtg gca cac act cta act gaa agc aga gta tta aag aac     687
Lys Asp Glu Val Ala His Thr Leu Thr Glu Ser Arg Val Leu Lys Asn
            190                 195                 200

act aga cat ccc ttt tta aca tcc ttg aaa tat tcc ttc cag aca aaa     735
Thr Arg His Pro Phe Leu Thr Ser Leu Lys Tyr Ser Phe Gln Thr Lys
        205                 210                 215

gac cgt ttg tgt ttt gtg atg gaa tat gtt aat ggg ggc gag ctg ttt     783
Asp Arg Leu Cys Phe Val Met Glu Tyr Val Asn Gly Gly Glu Leu Phe
    220                 225                 230

ttc cat ttg tcg aga gag cgg gtg ttc tct gag gac cgc aca cgt ttc     831
Phe His Leu Ser Arg Glu Arg Val Phe Ser Glu Asp Arg Thr Arg Phe
235                 240                 245                 250

tat ggt gca gaa att gtc tct gcc ttg gac tat cta cat tcc gga aag     879
Tyr Gly Ala Glu Ile Val Ser Ala Leu Asp Tyr Leu His Ser Gly Lys
                255                 260                 265

att gtg tac cgt gat ctc aag ttg gag aat cta atg ctg gac aaa gat     927
Ile Val Tyr Arg Asp Leu Lys Leu Glu Asn Leu Met Leu Asp Lys Asp
            270                 275                 280

ggc cac ata aaa att aca gat ttt gga ctt tgc aaa gaa ggg atc aca     975
Gly His Ile Lys Ile Thr Asp Phe Gly Leu Cys Lys Glu Gly Ile Thr
        285                 290                 295

gat gca gcc acc atg aag aca ttc tgt ggc act cca gaa tat ctg gca     1023
Asp Ala Ala Thr Met Lys Thr Phe Cys Gly Thr Pro Glu Tyr Leu Ala
    300                 305                 310

cca gag gtg tta gaa gat aat gac tat ggc cga gca gta gac tgg tgg     1071
Pro Glu Val Leu Glu Asp Asn Asp Tyr Gly Arg Ala Val Asp Trp Trp
315                 320                 325                 330

ggc cta ggg gtt gtc atg tat gaa atg atg tgt ggg agg tta cct ttc     1119
Gly Leu Gly Val Val Met Tyr Glu Met Met Cys Gly Arg Leu Pro Phe
                335                 340                 345

tac aac cag gac cat gag aaa ctt ttt gaa tta ata tta atg gaa gac     1167
Tyr Asn Gln Asp His Glu Lys Leu Phe Glu Leu Ile Leu Met Glu Asp
            350                 355                 360

att aaa ttt cct cga aca ctc tct tca gat gca aaa tca ttg ctt tca     1215
Ile Lys Phe Pro Arg Thr Leu Ser Ser Asp Ala Lys Ser Leu Leu Ser
        365                 370                 375

ggg ctc ttg ata aag gat cca aat aaa cgc ctt ggt gga gga cca gat     1263
Gly Leu Leu Ile Lys Asp Pro Asn Lys Arg Leu Gly Gly Gly Pro Asp
    380                 385                 390

gat gca aaa gaa att atg aga cac agt ttc ttc tct gga gta aac tgg     1311
Asp Ala Lys Glu Ile Met Arg His Ser Phe Phe Ser Gly Val Asn Trp
395                 400                 405                 410

caa gat gta tat gat aaa aag ctt gta cct cct ttt aaa cct caa gta     1359
Gln Asp Val Tyr Asp Lys Lys Leu Val Pro Pro Phe Lys Pro Gln Val
                415                 420                 425

aca tct gag aca gat act aga tat ttt gat gaa gaa ttt aca gct cag     1407
Thr Ser Glu Thr Asp Thr Arg Tyr Phe Asp Glu Glu Phe Thr Ala Gln
            430                 435                 440

act att aca ata aca cca cct gaa aaa tat gat gag gat ggt atg gac     1455
Thr Ile Thr Ile Thr Pro Pro Glu Lys Tyr Asp Glu Asp Gly Met Asp
        445                 450                 455

tgc atg gac aat gag agg cgg ccg cat ttc cct caa ttt tcc tac tct     1503
Cys Met Asp Asn Glu Arg Arg Pro His Phe Pro Gln Phe Ser Tyr Ser
    460                 465                 470

gca agt gga cga gaa taa gtctctttca ttctgctact tcactgtcat     1551
Ala Ser Gly Arg Glu
475

cttcaattta ttactgaaaa tgattcctgg acatcaccag tcctagctct tacacatagc     1611

aggggcacct tccgacatcc cagaccagcc aagggtcctc acccctcgcc acctttcacc     1671

ctcatgaaaa cacacataca cgcaaataca ctccagtttt tgtttttgca tgaaattgta     1731

tctcagtcta aggtctcatg ctgttgctgc tactgtctta ctattatagc aactttaaga     1791

agtaattttc caacctttgg aagtcatgag cccaccattg ttcatttgtg caccaattat     1851

catcttttga tcttttagtt tttccctcag tgaaggctaa atgagataca ctgattctag     1911

gtacattttt taactttcta gaagagaaaa actaactaga ctaagaagat ttagtttata     1971

aattcagaac aagcaattgt ggaagggtgg tggcgtgcat atgtaaagca catcagatcc     2031

gtgcgtgaag taggcatata tcactaagct gtggctggaa ttgattagga agcatttggt 2091

agaaggactg aacaactgtt gggatatata tatatatata taattttttt tttttaaatt 2151

cctggtggat actgtagaag aagcccatat cacatgtgga tgtcgagact tcacgggcaa 2211

tcatgagcaa gtgaacactg ttctaccaag aactgaaggc atatgcacag tcaaggtcac 2271

ttaaagggtc ttatgaaaca atttgagcca gagagcatct ttcccctgtg cttggaaacc 2331

tttttccttt cttgacattt atcacctctg atggctgaag aatgtagaca ggtataatga 2391

tactgctttt caccaaaatt tctacaccaa ggtaaacagg tgtttgcctt atttaatttt 2451

ttactttcag ttctacgtga attagctttt tctcagatgt tgaaactttg aatgtccttt 2511

tatgattttg tttatattgc agtagtattt attttttagt gatgagaatt gtatgtcatg 2571

ttagcaaacg cagctccaac ttatataaaa tagacttact gcagttactt ttgacccatg 2631

tgcaaggatt gtacacgctg atgagaatca tgcacttttt ctcctctgtt aaaaaaaatg 2691

ataaggctct gaaatggaat atattggtta gaatttggct ttgggagaag agatgctgcc 2751

atttaacccc ttggtactga aaatgagaaa atccccaact atgcatgcca aggggttaat 2811

gaaacaaata gctgttgacg tttgctcatt taagaatttg aaacgttatg atgacctggc 2871

aacaaaaagt aatgaagaaa attgagacct gagtgaagat aagaaatgat ctttacgtgg 2931

caaaatgaac acatcttgag tatttaggaa atgggcagtg aaggctaaga acctggtgtg 2991

tttcttggga tcatggtaca tttatcactg aattaagcca tcagggaaaa aacaacaaaa 3051

aaagagaaca cctccagctt ttctttttct gtatatactc atgtccccca gattccaaca 3111

tttctcactg aaagggggca tgtatgcaaa cctcatcttt ctccttcatt aatgatgatc 3171

ttcagattaa accctttggt gctaggagct gacaatttcc aaagcagcct gtgaagtcct 3231

aggggctggg ggccactctt gcggcaagca gaaggccatc ctactccgcg gagtgatcat 3291

ggaaatgtat tttagttaaa ctctgacagc tcccaaacgg aagactacag catgacgtag 3351

tattatgatt gcattgtatg aaagagcaag tgactttcta agtaggatga atcatattca 3411

tatgcagatg tcttagcctc ttgacgctgg aagtgtggat ttatagctat gaaaccactg 3471

ctggcagtgg gtgggccact gggactgacg ggggttaaag ggcattttac taaggcagct 3531

aagacatatt cagacatcaa cgttatcctt ctttttcata tttctacctg agtgaag 3588

<210> 18
<211> 479
<212> PRT
<213> Homo sapiens

<400> 18

Met Ser Asp Val Thr Ile Val Lys Glu Gly Trp Val Gln Lys Arg Gly
1               5                   10                  15

Glu Tyr Ile Lys Asn Trp Arg Pro Arg Tyr Phe Leu Leu Lys Thr Asp
            20                  25                  30

Gly Ser Phe Ile Gly Tyr Lys Glu Lys Pro Gln Asp Val Asp Leu Pro
            35                  40                  45

Tyr Pro Leu Asn Asn Phe Ser Val Ala Lys Cys Gln Leu Met Lys Thr
    50                  55                  60

Glu Arg Pro Lys Pro Asn Thr Phe Ile Ile Arg Cys Leu Gln Trp Thr
65                  70                  75                  80

Thr Val Ile Glu Arg Thr Phe His Val Asp Thr Pro Glu Glu Arg Glu
                85                  90                  95

Glu Trp Thr Glu Ala Ile Gln Ala Val Ala Asp Arg Leu Gln Arg Gln
            100                 105                 110

Glu Glu Glu Arg Met Asn Cys Ser Pro Thr Ser Gln Ile Asp Asn Ile
        115                 120                 125

Gly Glu Glu Glu Met Asp Ala Ser Thr Thr His His Lys Arg Lys Thr
    130                 135                 140

Met Asn Asp Phe Asp Tyr Leu Lys Leu Leu Gly Lys Gly Thr Phe Gly
145                 150                 155                 160

Lys Val Ile Leu Val Arg Glu Lys Ala Ser Gly Lys Tyr Tyr Ala Met
                165                 170                 175

Lys Ile Leu Lys Lys Glu Val Ile Ile Ala Lys Asp Glu Val Ala His
            180                 185                 190

Thr Leu Thr Glu Ser Arg Val Leu Lys Asn Thr Arg His Pro Phe Leu
        195                 200                 205

Thr Ser Leu Lys Tyr Ser Phe Gln Thr Lys Asp Arg Leu Cys Phe Val
    210                 215                 220

Met Glu Tyr Val Asn Gly Gly Glu Leu Phe Phe His Leu Ser Arg Glu
225                 230                 235                 240

Arg Val Phe Ser Glu Asp Arg Thr Arg Phe Tyr Gly Ala Glu Ile Val
                245                 250                 255

Ser Ala Leu Asp Tyr Leu His Ser Gly Lys Ile Val Tyr Arg Asp Leu
            260                 265                 270

Lys Leu Glu Asn Leu Met Leu Asp Lys Asp Gly His Ile Lys Ile Thr
        275                 280                 285

Asp Phe Gly Leu Cys Lys Glu Gly Ile Thr Asp Ala Ala Thr Met Lys
    290                 295                 300

Thr Phe Cys Gly Thr Pro Glu Tyr Leu Ala Pro Glu Val Leu Glu Asp
305                 310                 315                 320

Asn Asp Tyr Gly Arg Ala Val Asp Trp Trp Gly Leu Gly Val Val Met
                325                 330                 335

Tyr Glu Met Met Cys Gly Arg Leu Pro Phe Tyr Asn Gln Asp His Glu
            340                 345                 350

Lys Leu Phe Glu Leu Ile Leu Met Glu Asp Ile Lys Phe Pro Arg Thr
        355                 360                 365

Leu Ser Ser Asp Ala Lys Ser Leu Leu Ser Gly Leu Leu Ile Lys Asp
    370                 375                 380

Pro Asn Lys Arg Leu Gly Gly Gly Pro Asp Asp Ala Lys Glu Ile Met
385                 390                 395                 400

Arg His Ser Phe Phe Ser Gly Val Asn Trp Gln Asp Val Tyr Asp Lys
                405                 410                 415

Lys Leu Val Pro Pro Phe Lys Pro Gln Val Thr Ser Glu Thr Asp Thr
            420                 425                 430

Arg Tyr Phe Asp Glu Glu Phe Thr Ala Gln Thr Ile Thr Ile Thr Pro
        435                 440                 445

Pro Glu Lys Tyr Asp Glu Asp Gly Met Asp Cys Met Asp Asn Glu Arg
    450                 455                 460

Arg Pro His Phe Pro Gln Phe Ser Tyr Ser Ala Ser Gly Arg Glu
465                 470                 475

<210>  19
<211>  4706
<212>  DNA
<213>  Homo sapiens

<220>
<221>  CDS
<222>  (128)..(2479)
<223>

<400>  19
cgcggccccg gaggcagcag cagcggcggc ggcagccgga gcagtaggca cccgagcagc      60

gccagcggcc gagcgggcgg cttcctggcc tgggcgctcc ggtggcggcg gaggtgcgcg     120

cggagcc atg gtt atc atg tcg gag ttc agc gcg gac ccc gcg ggc cag       169
        Met Val Ile Met Ser Glu Phe Ser Ala Asp Pro Ala Gly Gln
        1               5                   10

ggt cag ggc cag cag aag ccc ctc cgg gtg ggt ttt tac gac atc gag       217
Gly Gln Gly Gln Gln Lys Pro Leu Arg Val Gly Phe Tyr Asp Ile Glu
15                  20                  25                  30

cgg acc ctg ggc aaa ggc aac ttc gcg gtg gtg aag ctg gcg cgg cat       265

Arg Thr Leu Gly Lys Gly Asn Phe Ala Val Val Lys Leu Ala Arg His
                35                  40                  45

cga gtc acc aaa acg cag gtt gca ata aaa ata att gat aaa aca cga      313
Arg Val Thr Lys Thr Gln Val Ala Ile Lys Ile Ile Asp Lys Thr Arg
            50                  55                  60

tta gat tca agc aat ttg gag aaa atc tat cgt gag gtt cag ctg atg      361
Leu Asp Ser Ser Asn Leu Glu Lys Ile Tyr Arg Glu Val Gln Leu Met
        65                  70                  75

aag ctt ctg aac cat cca cac atc ata aag ctt tac cag gtt atg gaa      409
Lys Leu Leu Asn His Pro His Ile Ile Lys Leu Tyr Gln Val Met Glu
    80                  85                  90

aca aag gac atg ctt tac atc gtc act gaa ttt gct aaa aat gga gaa      457
Thr Lys Asp Met Leu Tyr Ile Val Thr Glu Phe Ala Lys Asn Gly Glu
95                  100                 105                 110

atg ttt gat tat ttg act tcc aac ggg cac ctg agt gag aac gag gcg      505
Met Phe Asp Tyr Leu Thr Ser Asn Gly His Leu Ser Glu Asn Glu Ala
                115                 120                 125

cgg aag aag ttc tgg caa atc ctg tcg gcc gtg gag tac tgt cac gac      553
Arg Lys Lys Phe Trp Gln Ile Leu Ser Ala Val Glu Tyr Cys His Asp
            130                 135                 140

cat cac atc gtc cac cgg gac ctc aag acc gag aac ctc ctg ctg gat      601
His His Ile Val His Arg Asp Leu Lys Thr Glu Asn Leu Leu Leu Asp
        145                 150                 155

ggc aac atg gac atc aag ctg gca gat ttt gga ttt ggg aat ttc tac      649
Gly Asn Met Asp Ile Lys Leu Ala Asp Phe Gly Phe Gly Asn Phe Tyr
    160                 165                 170

aag tca gga gag cct ctg tcc acg tgg tgt ggg agc ccc ccg tat gcc      697
Lys Ser Gly Glu Pro Leu Ser Thr Trp Cys Gly Ser Pro Pro Tyr Ala
175                 180                 185                 190

gcc ccg gaa gtc ttt gag ggg aag gag tat gaa ggc ccc cag ctg gac      745
Ala Pro Glu Val Phe Glu Gly Lys Glu Tyr Glu Gly Pro Gln Leu Asp
                195                 200                 205

atc tgg agc ctg ggc gtg gtg ctg tac gtc ctg gtc tgc ggt tct ctc      793
Ile Trp Ser Leu Gly Val Val Leu Tyr Val Leu Val Cys Gly Ser Leu
            210                 215                 220

ccc ttc gat ggg cct aac ctg ccg acg ctg aga cag cgg gtg ctg gag      841
Pro Phe Asp Gly Pro Asn Leu Pro Thr Leu Arg Gln Arg Val Leu Glu
        225                 230                 235

ggc cgc ttc cgc atc ccc ttc ttc atg tct caa gac tgt gag agc ctg      889
Gly Arg Phe Arg Ile Pro Phe Phe Met Ser Gln Asp Cys Glu Ser Leu
    240                 245                 250

atc cgc cgc atg ctg gtg gtg gac ccc gcc agg cgc atc acc atc gcc      937
Ile Arg Arg Met Leu Val Val Asp Pro Ala Arg Arg Ile Thr Ile Ala
255                 260                 265                 270

cag atc cgg cag cac cgg tgg atg cgg gct gag ccc tgc ttg ccg gga      985
Gln Ile Arg Gln His Arg Trp Met Arg Ala Glu Pro Cys Leu Pro Gly
                275                 280                 285

ccc gcc tgc ccc gcc ttc tcc gca cac agc tac acc tcc aac ctg ggc     1033
Pro Ala Cys Pro Ala Phe Ser Ala His Ser Tyr Thr Ser Asn Leu Gly
            290                 295                 300

gac tac gat gag cag gcg ctg ggt atc atg cag acc ctg ggc gtg gac     1081

Asp Tyr Asp Glu Gln Ala Leu Gly Ile Met Gln Thr Leu Gly Val Asp
        305                 310                 315

cgg cag agg acg gtg gag tca ctg caa aac agc agc tat aac cac ttt 1129
Arg Gln Arg Thr Val Glu Ser Leu Gln Asn Ser Ser Tyr Asn His Phe
    320                 325                 330

gct gcc att tat tac ctc ctc ctt gag cgg ctc aag gag tat cgg aat 1177
Ala Ala Ile Tyr Tyr Leu Leu Leu Glu Arg Leu Lys Glu Tyr Arg Asn
335                 340                 345                 350

gcc cag tgc gcc cgc ccc ggg cct gcc agg cag ccg cgg cct cgg agc 1225
Ala Gln Cys Ala Arg Pro Gly Pro Ala Arg Gln Pro Arg Pro Arg Ser
                355                 360                 365

tcg gac ctc agt ggt ttg gag gtg cct cag gaa ggt ctt tcc acc gac 1273
Ser Asp Leu Ser Gly Leu Glu Val Pro Gln Glu Gly Leu Ser Thr Asp
            370                 375                 380

cct ttc cga cct gcc ttg ctg tgc ccg cag ccg cag acc ttg gtg cag 1321
Pro Phe Arg Pro Ala Leu Leu Cys Pro Gln Pro Gln Thr Leu Val Gln
        385                 390                 395

tcc gtc ctc cag gcc gag atg gac tgt gag ctc cag agc tcg ctg cag 1369
Ser Val Leu Gln Ala Glu Met Asp Cys Glu Leu Gln Ser Ser Leu Gln
    400                 405                 410

tgg ccc ttg ttc ttc ccg gtg gat gcc agc tgc agc gga gtg ttc cgg 1417
Trp Pro Leu Phe Phe Pro Val Asp Ala Ser Cys Ser Gly Val Phe Arg
415                 420                 425                 430

ccc cgg ccc gtg tcc cca agc agc ctg ctg gac aca gcc atc agt gag 1465
Pro Arg Pro Val Ser Pro Ser Ser Leu Leu Asp Thr Ala Ile Ser Glu
                435                 440                 445

gag gcc agg cag ggg ccg ggc cta gag gag gag cag gac acg cag gag 1513
Glu Ala Arg Gln Gly Pro Gly Leu Glu Glu Glu Gln Asp Thr Gln Glu
            450                 455                 460

tcc ctg ccc agc agc acg ggc cgg agg cac acc ctg gcc gag gtc tcc 1561
Ser Leu Pro Ser Ser Thr Gly Arg Arg His Thr Leu Ala Glu Val Ser
        465                 470                 475

acc cgc ctc tcc cca ctc acc gcg cca tgt ata gtc gtc tcc ccc tcc 1609
Thr Arg Leu Ser Pro Leu Thr Ala Pro Cys Ile Val Val Ser Pro Ser
    480                 485                 490

acc acg gca agt cct gca gag gga acc agc tct gac agt tgt ctg acc 1657
Thr Thr Ala Ser Pro Ala Glu Gly Thr Ser Ser Asp Ser Cys Leu Thr
495                 500                 505                 510

ttc tct gcg agc aaa agc ccc gcg ggg ctc agt ggc acc ccg gcc act 1705
Phe Ser Ala Ser Lys Ser Pro Ala Gly Leu Ser Gly Thr Pro Ala Thr
                515                 520                 525

cag ggg ctg ctg ggc gcc tgc tcc ccg gtc agg ctg gcc tcg ccc ttc 1753
Gln Gly Leu Leu Gly Ala Cys Ser Pro Val Arg Leu Ala Ser Pro Phe
            530                 535                 540

ctg ggg tcg cag tcc gcc acc cca gtg ctg cag gct cag ggg ggc ttg 1801
Leu Gly Ser Gln Ser Ala Thr Pro Val Leu Gln Ala Gln Gly Gly Leu
        545                 550                 555

gga gga gct gtt ctg ctc cct gtc agc ttc cag gag gga cgg cgg gcg 1849
Gly Gly Ala Val Leu Leu Pro Val Ser Phe Gln Glu Gly Arg Arg Ala
    560                 565                 570

tcg gac acc tca ctg act caa ggg ctg aag gcc ttt cgg cag cag ctg 1897

Ser Asp Thr Ser Leu Thr Gln Gly Leu Lys Ala Phe Arg Gln Gln Leu
575                 580                 585                 590

agg aag acc acg cgg acc aaa ggg ttt ctg gga ctg aac aaa atc aag      1945
Arg Lys Thr Thr Arg Thr Lys Gly Phe Leu Gly Leu Asn Lys Ile Lys
                595                 600                 605

ggg ctg gct cgc cag gtg tgc cag gcc ccc gcc agc cgg gcc agc agg      1993
Gly Leu Ala Arg Gln Val Cys Gln Ala Pro Ala Ser Arg Ala Ser Arg
            610                 615                 620

ggc ggc ctg agc ccc ttc cac gcc cct gca cag agc cca ggc ctg cac      2041
Gly Gly Leu Ser Pro Phe His Ala Pro Ala Gln Ser Pro Gly Leu His
        625                 630                 635

ggc ggc gca gcc ggc agc cgg gag ggc tgg agc ctg ctg gag gag gtg      2089
Gly Gly Ala Ala Gly Ser Arg Glu Gly Trp Ser Leu Leu Glu Glu Val
    640                 645                 650

cta gag cag cag agg ctg ctc cag tta cag cac cac ccg gcc gct gca      2137
Leu Glu Gln Gln Arg Leu Leu Gln Leu Gln His His Pro Ala Ala Ala
655                 660                 665                 670

ccc ggc tgc tcc cag gcc ccc cag ccg gcc cct gcc ccg ttt gtg atc      2185
Pro Gly Cys Ser Gln Ala Pro Gln Pro Ala Pro Ala Pro Phe Val Ile
                675                 680                 685

gcc ccc tgt gat ggc cct ggg gct gcc ccg ctc ccc agc acc ctc ctc      2233
Ala Pro Cys Asp Gly Pro Gly Ala Ala Pro Leu Pro Ser Thr Leu Leu
            690                 695                 700

acg tcg ggg ctc ccg ctg ctg ccg ccc cca ctc ctg cag acc ggc gcg      2281
Thr Ser Gly Leu Pro Leu Leu Pro Pro Pro Leu Leu Gln Thr Gly Ala
        705                 710                 715

tcc ccg gtg gcc tca gcg gcg cag ctc ctg gac aca cac ctg cac att      2329
Ser Pro Val Ala Ser Ala Ala Gln Leu Leu Asp Thr His Leu His Ile
    720                 725                 730

ggc acc ggc ccc acc gcc ctc ccc gct gtg ccc cca cca cgc ctg gcc      2377
Gly Thr Gly Pro Thr Ala Leu Pro Ala Val Pro Pro Pro Arg Leu Ala
735                 740                 745                 750

agg ctg gcc cca ggt tgt gag ccc ctg ggg ctg ctg cag ggg gac tgt      2425
Arg Leu Ala Pro Gly Cys Glu Pro Leu Gly Leu Leu Gln Gly Asp Cys
                755                 760                 765

gag atg gag gac ctg atg ccc tgc tcc cta ggc acg ttt gtc ctg gtg      2473
Glu Met Glu Asp Leu Met Pro Cys Ser Leu Gly Thr Phe Val Leu Val
            770                 775                 780

cag tga gggcagccct gcatcctggc acggacactg actcttacag caataacttc      2529
Gln

agaggaggtg aagacatctg gcctcaaagc caagaacttt ctagaagcga aataagcaat      2589

acgttaggtg ttttggcttt ttagtttatt tttgttttat ttttttcttg cactgagtga      2649

cctcaacttt gagtagggac tggaaacttt aggaagaaag ataattgagg ggcgtgtctg      2709

ggggcggggg caggagggga gcggggtgga gggaacacgt gcagtgccgt ggtgtgggga      2769

tctcggcccc tctctctggg ttcgtcgtgg ttgagatgat tacctcggac gtctacggaa      2829

acgagcgggc gcattgttgt ccgcttgtgt gtgtgtgtgt gtgtgtgtgt gtgcgcgtgc      2889

attgattact atccatttct ttagtcaacg ctctccactt cctgatttct gctttaagga      2949

aaactgtgaa ctttctgctt catgtatcag ttttaaagca gcccaggcaa agatcatcta 3009

cagattctag gaattctctc ccctgaaatc aaaacctgga agactttttt ttcttatttt 3069

agttgagaag tttcataaac tgctcaagga ttagttttcc aggactctgc ggaggaacgg 3129

caggaagaac ctcagagagg gcagaggtga cttcaaagtg ctggggactc cgtcctgagg 3189

gtcacttggc cctgagcccc tgcgtgccct tgcggaagcc cagaagcttc ttcctgctgc 3249

acctcccgtt tccgctgctg ctgacgttta tgcatttcat gatggggtcc aacaagaaca 3309

cctgacttgg gtgaagttgt gcaatattgg aggctgactg tagggctggg cagctgggag 3369

acaggctcat ggctcatggc tcatggctca gggcggtgcc tgccctgggc cgggaccccc 3429

ctccccaccc cccacctagg ctttttgggt tttgttcaag gaaggtaaag tgagaggttt 3489

aggtcagtgt ttttaagttt ttgttttttt tttaaagcaa atcctgtata tgtatctaca 3549

tgggagacag gtagacacta cttatttgtt acattttgta ctatacgttt gtgttccagg 3609

tttcagcttc cctcgctcct gttgttaaga agcgtccctg tcagcacagg tgtgcattga 3669

ggaaggggcc ccagggcctt cgctccctca gcactggggt ggaggcggca ggaaggggcg 3729

gcccttacct ggcaggtctg ggcgcacctt tagcaggtgg actccgtggg gctccaccag 3789

ccagaagcct ctggaaggca acgaaggcaa tgctgctccc tgagtccagt ccccgccccc 3849

aaacccagcc caggtgcctt cagctacttc ggcttcttaa accctgcagt gttaaacaga 3909

ggcattgaga aaggggaaag gcgggtattt ttaaaagcca aagattgacc cagttacttg 3969

agggtaggga ggcgggccca gtgcaggagg ctgcatccct ggcctgctgg tgcccaccgg 4029

gggctgtgcc tgtgccgggc cgcagggaag ctggctgccc ccattcctgc tgctgctgct 4089

gctgctgctc tgtggctgtt tcaaagactg ggcgaaaggc tgtccggagg gcagaccagg 4149

tgccttgccg cagagaaaac accaaagtct cctgttcgct cataaagaag tttttgggat 4209

gggagagaat ccagaccatc ttggggcagc caggcccttg ccttcatttt tacagaggta 4269

gcacaactga ttccaacaca aaaccccttc ccctttttaa aatgatttct gttctaatgc 4329

catagatcaa aggcctcaga aaccattgtg tgtttcctct ttgaagcaat gacaagcact 4389

ttactttcac ggtggttttt gttttttctt attgctgtgg aacctctttt ggaggacgtt 4449

aaaggcgtgt tttacttgtt tttttaagag tgtgtgatgt gtgttttgta gatttcttga 4509

cagtgctgta atacagacgg caatgcaata gcctatttaa agacactacg tgatctgatt 4569

gagatgtaca tagttttttt ttttaccata actgaattat tttatctctt atgttaacat 4629

gagaaatgta tgccaaatga ttagttgatg tatgtttttt aatttaatat ttaaataaaa 4689

tatttggaag gaaaaaa 4706

<210> 20
<211> 783
<212> PRT
<213> Homo sapiens

<400> 20

Met Val Ile Met Ser Glu Phe Ser Ala Asp Pro Ala Gly Gln Gly Gln
1               5                   10                  15

Gly Gln Gln Lys Pro Leu Arg Val Gly Phe Tyr Asp Ile Glu Arg Thr
            20                  25                  30

Leu Gly Lys Gly Asn Phe Ala Val Val Lys Leu Ala Arg His Arg Val
        35                  40                  45

Thr Lys Thr Gln Val Ala Ile Lys Ile Ile Asp Lys Thr Arg Leu Asp
    50                  55                  60

Ser Ser Asn Leu Glu Lys Ile Tyr Arg Glu Val Gln Leu Met Lys Leu
65                  70                  75                  80

Leu Asn His Pro His Ile Ile Lys Leu Tyr Gln Val Met Glu Thr Lys
                85                  90                  95

Asp Met Leu Tyr Ile Val Thr Glu Phe Ala Lys Asn Gly Glu Met Phe
            100                 105                 110

Asp Tyr Leu Thr Ser Asn Gly His Leu Ser Glu Asn Glu Ala Arg Lys
        115                 120                 125

Lys Phe Trp Gln Ile Leu Ser Ala Val Glu Tyr Cys His Asp His His
    130                 135                 140

Ile Val His Arg Asp Leu Lys Thr Glu Asn Leu Leu Leu Asp Gly Asn
145                 150                 155                 160

Met Asp Ile Lys Leu Ala Asp Phe Gly Phe Gly Asn Phe Tyr Lys Ser
                165                 170                 175

Gly Glu Pro Leu Ser Thr Trp Cys Gly Ser Pro Pro Tyr Ala Ala Pro
            180                 185                 190

Glu Val Phe Glu Gly Lys Glu Tyr Glu Gly Pro Gln Leu Asp Ile Trp
        195                 200                 205

Ser Leu Gly Val Val Leu Tyr Val Leu Val Cys Gly Ser Leu Pro Phe
    210                 215                 220

Asp Gly Pro Asn Leu Pro Thr Leu Arg Gln Arg Val Leu Glu Gly Arg
225                 230                 235                 240

Phe Arg Ile Pro Phe Phe Met Ser Gln Asp Cys Glu Ser Leu Ile Arg
                245                 250                 255

Arg Met Leu Val Val Asp Pro Ala Arg Arg Ile Thr Ile Ala Gln Ile
            260                 265                 270

Arg Gln His Arg Trp Met Arg Ala Glu Pro Cys Leu Pro Gly Pro Ala
        275                 280                 285

Cys Pro Ala Phe Ser Ala His Ser Tyr Thr Ser Asn Leu Gly Asp Tyr
    290                 295                 300

Asp Glu Gln Ala Leu Gly Ile Met Gln Thr Leu Gly Val Asp Arg Gln
305                 310                 315                 320

Arg Thr Val Glu Ser Leu Gln Asn Ser Ser Tyr Asn His Phe Ala Ala
                325                 330                 335

Ile Tyr Tyr Leu Leu Leu Glu Arg Leu Lys Glu Tyr Arg Asn Ala Gln
            340                 345                 350

Cys Ala Arg Pro Gly Pro Ala Arg Gln Pro Arg Pro Arg Ser Ser Asp
        355                 360                 365

Leu Ser Gly Leu Glu Val Pro Gln Glu Gly Leu Ser Thr Asp Pro Phe
    370                 375                 380

Arg Pro Ala Leu Leu Cys Pro Gln Pro Gln Thr Leu Val Gln Ser Val
385                 390                 395                 400

Leu Gln Ala Glu Met Asp Cys Glu Leu Gln Ser Ser Leu Gln Trp Pro
                405                 410                 415

Leu Phe Phe Pro Val Asp Ala Ser Cys Ser Gly Val Phe Arg Pro Arg
            420                 425                 430

Pro Val Ser Pro Ser Ser Leu Leu Asp Thr Ala Ile Ser Glu Glu Ala
        435                 440                 445

Arg Gln Gly Pro Gly Leu Glu Glu Glu Gln Asp Thr Gln Glu Ser Leu
    450                 455                 460

Pro Ser Ser Thr Gly Arg Arg His Thr Leu Ala Glu Val Ser Thr Arg
465                 470                 475                 480

Leu Ser Pro Leu Thr Ala Pro Cys Ile Val Val Ser Pro Ser Thr Thr
                485                 490                 495

Ala Ser Pro Ala Glu Gly Thr Ser Ser Asp Ser Cys Leu Thr Phe Ser
            500                 505                 510

Ala Ser Lys Ser Pro Ala Gly Leu Ser Gly Thr Pro Ala Thr Gln Gly
        515                 520                 525

Leu Leu Gly Ala Cys Ser Pro Val Arg Leu Ala Ser Pro Phe Leu Gly
    530                 535                 540

Ser Gln Ser Ala Thr Pro Val Leu Gln Ala Gln Gly Gly Leu Gly Gly
545                 550                 555                 560

Ala Val Leu Leu Pro Val Ser Phe Gln Glu Gly Arg Arg Ala Ser Asp
                565                 570                 575

Thr Ser Leu Thr Gln Gly Leu Lys Ala Phe Arg Gln Gln Leu Arg Lys
            580                 585                 590

Thr Thr Arg Thr Lys Gly Phe Leu Gly Leu Asn Lys Ile Lys Gly Leu
        595                 600                 605

Ala Arg Gln Val Cys Gln Ala Pro Ala Ser Arg Ala Ser Arg Gly Gly
    610                 615                 620

Leu Ser Pro Phe His Ala Pro Ala Gln Ser Pro Gly Leu His Gly Gly
625                 630                 635                 640

Ala Ala Gly Ser Arg Glu Gly Trp Ser Leu Leu Glu Glu Val Leu Glu
                645                 650                 655

Gln Gln Arg Leu Leu Gln Leu Gln His His Pro Ala Ala Ala Pro Gly
            660                 665                 670

Cys Ser Gln Ala Pro Gln Pro Ala Pro Ala Pro Phe Val Ile Ala Pro
        675                 680                 685

Cys Asp Gly Pro Gly Ala Ala Pro Leu Pro Ser Thr Leu Leu Thr Ser
    690                 695                 700

Gly Leu Pro Leu Leu Pro Pro Pro Leu Leu Gln Thr Gly Ala Ser Pro
705                 710                 715                 720

Val Ala Ser Ala Ala Gln Leu Leu Asp Thr His Leu His Ile Gly Thr
                725                 730                 735

Gly Pro Thr Ala Leu Pro Ala Val Pro Pro Pro Arg Leu Ala Arg Leu
            740                 745                 750

Ala Pro Gly Cys Glu Pro Leu Gly Leu Leu Gln Gly Asp Cys Glu Met
        755                 760                 765

Glu Asp Leu Met Pro Cys Ser Leu Gly Thr Phe Val Leu Val Gln
    770                 775                 780

<210>  21
<211>  21
<212>  RNA
<213>  Artificial

<220>
<223>  siRNA

<400>  21
gguucgagac auguguucut t                                                 21


<210>  22
<211>  21
<212>  RNA
<213>  Artificial

<220>
<223>  siRNA

<400>  22
ggaaggagac ccguguacat t                                                 21


<210>  23
<211>  21
<212>  RNA
<213>  Artificial

<220>
<223>  siRNA

<400>  23
ggagacccgu guacaguaut t                                                 21